# EUROPEAN PATENT APPLICATION

(11) **EP 2 662 383 A1**
(43) Date of publication of application: **13.11.2013**
(21) Application number: 13177308.7
(22) Date of filing: 25.08.2009
(51) Int. Cl.: C07K 14/47, C07K 14/705, C07K 14/52, C07K 14/715, C12N 15/62

(54) **PD-I antagonists and methods for treating infectious disease**

(30) Priority: 25.08.2008 US 91502 P; 25.08.2008 US 91694 P; 25.08.2008 US 91705 P; 25.08.2008 US 91709 P; 05.01.2009 US 142548 P; 01.04.2009 US 165652 P
(62) Divisional of application: 09807659.9
(71) Applicant: Amplimmune, Inc., Rockville, MD 20850 (US)
(72) Inventor: Langermann, Solomon, Baltimore, MD 21215 (US)
(74) Representative: Bond, Christopher William

(57) **Abstract**

Methods and compositions for treating an infection or disease that results from (1) failure to elicit rapid T cell mediated responses, (2) induction of T cell exhaustion, T cell anergy or both, or (3) failure to activate monocytes, macrophages, dendritic cells and/or other APCs, for example, as required to kill intracellular pathogens. The method and compositions solve the problem of undesired T cell inhibition by binding to and blocking PD-I to prevent or reduce inhibitory signal transduction, or by binding to Hgands of PD-I such as PD-L1, thereby preventing (in whole or in part) the ligand from binding to PD-I to deliver an inhibitory signal. The immune response can be modulated by providing antagonists which bind with different affinity (i.e., more or less as required), by varying the dosage of agent which is administered, by intermittent dosing over a regime, and combinations thereof, that provides for dissociation of agent from the molecule to which it is bound prior to being administered again (similar to what occurs with antigen elicitation using priming and boosting). In some cases it may be particularly desirable to stimulate the immune system, then remove the stimulation.

## Description

### CROSS-REFERENCE TO RELATED APPLICATIONS

This application claims priority to and benefit of U.S. Provisional Application No. 61/091,502 filed on August 25, 2008; U.S. Provisional Application No. 61/091,694, filed on August 25, 2008, U.S. Provisional Application No. 61/091,709, filed on August 25, 2008, U.S. Provisional Application No. 61/091,705, filed on August 25, 2008, U.S. Provisional Application No. 61/142,548 filed on January 5, 2009, and U.S. Provisional Application No. 61/165,652, filed on April 1, 2009, and where permissible are incorporated by reference in their entireties.

### FIELD OF THE INVENTION

This invention generally relates to immunomodulatory compositions and methods for treating diseases such as cancer or infections, in particular to diseases inducing T cell exhaustion, T cell anergy, or both, or diseases where intracellular pathogens. i.e. e.g. Leishmania, evade immune response by upregulating PD-1 ligands on APCs (e.g. monocytes, dendritic cells, macrophages) or epithelial cells.

### BACKGROUND OF THE INVENTION

Host resistance to microbial infection integrates two major and overlapping defense systems, innate and adaptive immunity. Intracellular pathogens - including viruses, bacteria and parasites - can quickly relay activation signals that stimulate non-specific humoral and cellular effector responses in the infected host early after infection. Assisted by these innate defense responses, the rate of microbial growth is delayed for several days, while the adaptive branch of immunity is primed and prompted to confront the pathogens for the long term (adaptive/long-term immunity). These immune responses are mediated by T cells. For many intracellular pathogens, protective immunity requires both the generation of CD4+ helper T cells that produce compounds such as cytokines that stimulate other immune cells to help fight infection early-on, cell mediated responses mediated predominantly by CD8+ cytotoxic T lymphocytes (CTL) that eliminate pathogen-infected host cells, and antibody responses mediated by T helper cells. However, infection can become established and persist when the organisms bypass early immune activation and impair effector immune responses and long-term memory responses. This results in acute and chronic infections.

Studies have demonstrated that early immune subversion is often targeted against intracellular pathways involved in antigen processing and/or presentation by class I MHC molecules. This results in poor initial immune activation and little or no primary response to the organism. This allows the organisms to become established and for intracellular pathogens to remain "hidden" from the immune system. More recent studies have shown that in many cases these pathogens stimulate a low but measurable, specific immune response. However, chronic infections result when T cells become "exhausted" by the fight with the pathogen, undergoing profound changes that make them progressively less effective over time. This is a phenomenon known as T cell exhaustion.

B7 proteins act to provide a second signal to immune cells (e.g. T cells) that stimulates or inhibits the immune response. PD-L1 (B7-H1) and PD-L2 (PD-DC) are inhibitory members of the B7 family of molecules that bind to the common receptor, PD-1. PD-L1 is broadly expressed on a wide variety of tissue and cell types, while PD-L2 expression is predominantly restricted to activated dendritic cells (DC) and macrophages. PD-1, a member of the CD28 family of receptors, is inducibly expressed on activated T cells, B cells, natural killer (NK) cells, monocytes, DC, and macrophages. T cell exhaustion has been shown to be caused by inhibitory T cell signaling through the PD-1 receptor, which negatively regulates T cell function.

The primary result of PD-1 ligation by its ligands is to inhibit signaling downstream of the T cell Receptor (TCR). Therefore, signal transduction via PD-1 usually provides a suppressive or inhibitory signal to the T cell that results in decreased T cell proliferation or other reduction in T cell activation. PD-1 signaling is thought to require binding to a PD-1 ligand in close proximity to a peptide antigen presented by major histocompatibility complex (MHC), which is bound to the TCR (Freeman Proc. Natl. Acad. Sci. U. S. A 105:10275-10276 (2008).). PD-L1 is the predominant PD-1 ligand causing inhibitory signal transduction in T cells.

As a result of poor primary and effector immune responses against many intracellular pathogens, no effective vaccines exist against many of these organisms such as human immunodeficiency virus (HIV), hepatitis C virus (HCV), herpes simplex virus (HSV), *M. tuberculosis, C. trachomitis,* malaria, among others. This is a severe problem where chronic infections have taken hold and the host immune system fails to clear these chronic or latent infections. Poor primary and effector responses to an antigen/vaccine also poses a problem in cases where rapid immunity is required (even where otherwise effective vaccines can be made), for example during endemic/pandemic outbreaks such as flu, or in the event of a bioterrorism attack with infectious agents (e.g. anthrax), as well as in the pediatric and aging population where immune systems are undeveloped or weakened.

One approach to improving immunogenicity and protection of vaccines is the use of adjuvants. Adjuvants are ingredients added to a vaccine to improve the immune response. Most of the adjuvants that have been developed or are being tested elicit predominantly innate immune responses (not antigen-specific), antibody responses and in very few cases modest T cell responses. None of the adjuvants available induce a potent effector response or rapid T cell proliferation response which is what is required to augment primary responses and elicit protective immunity against intracellular pathogens.

Thus, it is an object of the invention to provide a vaccine adjuvant that enhances both primary and effector immune responses.

It is another object to provide compositions that provide a more rapid induction of protection as well as robust effector responses against chronic infections.

It is another object to provide compositions and methods for treating infections that induce T cell exhaustion, T cell anergy, or both.

It is yet another object of the invention to provide compositions and methods for treating intracellular infections of antigen presenting cells, including monocytes, dendritic cells, macrophages.

### SUMMARY OF THE INVENTION

Methods and compositions for treating an infection or disease that results from (1) failure to elicit rapid T cell mediated responses, (2) induction of T cell exhaustion, T cell anergy or both, or (3) failure to activate monocytes, macrophages, dendritic cells and/or other APCs, for example, as required to kill intracellular pathogens. These may be caused by an acute (e.g. toxin-induced), chronic, slow, or latent infection. The method and compositions of the invention solve the problem of undesired T cell inhibition by binding to and blocking PD-1 to prevent or reduce inhibitory signal transduction, or by binding to and blocking ligands of PD-1 such as PD-L1, thereby preventing (in whole or in part) the ligand from binding to PD-1 to deliver an inhibitory signal. These molecules are referred to generally as PD-1 antagonists, and include both compounds that bind directly to PD-1 or a ligand such as PD-L1. In either case, T cell responses, such as T cell proliferation or activation, are increased. In addition, the PD-1 antagonists may bind to and block PD-1 ligands expressed on antigen presenting cells (APCs, such as monocytes, macrophages, dendritic cells, epithelial cells etc) which are upregulated by intracellular pathogens.

There are two mechanisms by which an immune response can be enhanced or augmented: 1) Interfering with molecules that inhibit T cell activity, for example, where the molecule is PD-1, and one either a) blocks the receptor (PD-1) or b) blocks the ligand (B7-H1 or B7-DC), or 2) Augmenting molecules that activate T cell activity, for example, where the molecule is CD28, and an agonist is added. The immune response can be modulated by providing antagonists which bind with different affinity (i.e., more or less as required), by varying the dosage of agent which is administered, by intermittent dosing over a regime, and combinations thereof, that provides for dissociation of agent from the molecule to which it is bound prior to being administered again (similar to what occurs with antigen elicitation using priming and boosting),. In some cases it may be particularly desirable to stimulate the immune system, and then remove the stimulation. The affinity of the antagonist for its binding partner can be used to determine the period of time required for dissociation - a higher affinity agent will take longer to dissociate than a lower affinity agent. Combinations of antagonists that bind to either PD-1 or a ligand, or which bind with different affinities to the same molecule, can also be used to modulate the degree of immunostimulation.

The compositions include PD-1 antagonists that: (i) bind to and block PD-1 without inducing inhibitory signal transduction through PD-1 and prevents binding of ligands, such as PD-L1 and PD-L2, thereby preventing activation of the PD-1 mediated inhibitory signal; or (ii) bind to ligands of PD-1 and prevent binding to the PD-1 receptor, thereby preventing activation of the PD-1 mediated inhibitory signal.

A preferred composition includes an effective amount of a non-antibody PD-1 antagonist such as a PD-L2 fusion protein (PD-L2-Ig) to reduce or overcome lack of sufficient T cell responses, T cell exhaustion, T cell anergy, as well as activation of monocytes, macrophages, dendritic cells and other APCs, or all of these effects in a subject. PD-1 antagonists also include PD-L1 proteins, fragments, variants or fusions thereof that bind to PD-1 without triggering inhibitory signal transduction through PD-1. These fragments of PD-L1 are also referred to as non-functional PD-L1 fragments. PD-L2 polypeptides, fusion proteins, and non-functional PD-L1 fragments can inhibit or reduce the inhibitory signal transduction that occurs through PD-1 in T cells by preventing endogenous ligands of PD-1 from interacting with PD-1. Additional preferred PD-1 antagonists include PD-1 or soluble fragments thereof, that bind to ligands of PD-1 and prevent binding to the endogenous PD-1 receptor on T cells. These fragments of PD-1 are also referred to as soluble PD-1 fragments. Other PD-1 antagonists include B7.1 or soluble fragments thereof, that can bind to PD-L1 and prevent binding of PD-L1 to PD-1.

Additional embodiments include antibodies that bind to and block either the PD-1 receptor, without causing inhibitory signal transduction, or ligands of the PD-1 receptor, such as PD-L1 and PD-L2. The PD-L2 polypeptides, fusion proteins, and non-functional PD-L1 fragments may also activate T cells by binding to another receptor on the T cells or APCs.

The action of the PD-1 antagonists helps overcome T cell exhaustion, T cell anergy, or both, as well as activate monocytes, macrophages, dendritic cells and other APCs induced by infections or cancer. Representative infections that can be treated with the PD-L2 polypeptides or fusion proteins include, but are not limited to, infections caused by a virus, bacterium, parasite, protozoan, or fungus. Exemplary viral infections that can be treated include, but are not limited to, infections caused by hepatitis virus, human immunodeficiency virus (HIV), human T-lymphotrophic virus (HTLV), herpes virus, influenza, Epstein-Barr virus, filovirus, or a human papilloma virus. Other infections that can be treated include those caused by *Plasmodium, Mycoplasma, M tuberculosis, Bacillus anthracis, Staphylococcus,* and *C. trachomitis.*

The PD-1 antagonists can be administered in combination or alternation with a vaccine containing one or more antigens such as viral antigens, bacterial antigens, protozoan antigens, and tumor specific antigens. The PD-1 antagonists can be used as effective adjuvants with vaccines to increase primary immune responses and effector cell responses in subjects. Preferred subjects to be treated have a weakened or compromised immune system, are greater than 65 years old, or are less than 2 years of age.

### BRIEF DESCRIPTION OF THE DRAWINGS

Figures 1A-B are graphs showing B7-DC-Ig binding to PD-1 in a PD-1 binding ELISA.
Figure 2 is a graph showing that B7-DC-Ig binds to PD-1 expressing CHO cells.
Figure 3 is a graph showing that B7-DC-Ig competes with B7-H1 for binding to PD-1.
Figure 4 shows that B7-DC-Ig combination treatment resulted in generation of antigen-specific memory CTLs in a tumor model.
Figure 5 shows that B7-DC-Ig reduced HSV-2 viral particle shedding and enhanced mouse survival in the presence of a HSV-2 vaccine.

### DETAILED DESCRIPTION OF THE INVENTION

### I. Definitions

As used herein the term "isolated" is meant to describe a compound of interest (e.g., either a polynucleotide or a polypeptide) that is in an environment different from that in which the compound naturally occurs e.g. separated from its natural milieu such as by concentrating a peptide to a concentration at which it is not found in nature. "Isolated" is meant to include compounds that are within samples that are significantly enriched for the compound of interest and/or in which the compound of interest is partially or significantly purified. "Significantly" means statistically signficantly greater.

As used herein, the term "polypeptide" refers to a chain of amino acids of any length, regardless of modification (e.g., phosphorylation or glycosylation).

As used herein, a "variant" polypeptide contains at least one amino acid sequence alteration as compared to the amino acid sequence of the corresponding wild-type polypeptide.

As used herein, an "amino acid sequence alteration" can be, for example, a substitution, a deletion, or an insertion of one or more amino acids.

As used herein, a "vector" is a replicon, such as a plasmid, phage, or cosmid, into which another DNA segment may be inserted so as to bring about the replication of the inserted segment. The vectors described herein can be expression vectors.

As used herein, an "expression vector" is a vector that includes one or more expression control sequences

As used herein, an "expression control sequence" is a DNA sequence that controls and regulates the transcription and/or translation of another DNA sequence.

As used herein, "operably linked" means incorporated into a genetic construct so that expression control sequences effectively control expression of a coding sequence of interest.

As used herein, a "fragment" of a polypeptide refers to any subset of the polypeptide that is a shorter polypeptide of the full length protein. Generally, fragments will be five or more amino acids in length.

As used herein, "valency" refers to the number of binding sites available per molecule.

As used herein, "conservative" amino acid substitutions are substitutions wherein the substituted amino acid has similar structural or chemical properties.

As used herein, "non-conservative" amino acid substitutions are those in which the charge, hydrophobicity, or bulk of the substituted amino acid is significantly altered.

As used herein, "isolated nucleic acid" refers to a nucleic acid that is separated from other nucleic acid molecules that are present in a mammalian genome, including nucleic acids that normally flank one or both sides of the nucleic acid in a mammalian genome.

As used herein with respect to nucleic acids, the term "isolated" includes any non-naturally-occurring nucleic acid sequence, since such non-naturally-occurring sequences are not found in nature and do not have immediately contiguous sequences in a naturally-occurring genome.

As used herein, the term "host cell" refers to prokaryotic and eukaryotic cells into which a recombinant expression vector can be introduced.

As used herein, "transformed" and "transfected" encompass the introduction of a nucleic acid (e.g., a vector) into a cell by a number of techniques known in the art.

As used herein, the term "antibody" is meant to include both intact molecules as well as fragments thereof that include the antigen-binding site. These include Fab and F(ab')₂ fragments which lack the Fc fragment of an intact antibody.

By "immune cell" is meant a cell of hematopoietic origin and that plays a role in the immune response. Immune cells include lymphocytes (e.g., B cells and T cells), natural killer cells, and myeloid cells (e.g., monocytes, macrophages, eosinophils, mast cells, basophils, and granulocytes).

The term 'T cell" refers to a CD4+ T cell or a CD8+ T cell. The term T cell includes both TH1 cells, TH2 cells and Thl7 cells.

The term "T cell cytoxicity" includes any immune response that is mediated by CD8+ T cell activation. Exemplary immune responses include cytokine production, CD8+ T cell proliferation, granzyme or perforin production, and clearance of an infectious agent.

The term "immune cell" refers to T cells, B cells, and lymphocytes.

The term "inhibitory signal transduction" refers to signaling through the PD-1 receptor by PD-L1, or any other ligand, having the effect of suppressing, or otherwise reducing, T cell responses, whether by reducing T cell proliferation or by any other inhibitory mechanism..

### II. PD-1 Antagonists

A preferred PD-1 antagonist compound for interfering with the interaction between PD-1 and PD-L1 is PD-L2 (also known as B7-DC), the extracellular domain of PD-L2, fusion proteins of PD-L2, and variants thereof which bind to and block PD-1 without triggering inhibitory signal transduction through PD-1, and prevent binding of PD-L1 to PD-1. Additional PD-1 antagonists include fragments of PD-L1 that bind to PD-1 without triggering inhibitory signal transduction through PD-1, PD-1 or soluble fragments thereof that bind to ligands of PD-1 and prevent binding to the endogenous PD-1 receptor on T cells, and B7.1 or soluble fragments thereof that can bind to PD-L1 and prevent binding of PD-L1 to PD-1. In certain embodiments, PD-1 antagonists increase T cell cytotoxicity in a subject. The multiple functionality PD-1 antagonists helps to induce a robust immune response in subjects and overcome T cell exhaustion and T cell anergy.

PD-1 antagonists bind to ligands of PD-1 and interfere with or inhibit the binding of the ligands to the PD-1 receptor, or bind directly to the PD-1 receptor without engaging in signal transduction through the PD-1 receptor. In preferred embodiments, the PD-1 antagonists bind directly to PD-1 and block PD-1 inhibitory signal transduction. In other embodiments the PD-1 antagonists bind to ligands of PD-1 and reduce or inhibit the ligands from triggering inhibitory signal transduction through the PD-1. In still another embodiment, the PD-1 antagonists can activate T cells by binding to a receptor other than the PD-1 receptor.

The PD-1 antagonists can be small molecule antagonists. The term "small molecule" refers to small organic compounds having a molecular weight of more than 100 and less than about 2,500 daltons, preferably between 100 and 2000, more preferably between about 100 and about 1250, more preferably between about 100 and about 1000, more preferably between about 100 and about 750, more preferably between about 200 and about 500 daltons. The small molecules often include cyclical carbon or heterocyclic structures and/or aromatic or polyaromatic structures substituted with one or more functional groups. The small molecule antagonists reduce or interfere with PD-1 receptor signal transduction by binding to ligands of PD-1 such as PD-L1 and PD-L2 and preventing the ligand from interacting with PD-1 or by binding directly to the PD-1 receptor without triggering signal transduction through the PD-1 receptor.

Exemplary PD-1 antagonists include, but are not limited to, PD-L2, PD-L1, PD-1 or B7-1 polypeptides, and variants, fragments or fusion proteins thereof. Additional embodiments include antibodies that bind to any of these proteins.

### A. PD-L2 Based PD-1 antagonists

### 1. PD-L2 Based PD-1 antagonists that Bind to PD-1

PD-1 antagonists bind to PD-1 on immune cells and block inhibitory PD-1 signaling. PD-1 signal transduction is thought to require binding to PD-1 by a PD-1 ligand (PD-L2 or PD-L1; typically PD-L1) in close proximity to the TCR:MHC complex within the immune synapse. Therefore, proteins, antibodies or small molecules that block inhibitory signal transduction through PD-1 and optionally prevent co-ligation of PD-1 and TCR on the T cell membrane are useful PD-1 antagonists.

Representative polypeptide antagonists include, but are not limited to, PD-L2 polypeptides, fragments thereof, fusion proteins thereof, and variants thereof. PD-L2 polypeptides that bind to PD-1 and block inhibitory signal transduction through PD-1 are one of the preferred embodiments. Other embodiments include PD-1 antagonists that prevent native ligands of PD-1 from binding and triggering signal transduction. In certain embodiments, it is believed that the disclosed PD-L2 polypeptides have reduced or no ability to trigger signal transduction through the PD-1 receptor because there is no co-ligation of the TCR by the peptide-MHC complex in the context of the immune synapse. Because signal transduction through the PD-1 receptor transmits a negative signal that attenuates T-cell activation and T-cell proliferation, inhibiting the PD-1 signal transduction pathway allows cells to be activated that would otherwise be attenuated.

### 2. Exemplary PD-L2 Polypeptide PD-1

### Antagonists

Murine PD-L2 polypeptides can have at least 80%, 85%, 90%, 95%, 99% or 100% sequence identity to:

MLLLLPILNL SLQLHPVAAL FTVTAPKEVY TVDVGSSVSL ECDFDRRECT ELEGIRASLQ 60
KVENDTSLQS ERATLLEEQL PLGKALFHIP SVQVRDSGQY RCLVICGAAW DYKYLTVKVK 120
ASYMRIDTRI LEVPGTGEVQ LTCQARGYPL AEVSWQNVSV PANTSHIRTP EGLYQVTSVL 180
RLKPQPSRNF SCMFWNAHMK ELTSAIIDPL SRMEPKVPRT WPLHVFIPAC TIALIFLAIV 240
IIQRKRI 247
(SEQ ID NO:1) or

LFTVTAPKEV YTVDVGSSVS LECDFDRREC TELEGIRASL QKVENDTSLQ SERATLLEEQ 60
LPLGKALFHI PSVQVRDSGQ YRCLVICGAA WDYKYLTVKV KASYMRIDTR ILEVPGTGEV 120
QLTCQARGYP LAEVSWQNVS VPANTSHIRT PEGLYQVTSV LRLKPQPSRN FSCMFWNAHM 180
KELTSAIIDP LSRMEPKVPR TWPLHVFIPA CTIALIFLAI VIIQRKRI 228
(SEQ ID NO:2).

Human PD-L2 polypeptides can have at least 80%, 85%, 90%, 95%, 99% or 100% sequence identity to:

MIFLLLMLSL ELQLHQIAAL FTVTVPKELY IIEHGSNVTL ECNFDTGSHV NLGAITASLQ 60
KVENDTSPHR ERATLLEEQL PLGKASFHIP QVQVRDEGQY QCIIIYGVAW DYKYLTLKVK 120
ASYRKINTHI LKVPETDEVE LTCQATGYPL AEVSWPNVSV PANTSHSRTP EGLYQVTSVL 180

RLKPPPGRNF SCVFWNTHVR ELTLASIDLQ SQMEPRTHPT WLLHIFIPFC IIAFIFIATV 240
IALRKQLCQK LYSSKDTTKR PVTTTKREVN SAI 273
(SEQ ID NO:3) or

LFTVTVPKEL YIIEHGSNVT LECNFDTGSH VNLGAITASL QKVENDTSPH RERATLLEEQ 60
LPLGKASFHI PQVQVRDEGQ YQCIIIYGVA WDYKYLTLKV KASYRKINTH ILKVPETDEV 120
ELTCQATGYP LAEVSWPNVS VPANTSHSRT PEGLYQVTSV LRLKPPPGRN FSCVFWNTHV 180
RELTLASIDL QSQMEPRTHP TWLLHIFIPF CIIAFIFIAT VIALRKQLCQ KLYSSKDTTK 240
RPVTTTKREV NSAI 254
(SEQ ID NO:4).

Non-human primate (*Cynomolgus*) PD-L2 polypeptides can have at least 80%, 85%, 90%, 95%, 99% or 100% sequence identity to:

MIFLLLMLSL ELQLHQIAAL FTVTVPKELY IIEHGSNVTL ECNFDTGSHV NLGAITASLQ 60
KVENDTSPHR ERATLLEEQL PLGKASFHIP QVQVRDEGQY QCIIIYGVAW DYKYLTLKVK 120
ASYRKINTHI LKVPETDEVE LTCQATGYPL AEVSWPNVSV PANTSHSRTP EGLYQVTSVL 180
RLKPPPGRNF SCVFWNTHVR ELTLASIDLQ SQMEPRTHPT WLLHIFIPSC IIAFIFIATV 240
IALRKQLCQK LYSSKDATKR PVTTTKREVN SAI 273

(SEQ ID NO:5) or
LFTVTVPKEL YIIEHGSNVT LECNFDTGSH VNLGAITASL QKVENDTSPH RERATLLEEQ 60
LPLGKASFHI PQVQVRDEGQ YQCIIIYGVA WDYKYLTLKV KASYRKINTH ILKVPETDEV 120
ELTCQATGYP LAEVSWPNVS VPANTSHSRT PEGLYQVTSV LRLKPPPGRN FSCVFWNTHV 180
RELTLASIDL QSQMEPRTHP TWLLHIFIPS CIIAFIFIAT VIALRKQLCQ KLYSSKDATK 240
RPVTTTKREV NSAI 254
(SEQ ID NO:6)

SEQ ID NOs: 1, 3 and 5 each contain a signal peptide.

### B. PD-L1 Based PD-1 Antagonists

### 1. PD-L1 Based PD-1 Antagonists that Bind to PD-1

### Receptors

Other PD-1 antagonists that bind to the PD-1 receptor include, but are not limited to, PD-L1 polypeptides, fragments thereof, fusion proteins thereof, and variants thereof. These PD-1 polypeptide antagonists bind to and block the PD-1 receptor and have reduced or no ability to trigger inhibitory signal transduction through the PD-1 receptor. In one embodiment, it is believed that the PD-L1 polypeptides have reduced or no ability to trigger signal transduction through the PD-1 receptor because there is no co-ligation of the TCR by the peptide-MHC complex in the context of the immune synapse. Because signal transduction through the PD-1 receptor transmits a negative signal that attenuates T-cell activation and T-cell proliferation, inhibiting the PD-1 signal transduction using PD-L1 polypeptides allows cells to be activated that would otherwise be attenuated.

### 2. Exemplary PD-L1 Polypeptide PD-1

### Antagonists

Murine PD-L1 polypeptides can have at least 80%, 85%, 90%, 95%, 99% or 100% sequence identity to:

MRIFAGIIFT ACCHLLRAFT ITAPKDLYVV EYGSNVTMEC RFPVERELDL LALVVYWEKE 60
DEQVIQFVAG EEDLKPQHSN FRGRASLPKD QLLKGNAALQ ITDVKLQDAG VYCCIISYGG 120
ADYKRITLKV NAPYRKINQR ISVDPATSEH ELICQAEGYP EAEVIWTNSD HQPVSGKRSV 180
TTSRTEGMLL NVTSSLRVNA TANDVFYCTF WRSQPGQNHT AELIIPELPA THPPQNRTHW 240
VLLGSILLFL IVVSTVLLFL RKQVRMLDVE KCGVEDTSSK NRNDTQFEET 290
(SEQ ID NO:7) or

 60
 120
 180
 240
 272
(SEQ ID NO:8).

Human PD-L1 polypeptides can have at least 80%, 85%, 90%, 95%, 99% or 100% sequence identity to:

MRIFAVFIFM TYWHLLNAFT VTVPKDLYVV EYGSNMTIEC KFPVEKQLDL AALIVYWEME 60
DKNIIQFVHG EEDLKVQHSS YRQRARLLKD QLSLGNAALQ ITDVKLQDAG VYRCMISYGG 120
ADYKRITVKV NAPYNKINQR ILVVDPVTSE HELTCQAEGY PKAEVIWTSS DHQVLSGKTT 180
TTNSKREEKL FNVTSTLRIN TTTNEIFYCT FRRLDPEENH TAELVIPELP LAHPPNERTH 240
LVILGAILLC LGVALTFIFR LRKGRMMDVK KCGIQDTNSK KQSDTHLEET 290
(SEQ ID NO:9) or

FTVTVPKDLY VVEYGSNMTI ECKFPVEKQL DLAALIVYWE MEDKNIIQFV HGEEDLKVQH 60
SSYRQRARLL KDQLSLGNAA LQITDVKLQD AGVYRCMISY GGADYKRITV KVNAPYNKIN 120
QRILVVDPVT SEHELTCQAE GYPKAEVIWT SSDHQVLSGK TTTTNSKREE KLFNVTSTLR 180
INTTTNEIFY CTFRRLDPEE NHTAELVIPE LPLAHPPNER THLVILGAIL LCLGVALTFI 240
FRLRKGRMMD VKKCGIQDTN SKKQSDTHLE ET 272
(SEQ ID NO:10).

SEQ ID NOs: 7 and 9 each contain a signal peptide.

### C. B7.1 and PD-1 Based PD-1 Antagonists

### 1. B7.1 and PD-1 Based PD-1 Antagonists that Bind to PD-L1 and PD-L2

Other useful polypeptides include the PD-1 receptor protein, or soluble fragments thereof, which can bind to the PD-1 ligands, such as PD-L1 or PD-L2, and prevent binding to the endogenous PD-1 receptor, thereby preventing inhibitory signal transduction. Such fragments also include the soluble ECD portion of the PD-1 protein that optionally includes mutations, such as the A99L mutation, that increases binding to the natural ligands. PD-L1 has also been shown to bind the protein B7.1 (Butte, et al., Immunity, 27(1): 111-122 (2007)). Therefore, B7.1 or soluble fragments thereof, which can bind to the PD-L1 ligand and prevent binding to the endogenous PD-1 receptor, thereby preventing inhibitory signal transduction, are also useful.

### 2. Exemplary B7.1 Polypeptide PD-1

### Antagonists

Murine B7.1 polypeptides can have at least 80%, 85%, 90%, 95%, 99% or 100% sequence identity to:

MACNCQLMQD TPLLKFPCPR LILLFVLLIR LSQVSSDVDE QLSKSVKDKV LLPCRYNSPH 60
EDESEDRIYW QKHDKVVLSV IAGKLKVWPE YKNRTLYDNT TYSLIILGLV LSDRGTYSCV 120
VQKKERGTYE VKHLALVKLS IKADFSTPNI TESGNPSADT KRITCFASGG FPKPRFSWLE 180
NGRELPGINT TISQDPESEL YTISSQLDFN TTRNHTIKCL IKYGDAHVSE DFTWEKPPED 240
PPDSKNTLVL FGAGFGAVIT VVVIVVIIKC FCKHRSCFRR NEASRETNNS LTFGPEEALA 300
EQTVFL 306
(SEQ ID NO:11) or

VDEQLSKSVK DKVLLPCRYN SPHEDESEDR IYWQKHDKVV LSVIAGKLKV WPEYKNRTLY 60
DNTTYSLIIL GLVLSDRGTY SCVVQKKERG TYEVKHLALV KLSIKADFST PNITESGNPS 120
ADTKRITCFA SGGFPKPRFS WLENGRELPG INTTISQDPE SELYTISSQL DFNTTRNHTI 180
KCLIKYGDAH VSEDFTWEKP PEDPPDSKNT LVLFGAGFGA VITVVVIVVI IKCFCKHRSC 240
FRRNEASRET NNSLTFGPEE ALAEQTVFL 269
(SEQ ID NO:12).

Human B7.1 polypeptides can have at least 80%, 85%, 90%, 95%, 99% or 100% sequence identity to:

MGHTRRQGTS PSKCPYLNFF QLLVLAGLSH FCSGVIHVTK EVKEVATLSC GHNVSVEELA 60
QTRIYWQKEK KMVLTMMSGD MNIWPEYKNR TIFDITNNLS IVILALRPSD EGTYECVVLK 120
YEKDAFKREH LAEVTLSVKA DFPTPSISDF EIPTSNIRRI ICSTSGGFPE PHLSWLENGE 180
ELNAINTTVS QDPETELYAV SSKLDFNMTT NHSFMCLIKY GHLRVNQTFN WNTTKQEHFP 240
DNLLPSWAIT LISVNGIFVI CCLTYCFAPR CRERRRNERL RRESVRPV 288
(SEQ ID NO:13) or

VIHVTKEVKE VATLSCGHNV SVEELAQTRI YWQKEKKMVL TMMSGDMNIW PEYKNRTIFD 60
ITNNLSIVIL ALRPSDEGTY ECVVLKYEKD AFKREHLAEV TLSVKADFPT PSISDFEIPT 120
SNIRRIICST SGGFPEPHLS WLENGEELNA INTTVSQDPE TELYAVSSKL DFNMTTNHSF 180
MCLIKYGHLR VNQTFNWNTT KQEHFPDNLL PSWAITLISV NGIFVICCLT YCFAPRCRER 240
RRNERLRRES VRPV 254
(SEQ ID NO:14).

SEQ ID NOs: 11 and 13 each contain a signal peptide.

### 3. Exemplary PD-1 Polypeptide PD-1

### Antagonists

Human PD-1 polypeptides can have at least 80%, 85%, 90%, 95%, 99% or 100% sequence identity to:

MQIPQAPWPV VWAVLQLGWR PGWFLDSPDR PWNPPTFFPA LLVVTEGDNA TFTCSFSNTS 60
ESFVLNWYRM SPSNQTDKLA AFPEDRSQPG QDCRFRVTQL PNGRDFHMSV VRARRNDSGT 120
YLCGAISLAP KAQIKESLRA ELRVTERRAE VPTAHPSPSP RPAGQFQTLV VGVVGGLLGS 180
LVLLVWVLAV ICSRAARGTI GARRTGQPLK EDPSAVPVFS VDYGELDFQW REKTPEPPVP 240
CVPEQTEYAT IVFPSGMGTS SPARRGSADG PRSAQPLRPE DGHCSWPL 288
(SEQ ID NO:15)

Non-human primate (*Cynomolgus*) PD-1 polypeptides can have at least 80%, 85%, 90%, 95%, 99% or 100% sequence identity to:

MQIPQAPWPV VWAVLQLGWR PGWFLESPDR PWNAPTFSPA LLLVTEGDNA TFTCSFSNAS 60
ESFVLNWYRM SPSNQTDKLA AFPEDRSQPG QDCRFRVTRL PNGRDFHMSV VRARRNDSGT 120
YLCGAISLAP KAQIKESLRA ELRVTERRAE VPTAHPSPSP RPAGQFQTLV VGVVGGLLGS 180
LVLLVWVLAV ICSRAARGTI GARRTGQPLK EDPSAVPVFS VDYGELDFQW REKTPEPPVP 240
CVPEQTEYAT IVFPSGMGTS SPARRGSADG PRSAQPLRPE DGHCSWPL 288
(SEQ ID NO:16)

SEQ ID NOs: 15 and 16 each contain a signal peptide.

### D. Fragments of PD-1 Antagonist Polypeptides

The PD-1 antagonist polypeptides can be full-length polypeptides, or can be a fragment of a full length polypeptide. As used herein, a fragment of a PD-1 antagonist polypeptide refers to any subset of the polypeptide that is a shorter polypeptide of the full length protein.

Useful fragments are those that retain the ability to bind to their natural ligands. A PD-1 antagonist polypeptide that is a fragment of full-length PD-1 antagonist polypeptide typically has at least 20 percent, 30 percent, 40 percent, 50 percent, 60 percent, 70 percent, 80 percent, 90 percent, 95 percent, 98 percent, 99 percent, 100 percent, or even more than 100 percent of the ability to bind its natural ligand(s) as compared to the full-length PD-1 antagonist polypeptide.

For example, useful fragments of PD-L2 and PD-L1 are those that retain the ability to bind to PD-1. PD-L2 and PD-L1 fragments typically have at least 20 percent, 30 percent, 40 percent, 50 percent, 60 percent, 70 percent, 80 percent, 90 percent, 95 percent, 98 percent, 99 percent, 100 percent, or even more than 100 percent of the ability to bind to PD-1 as compared to full length PD-L2 and PD-L1.

Fragments of PD-1 antagonist polypeptides include soluble fragments. Soluble PD-1 antagonist polypeptide fragments are fragments of PD-1 antagonist polypeptides that may be shed, secreted or otherwise extracted from the producing cells. Soluble fragments of PD-1 antagonist polypeptides include some or all of the extracellular domain of the polypeptide, and lack some or all of the intracellular and/or transmembrane domains. In one embodiment, PD-1 antagonist polypeptide fragments include the entire extracellular domain of the PD-1 antagonist polypeptide. It will be appreciated that the extracellular domain can include 1, 2, 3, 4, or 5 amino acids from the transmembrane domain. Alternatively, the extracellular domain can have 1, 2, 3, 4, or 5 amino acids removed from the C-terminus, N-terminus, or both.

Generally, the PD-1 antagonist polypeptides or fragments thereof are expressed from nucleic acids that include sequences that encode a signal sequence. The signal sequence is generally cleaved from the immature polypeptide to produce the mature polypeptide lacking the signal sequence. The signal sequence of PD-1 antagonist polypeptides can be replaced by the signal sequence of another polypeptide using standard molecule biology techniques to affect the expression levels, secretion, solubility, or other property of the polypeptide. The signal sequence that is used to replace the PD-1 antagonist polypeptide signal sequence can be any known in the art.

### 1. PD-L2 extracellular domains

### a. Human PD-L2 extracellular domains

In one embodiment, the PD-1 antagonist polypeptide includes the extracellular domain of human PD-L2 or a fragment thereof. The PD-1 antagonist polypeptide can be encoded by a nucleotide sequence having at least 80%, 85%, 90%, 95%, 99%, or 100% sequence identity to:

atgatctttc ttctcttgat gctgtctttg gaattgcaac ttcaccaaat cgcggccctc 60
tttactgtga ccgtgccaaa agaactgtat atcattgagc acgggtccaa tgtgaccctc 120
gaatgtaact ttgacaccgg cagccacgtt aacctggggg ccatcactgc cagcttgcaa 180
aaagttgaaa acgacacttc acctcaccgg gagagggcaa ccctcttgga ggagcaactg 240
ccattgggga aggcctcctt tcatatccct caggtgcagg ttcgggatga gggacagtac 300

cagtgcatta ttatctacgg cgtggcttgg gattacaagt atctgaccct gaaggtgaaa 360
gcgtcctatc ggaaaattaa cactcacatt cttaaggtgc cagagacgga cgaggtggaa 420
ctgacatgcc aagccaccgg ctacccgttg gcagaggtca gctggcccaa cgtgagcgta 480
cctgctaaca cttctcattc taggacaccc gagggcctct accaggttac atccgtgctc 540
cgcctcaaac cgcccccagg ccggaatttt agttgcgtgt tttggaatac ccacgtgcga 600
gagctgactc ttgcatctat tgatctgcag tcccagatgg agccacggac tcatccaact 660
tgg 663
(SEQ ID NO:17).

In another embodiment, the PD-1 antagonist polypeptide can have at least 80%, 85%, 90%, 95%, 99%, or 100% sequence identity to the human

amino acid sequence: MIFLLLMLSL ELQLHQIAAL FTVTVPKELY IIEHGSNVTL
MIFLLLMLSL ELQLHQIAAL FTVTVPKELY IIEHGSNVTL ECNFDTGSHV NLGAITASLQ 60
KVENDTSPHR ERATLLEEQL PLGKASFHIP QVQVRDEGQY QCIIIYGVAW DYKYLTLKVK 120
ASYRKINTHI LKVPETDEVE LTCQATGYPL AEVSWPNVSV PANTSHSRTP EGLYQVTSVL 180
RLKPPPGRNF SCVFWNTHVR ELTLASIDLQ SQMEPRTHPT W 221
(SEQ ID NO:18).

It will be appreciated that the signal sequence will be removed in the mature protein. Additionally, it will be appreciated that signal peptides from other organisms can be used to enhance the secretion of the protein from a host during manufacture. SEQ ID NO:19 provides the human amino acid sequence of SEQ ID NO:18 without the signal sequence:

LFTVTVPKEL YIIEHGSNVT LECNFDTGSH VNLGAITASL QKVENDTSPH RERATLLEEQ 60
LPLGKASFHI PQVQVRDEGQ YQCIIIYGVA WDYKYLTLKV KASYRKINTH ILKVPETDEV 120
ELTCQATGYP LAEVSWPNVS VPANTSHSRT PEGLYQVTSV LRLKPPPGRN FSCVFWNTHV 180
RELTLASIDL QSQMEPRTHP TW 202
(SEQ ID NO:19).

In another embodiment, the PD-1 antagonist polypeptide includes the IgV domain of human PD-L2. The first fusion partner can be encoded by a nucleotide sequence having at least 80%, 85%, 90%, 95%, 99%, or 100% sequence identity to:

tttactgtga ccgtgccaaa agaactgtat atcattgagc acgggtccaa tgtgaccctc 60
gaatgtaact ttgacaccgg cagccacgtt aacctggggg ccatcactgc cagcttgcaa 120
aaagttgaaa acgacacttc acctcaccgg gagagggcaa ccctcttgga ggagcaactg 180
ccattgggga aggcctcctt tcatatccct caggtgcagg ttcgggatga gggacagtac 240
cagtgcatta ttatctacgg cgtggcttgg gattacaagt atctgaccct gaag 294
(SEQ ID NO:20).

The PD-1 antagonist polypeptide can have at least 80%, 85%, 90%, 95%, 99%, or 100% sequence identity to the human amino acid sequence: FTVTVPKELY IIEHGSNVTL ECNFDTGSHV NLGAITASLQ KVENDTSPHR ERATLLEEQL 60 PLGKASFHIP QVQVRDEGQY QCIIIYGVAW DYKYLTLK 98 (SEQ ID NO:21), also referred to as PD-L2V.

### b. Non-human primate PD-L2 extracellular domains

In one embodiment, the PD-1 antagonist polypeptide includes the extracellular domain of non-human primate (*Cynomolgus*) PD-L2 or a fragment thereof. The PD-1 antagonist polypeptide can be encoded by a nucleotide sequence having at least 80%, 85%, 90%, 95%, 99%, or 100% sequence identity to:

atgatcttcc tcctgctaat gttgagcctg gaattgcagc ttcaccagat agcagcttta 60
ttcacagtga cagtccctaa ggaactgtac ataatagagc atggcagcaa tgtgaccctg 120
gaatgcaact ttgacactgg aagtcatgtg aaccttggag caataacagc cagtttgcaa 180
aaggtggaaa atgatacatc cccacaccgt gaaagagcca ctttgctgga ggagcagctg 240
cccctaggga aggcctcgtt ccacatacct caagtccaag tgagggacga aggacagtac 300
caatgcataa tcatctatgg ggtcgcctgg gactacaagt acctgactct gaaagtcaaa 360
gcttcctaca ggaaaataaa cactcacatc ctaaaggttc cagaaacaga tgaggtagag 420
ctcacctgcc aggctacagg ttatcctctg gcagaagtat cctggccaaa cgtcagcgtt 480
cctgccaaca ccagccactc caggacccct gaaggcctct accaggtcac cagtgttctg 540
cgcctaaagc caccccctgg cagaaacttc agctgtgtgt tctggaatac tcacgtgagg 600
gaacttactt tggccagcat tgaccttcaa agtcagatgg aacccaggac ccatccaact 660
tgg 663
(SEQ ID NO:22).

In another embodiment, the PD-1 antagonist polypeptide can have at least 80%, 85%, 90%, 95%, 99%, or 100% sequence identity to the non-human primate amino acid sequence:

MIFLLLMLSL ELQLHQIAAL FTVTVPKELY IIEHGSNVTL ECNFDTGSHV NLGAITASLQ 60
KVENDTSPHR ERATLLEEQL PLGKASFHIP QVQVRDEGQY QCIIIYGVAW DYKYLTLKVK 120
ASYRKINTHI LKVPETDEVE LTCQATGYPL AEVSWPNVSV PANTSHSRTP EGLYQVTSVL 180
RLKPPPGRNF SCVFWNTHVR ELTLASIDLQ SQMEPRTHPT W 221
(SEQ ID NO:23).

The signal sequence will be removed in the mature protein. Additionally, signal peptides from other organisms can be used to enhance the secretion of the fusion protein from a host during manufacture. SEQ ID NO:24 provides the non-human primate amino acid sequence of SEQ ID NO:23 without the signal sequence:

LFTVTVPKEL YIIEHGSNVT LECNFDTGSH VNLGAITASL QKVENDTSPH RERATLLEEQ 60
LPLGKASFHI PQVQVRDEGQ YQCIIIYGVA WDYKYLTLKV KASYRKINTH ILKVPETDEV 120
ELTCQATGYP LAEVSWPNVS VPANTSHSRT PEGLYQVTSV LRLKPPPGRN FSCVFWNTHV 180
RELTLASIDL QSQMEPRTHP TW 202

(SEQ ID NO:24).

In another embodiment, the PD-1 antagonist polypeptide includes the IgV domain of non-human primate PD-L2. The first fusion partner can be encoded by a nucleotide sequence having at least 80%, 85%, 90%, 95%, 99%, or 100% sequence identity to:

ttcacagtga cagtccctaa ggaactgtac ataatagagc atggcagcaa tgtgaccctg 60
gaatgcaact ttgacactgg aagtcatgtg aaccttggag caataacagc cagtttgcaa 120
aaggtggaaa atgatacatc cccacaccgt gaaagagcca ctttgctgga ggagcagctg 180
cccctaggga aggcctcgtt ccacatacct caagtccaag tgagggacga aggacagtac 240
caatgcataa tcatctatgg ggtcgcctgg gactacaagt acctgactct gaaa 294
(SEQ ID NO:25).

The PD-1 antagonist polypeptide can have at least 80%, 85%, 90%, 95%, 99%, or 100% sequence identity to the non-human primate amino acid sequence: 
FTVTVPKELY IIEHGSNVTL ECNFDTGSHV NLGAITASLQ KVENDTSPHR ERATLLEEQL 60
PLGKASFHIP QVQVRDEGQY QCIIIYGVAW DYKYLTLK 98
 (SEQ ID NO:26), also referred to as PD-L2V.

### d. Murine PD-L2 extracellular domains

In one embodiment, the PD-1 antagonist polypeptide includes the extracellular domain of murine PD-L2 or a fragment thereof. The PD-1 antagonist polypeptide can be encoded by a nucleotide sequence having at least 80%, 85%, 90%, 95%, 99%, or 100% sequence identity to:

atgctgctcc tgctgccgat actgaacctg agcttacaac ttcatcctgt agcagcttta 60
ttcaccgtga cagcccctaa agaagtgtac accgtagacg tcggcagcag tgtgagcctg 120
gagtgcgatt ttgaccgcag agaatgcact gaactggaag ggataagagc cagtttgcag 180
aaggtagaaa atgatacgtc tctgcaaagt gaaagagcca ccctgctgga ggagcagctg 240
cccctgggaa aggctttgtt ccacatccct agtgtccaag tgagagattc cgggcagtac 300
cgttgcctgg tcatctgcgg ggccgcctgg gactacaagt acctgacggt gaaagtcaaa 360
gcttcttaca tgaggataga cactaggatc ctggaggttc caggtacagg ggaggtgcag 420
cttacctgcc aggctagagg ttatccccta gcagaagtgt cctggcaaaa tgtcagtgtt 480
cctgccaaca ccagccacat caggaccccc gaaggcctct accaggtcac cagtgttctg 540
cgcctcaagc ctcagcctag cagaaacttc agctgcatgt tctggaatgc tcacatgaag 600
gagctgactt cagccatcat tgaccctctg agtcggatgg aacccaaagt ccccagaacg 660
tgg 663
(SEQ ID NO:27).

In another embodiment, the PD-1 antagonist polypeptide can have at least 80%, 85%, 90%, 95%, 99%, or 100% sequence identity to the murine amino acid sequence:

MLLLLPILNL SLQLHPVAAL FTVTAPKEVY TVDVGSSVSL ECDFDRRECT ELEGIRASLQ 60
KVENDTSLQS ERATLLEEQL PLGKALFHIP SVQVRDSGQY RCLVICGAAW DYKYLTVKVK 120
ASYMRIDTRI LEVPGTGEVQ LTCQARGYPL AEVSWQNVSV PANTSHIRTP EGLYQVTSVL 180
RLKPQPSRNF SCMFWNAHMK ELTSAIIDPL SRMEPKVPRT W 221
(SEQ ID NO:28).

The signal sequence will be removed in the mature protein. Additionally, signal peptides from other organisms can be used to enhance the secretion of the protein from a host during manufacture. SEQ ID NO:29 provides the murine amino acid sequence of SEQ ID NO:28 without the signal sequence:

LFTVTAPKEV YTVDVGSSVS LECDFDRREC TELEGIRASL QKVENDTSLQ SERATLLEEQ 60
LPLGKALFHI PSVQVRDSGQ YRCLVICGAA WDYKYLTVKV KASYMRIDTR ILEVPGTGEV 120
QLTCQARGYP LAEVSWQNVS VPANTSHIRT PEGLYQVTSV LRLKPQPSRN FSCMFWNAHM 180
KELTSAIIDP LSRMEPKVPR TW 202
(SEQ ID NO:29).

In another embodiment, the PD-1 antagonist polypeptide includes the IgV domain of murine PD-L2. The first fusion partner can be encoded by a nucleotide sequence having at least 80%, 85%, 90%, 95%, 99%, or 100% sequence identity to:

ttcaccgtga cagcccctaa agaagtgtac accgtagacg tcggcagcag tgtgagcctg 60
gagtgcgatt ttgaccgcag agaatgcact gaactggaag ggataagagc cagtttgcag 120
aaggtagaaa atgatacgtc tctgcaaagt gaaagagcca ccctgctgga ggagcagctg 180
cccctgggaa aggctttgtt ccacatccct agtgtccaag tgagagattc cgggcagtac 240
cgttgcctgg tcatctgcgg ggccgcctgg gactacaagt acctgacggt gaaa 294
(SEQ ID NO:30).

The PD-1 antagonist polypeptide can have at least 80%, 85%, 90%, 95%, 99%, or 100% sequence identity to the murine amino acid sequence:

FTVTAPKEVY TVDVGSSVSL ECDFDRRECT ELEGIRASLQ KVENDTSLQS ERATLLEEQL 60
PLGKALFHIP SVQVRDSGQY RCLVICGAAW DYKYLTVK 98
(SEQ ID NO:31), also referred to as PD-L2V.

### d. PD-L2 extracellular domain fragments

The PD-L2 extracellular domain can contain one or more amino acids from the signal peptide or the putative transmembrane domain of PD-L2. During secretion, the number of amino acids of the signal peptide that are cleaved can vary depending on the expression system and the host. Additionally, fragments of PD-L2 extracellular domain missing one or more amino acids from the C-terminus or the N-terminus that retain the ability to bind to PD-1 can be used.

Exemplary suitable fragments of murine PD-L2 that can be used as a first fusion partner include, but are not limited to, the following:
24-221, 24-220, 24-219, 24-218, 24-217, 24-216, 24-215,
23-221, 23-220, 23-219, 23-218, 23-217, 23-216, 23-215,
22-221, 22-220, 22-219, 22-218, 22-217, 22-216, 22-215,
21-221, 21-220, 21-219, 21-218, 21-217, 21-216, 21-215,
20-221, 20-220, 20-219, 20-218, 20-217, 20-216, 20-215,
19-221, 19-220, 19-219, 19-218, 19-217, 19-216, 19-215,
18-221, 18-220, 18-219, 18-218, 18-217, 18-216, 18-215,
17-221, 17-220, 17-219, 17-218, 17-217,17-216, 17-215,
16-221, 16-220, 16-219, 16-218, 16-217, 16-216, 16-215, of SEQ ID NO:53.

Additional suitable fragments of murine PD-L2 include, but are not limited to, the following:
20-221, 33-222, 33-223, 33-224, 33-225, 33-226, 33-227,
21-221, 21-222, 21-223, 21-224, 21-225, 21-226, 21-227,
22-221, 22-222, 22-223, 22-224, 22-225, 22-226, 22-227,
23-221, 23-222, 23-223, 23-224, 23-225, 23-226, 23-227,
24-221, 24-222, 24-223, 24-224, 24-225, 24-226, 24-227,
of SEQ ID NO:1, optionally with one to five amino acids of a signal peptide attached to the N-terminal end. The signal peptide may be any disclosed herein, including the signal peptide contained within SEQ ID NO:1, or may be any signal peptide known in the art.

Exemplary suitable fragments of human PD-L2 that can be used as a first fusion partner include, but are not limited to, the following:
24-221, 24-220, 24-219, 24-218, 24-217, 24-216, 24-215,
23-221, 23-220, 23-219, 23-218, 23-217, 23-216, 23-215,
22-221, 22-220, 22-219, 22-218, 22-217, 22-216, 22-215,
21-221, 21-220, 21-219, 21-218, 21-217, 21-216, 21-215,
20-221, 20-220, 20-219, 20-218, 20-217, 20-216, 20-215,
19-221, 19-220, 19-219, 19-218, 19-217, 19-216, 19-215,
18-221, 18-220, 18-219, 18-218, 18-217, 18-216, 18-215,
17-221, 17-220, 17-219, 17-218, 17-217, 17-216, 17-215,
16-221, 16-220, 16-219, 16-218, 16-217, 16-216, 16-215, of SEQ ID NO:56.

Additional suitable fragments of human PD-L2 include, but are not limited to, the following:
20-221, 33-222, 33-223, 33-224, 33-225, 33-226, 33-227,
21-221, 21-222, 21-223, 21-224, 21-225, 21-226, 21-227,
22-221, 22-222, 22-223, 22-224, 22-225, 22-226, 22-227,
23-221, 23-222, 23-223, 23-224, 23-225, 23-226, 23-227,
24-221, 24-222, 24-223, 24-224, 24-225, 24-226, 24-227, of SEQ ID NO:3, optionally with one to five amino acids of a signal peptide attached to the N-terminal end. The signal peptide may be any disclosed herein, including the signal peptide contained within SEQ ID NO:3, or may be any signal peptide known in the art.

Exemplary suitable fragments of non-human primate PD-L2 that can be used as a first fusion partner include, but are not limited to, the following:
24-221, 24-220, 24-219, 24-218, 24-217, 24-216, 24-215,
23-221, 23-220, 23-219, 23-218, 23-217, 23-216, 23-215,
22-221, 22-220, 22-219, 22-218, 22-217, 22-216, 22-215,
21-221, 21-220, 21-219, 21-218, 21-217, 21-216, 21-215,
20-221, 20-220, 20-219, 20-218, 20-217, 20-216, 20-215,
19-221, 19-220, 19-219, 19-218, 19-217, 19-216, 19-215,
18-221, 18-220, 18-219, 18-218, 18-217, 18-216, 18-215,
17-221, 17-220, 17-219, 17-218, 17-217, 17-216, 17-215,
16-221,16-220, 16-219, 16-218, 16-217, 16-216, 16-215, of SEQ ID NO:5.

Additional suitable fragments of non-human primate PD-L2 include, but are not limited to, the following:
20-221, 33-222, 33-223, 33-224, 33-225, 33-226, 33-227,
21-221, 21-222, 21-223, 21-224, 21-225, 21-226, 21-227,
22-221, 22-222, 22-223, 22-224, 22-225, 22-226, 22-227,
23-221, 23-222, 23-223, 23-224, 23-225, 23-226, 23-227,
24-221, 24-222, 24-223, 24-224, 24-225, 24-226, 24-227, of SEQ ID NO:5, optionally with one to five amino acids of a signal peptide attached to the N-terminal end. The signal peptide may be any disclosed herein, including the signal peptide contained within SEQ ID NO:5, or may be any signal peptide known in the art.

PD-L2 proteins also include a PD-1 binding fragment of amino acids 20-121 of SEQ ID NO:3 (human full length), or amino acids 1-102 of SEQ ID NO:23 (extracellular domain or ECD). In specific embodiments thereof, the PD-L2 polypeptide or PD-1 binding fragment also incorporates amino acids WDYKY at residues 110-114 of SEQ ID NO:3 or WDYKY at residues 91-95 of SEQ ID NO:23. By way of non-limiting examples, such a PD-1 binding fragment comprises at least 10, at least 20, at least 30, at least 40, at least 50, at least 60, at least 70, at least 75, at least 80, at least 85, at least 90, at least 95, or at least 100 contiguous amino acids of the sequence of amino acids 20-121 of SEQ ID NO:3, wherein a preferred embodiment of each such PD-1 binding fragment would comprise as a sub-fragment the amino acids WDYKY found at residues 110-114 of SEQ ID NO:3 or WDYKY at residues 91-95 of SEQ ID NO:23

### 2. PD-L1 extracellular domains

In one embodiment, the variant PD-L1 polypeptide includes all or part of the extracellular domain. The amino acid sequence of a representative extracellular domain of PD-L1 can have 80%, 85%, 90%, 95%, or 99% sequence identity to

FTVTVPKDLY VVEYGSNMTI ECKFPVEKQL DLAALIVYWE MEDKNIIQFV HGEEDLKVQH 60
SSYRQRARLL KDQLSLGNAA LQITDVKLQD AGVYRCMISY GGADYKRITV KVNAPYNKIN 120
QRILVVDPVT SEHELTCQAE GYPKAEVIWT SSDHQVLSGK TTTTNSKREE KLFNVTSTLR 180
INTTTNEIFY CTFRRLDPEE NHTAELVIPE LPLAHPPNER 220
(SEQ ID NO:32).

The transmembrane domain of PD-L1 begins at amino acid position 239 of SEQ ID NO:9. It will be appreciated that the suitable fragments of PD-L1 can include 1, 2, 3, 4, 5, 6, 7, 8, 9, or 10 contiguous amino acids of a signal peptide sequence, for example SEQ ID NO:9 or variants thereof, 1, 2, 3, 4, 5, 6, 7, 8, 9, or 10 amino acids of the transmembrane domain, or combinations thereof.

The extracellular domain of murine PD-L1 has the following amino acid sequence

FTITAPKDLY VVEYGSNVTM ECRFPVEREL DLLALVVYWE KEDEQVIQFV AGEEDLKPQH 60
SNFRGRASLP KDQLLKGNAA LQITDVKLQD AGVYCCIISY GGADYKRITL KVNAPYRKIN 120
QRISVDPATS EHELICQAEG YPEAEVIWTN SDHQPVSGKR SVTTSRTEGM LLNVTSSLRV 180
NATANDVFYC TFWRSQPGQN HTAELIIPEL PATHPPQNRT HWVLLGSILL FLIVVSTVL 239
(SEQ ID NO:33).

The transmembrane domain of the murine PD-L1 begins at amino acid position 240 of SEQ ID NO:7. In certain embodiments the PD-L1 polypeptide includes the extracellular domain of murine PD-L1 with 1, 2, 3, 4, 5, 6, 7, 8, 9, or 10 contiguous amino acids of a signal peptide, 1, 2, 3, 4, 5, 6, 7, 8, 9, or 10 contiguous amino acids of the transmembrane domain, or combinations thereof.

### 3. B7.1 extracellular domains

### a. Murine B7.1 extracellular domains

In one embodiment, the PD-1 antagonist polypeptide includes the extracellular domain of murine B7.1 or a fragment thereof. The PD-1 antagonist polypeptide can be encoded by a nucleotide sequence having at least 80%, 85%, 90%, 95%, 99%, or 100% sequence identity to:

atggcttgca attgtcagtt gatgcaggat acaccactcc tcaagtttcc atgtccaagg 60
ctcattcttc tctttgtgct gctgattcgt ctttcacaag tgtcttcaga tgttgatgaa 120
caactgtcca agtcagtgaa agataaggta ttgctgcctt gccgttacaa ctctcctcat 180
gaagatgagt ctgaagaccg aatctactgg caaaaacatg acaaagtggt gctgtctgtc 240
attgctggga aactaaaagt gtggcccgag tataagaacc ggactttata tgacaacact 300
acctactctc ttatcatcct gggcctggtc ctttcagacc ggggcacata cagctgtgtc 360
gttcaaaaga aggaaagagg aacgtatgaa gttaaacact tggctttagt aaagttgtcc 420
atcaaagctg acttctctac ccccaacata actgagtctg gaaacccatc tgcagacact 480
aaaaggatta cctgctttgc ttccgggggt ttcccaaagc ctcgcttctc ttggttggaa 540
aatggaagag aattacctgg catcaatacg acaatttccc aggatcctga atctgaattg 600
tacaccatta gtagccaact agatttcaat acgactcgca accacaccat taagtgtctc 660
attaaatatg gagatgctca cgtgtcagag gacttcacct gggaaaaacc cccagaagac 720
cctcctgata gcaagaac 738
(SEQ ID NO:34).

In another embodiment, the PD-1 antagonist polypeptide can have at least 80%, 85%, 90%, 95%, 99%, or 100% sequence identity to the murine amino acid sequence:

MACNCQLMQD TPLLKFPCPR LILLFVLLIR LSQVSSDVDE QLSKSVKDKV LLPCRYNSPH 60
EDESEDRIYW QKHDKVVLSV IAGKLKVWPE YKNRTLYDNT TYSLIILGLV LSDRGTYSCV 120
VQKKERGTYE VKHLALVKLS IKADFSTPNI TESGNPSADT KRITCFASGG FPKPRFSWLE 180
NGRELPGINT TISQDPESEL YTISSQLDFN TTRNHTIKCL IKYGDAHVSE DFTWEKPPED 240
PPDSKN 246
(SEQ ID NO:35).

The signal sequence will be removed in the mature protein. Additionally, signal peptides from other organisms can be used to enhance the secretion of the protein from a host during manufacture. SEQ ID NO:36 provides the murine amino acid sequence of SEQ ID NO:35 without the signal sequence:

VDEQLSKSVK DKVLLPCRYN SPHEDESEDR IYWQKHDKVV LSVIAGKLKV WPEYKNRTLY 60
DNTTYSLIIL GLVLSDRGTY SCVVQKKERG TYEVKHLALV KLSIKADFST PNITESGNPS 120
ADTKRITCFA SGGFPKPRFS WLENGRELPG INTTISQDPE SELYTISSQL DFNTTRNHTI 180
KCLIKYGDAH VSEDFTWEKP PEDPPDSKN 209
(SEQ ID NO:36).

In another embodiment, the PD-1 antagonist polypeptide includes the IgV domain of murine B7.1. The first fusion partner can be encoded by a nucleotide sequence having at least 80%, 85%, 90%, 95%, 99%, or 100% sequence identity to:

gttgatgaac aactgtccaa gtcagtgaaa gataaggtat tgctgccttg ccgttacaac 60
tctcctcatg aagatgagtc tgaagaccga atctactggc aaaaacatga caaagtggtg 120
ctgtctgtca ttgctgggaa actaaaagtg tggcccgagt ataagaaccg gactttatat 180
gacaacacta cctactctct tatcatcctg ggcctggtcc tttcagaccg gggcacatac 240
agctgtgtcg ttcaaaagaa ggaaagagga acgtatgaag ttaaacactt g 291
(SEQ ID NO:37).

The PD-1 antagonist polypeptide can have at least 80%, 85%, 90%, 95%, 99%, or 100% sequence identity to the murine amino acid sequence:

VDEQLSKSVK DKVLLPCRYN SPHEDESEDR IYWQKHDKVV LSVIAGKLKV WPEYKNRTLY 60
DNTTYSLIIL GLVLSDRGTY SCVVQKKERG TYEVKHL 97
(SEQ ID NO:38), also referred to as B7.1V.

### b. Human B7.1 extracellular domains

In one embodiment, the PD-1 antagonist polypeptide includes the extracellular domain of human B7.1 or a fragment thereof. The PD-1 antagonist polypeptide can be encoded by a nucleotide sequence having at least 80%, 85%, 90%, 95%, 99%, or 100% sequence identity to:

atgggccaca cacggaggca gggaacatca ccatccaagt gtccatacct caatttcttt 60
cagctcttgg tgctggctgg tctttctcac ttctgttcag gtgttatcca cgtgaccaag 120

gaagtgaaag aagtggcaac gctgtcctgt ggtcacaatg tttctgttga agagctggca 180
caaactcgca tctactggca aaaggagaag aaaatggtgc tgactatgat gtctggggac 240
atgaatatat ggcccgagta caagaaccgg accatctttg atatcactaa taacctctcc 300
attgtgatcc tggctctgcg cccatctgac gagggcacat acgagtgtgt tgttctgaag 360
tatgaaaaag acgctttcaa gcgggaacac ctggctgaag tgacgttatc agtcaaagct 420
gacttcccta cacctagtat atctgacttt gaaattccaa cttctaatat tagaaggata 480
atttgctcaa cctctggagg ttttccagag cctcacctct cctggttgga aaatggagaa 540
gaattaaatg ccatcaacac aacagtttcc caagatcctg aaactgagct ctatgctgtt 600
agcagcaaac tggatttcaa tatgacaacc aaccacagct tcatgtgtct catcaagtat 660
ggacatttaa gagtgaatca gaccttcaac tggaatacaa ccaagcaaga gcattttcct 720
gataacctgc tc 732
(SEQ ID NO:39).

In another embodiment, the PD-1 antagonist polypeptide can have at least 80%, 85%, 90%, 95%, 99%, or 100% sequence identity to the human

amino acid sequence: MIFLLLMLSL ELQLHQIAAL FTVTVPKELY IIEHGSNVTL
MGHTRRQGTS PSKCPYLNFF QLLVLAGLSH FCSGVIHVTK EVKEVATLSC GHNVSVEELA 60
QTRIYWQKEK KMVLTMMSGD MNIWPEYKNR TIFDITNNLS IVILALRPSD EGTYECVVLK 120
YEKDAFKREH LAEVTLSVKA DFPTPSISDF EIPTSNIRRI ICSTSGGFPE PHLSWLENGE 180
ELNAINTTVS QDPETELYAV SSKLDFNMTT NHSFMCLIKY GHLRVNQTFN WNTTKQEHFP 240
DNL 243
(SEQ ID NO:40).

The signal sequence will be removed in the mature protein. Additionally, signal peptides from other organisms can be used to enhance the secretion of the protein from a host during manufacture. SEQ ID NO:41 provides the human amino acid sequence of SEQ ID NO:40 without the signal sequence:

VIHVTKEVKE VATLSCGHNV SVEELAQTRI YWQKEKKMVL TMMSGDMNIW PEYKNRTIFD 60
ITNNLSIVIL ALRPSDEGTY ECVVLKYEKD AFKREHLAEV TLSVKADFPT PSISDFEIPT 120
SNIRRIICST SGGFPEPHLS WLENGEELNA INTTVSQDPE TELYAVSSKL DFNMTTNHSF 180
MCLIKYGHLR VNQTFNWNTT KQEHFPDNL 209
(SEQ ID NO:41).

In another embodiment, the PD-1 antagonist polypeptide includes the IgV domain of human B7.1. The first fusion partner can be encoded by a nucleotide sequence having at least 80%, 85%, 90%, 95%, 99%, or 100% sequence identity to:

gttatccacg tgaccaagga agtgaaagaa gtggcaacgc tgtcctgtgg tcacaatgtt 60
tctgttgaag agctggcaca aactcgcatc tactggcaaa aggagaagaa aatggtgctg 120
actatgatgt ctggggacat gaatatatgg cccgagtaca agaaccggac catctttgat 180
atcactaata acctctccat tgtgatcctg gctctgcgcc catctgacga gggcacatac 240
gagtgtgttg ttctgaagta tgaaaaagac gctttcaagc gggaacacct ggctgaagtg 300

acg 303
(SEQ ID NO:42).

The PD-1 antagonist polypeptide can have at least 80%, 85%, 90%, 95%, 99%, or 100% sequence identity to the human amino acid sequence: 
VIHVTKEVKE VATLSCGHNV SVEELAQTRI YWQKEKKMVL TMMSGDMNIW PEYKNRTIFD 60
ITNNLSIVIL ALRPSDEGTY ECVVLKYEKD AFKREHLAEV T 101
 (SEQ ID NO:43), also referred to as B7.1V.

### 3. B7.1 extracellular domain fragments

Exemplary suitable fragments of murine B7.1 that can be used as a costimulatory polypeptide domain include, but are not limited to, the following:
42-246, 42-245, 42-244, 42-243, 42-242, 42-241, 42-240,
41-246, 41-245, 41-244, 41-243, 41-242, 41-241, 41-240,
40-246, 40-245, 40-244, 40-243, 40-242, 40-241, 40-240,
39-246, 39-245, 39-244, 39-243, 39-242, 39-241, 39-240,
38-246, 38-245, 38-244, 38-243, 38-242, 38-241, 38-240,
37-246, 37-245, 37-244, 37-243, 37-242, 37-241, 37-240,
36-246, 36-245, 36-244, 36-243, 36-242, 36-241, 36-240,
35-246, 35-245, 35-244, 35-243, 35-242, 35-241, 35-240,
34-246, 34-245, 34-244, 34-243, 34-242, 34-241, 34-240, of SEQ ID NO:11.

Additional suitable fragments of murine B7.1 include, but are not limited to, the following:
38-246, 38-247, 38-248, 38-249, 38-250, 38-251, 38-252,
39-246, 39-247, 39-248, 39-249, 39-250, 39-251, 39-252,
40-246, 40-247, 40-248, 40-249, 40-250, 40-251, 40-252,
41-246, 41-247, 41-248, 41-249, 41-250, 41-251, 41-252,
42-246,42-247,42-248,42-249,42-250,42-251,42-252, of SEQ ID NO:11, optionally with one to five amino acids of a signal peptide attached to the N-terminal end. The signal peptide may be any disclosed herein, including the signal peptide contained within SEQ ID NO:11, or may be any signal peptide known in the art.

Exemplary suitable fragments of human B7.1 that can be used as a costimulatory polypeptide domain include, but are not limited to, the following:
39-243, 39-242, 39-241, 39-240, 39-239, 39-238, 39-237,
38-243, 38-242, 38-241, 38-240, 38-239, 38-238, 38-237,
37-243, 37-242, 37-241, 37-240, 37-239, 37-238, 37-237,
36-243, 36-242, 36-241, 36-240, 36-239, 36-238, 36-237,
35-243, 35-242, 35-241, 35-190, 35-239, 35-238, 35-237,
34-243,34-242,34-241,34-240,34-239,34-238,34-237,
33-243, 33-242, 33-241, 33-240, 33-239, 33-238, 33-237,
32-243, 32-242, 32-241, 32-240, 32-239, 32-238, 32-237,
31-243, 31-242, 31-241, 31-240, 31-239, 31-238, 31-237, of SEQ ID NO:13.

Additional suitable fragments of human B7.1 include, but are not limited to, the following:
35-243, 35-244, 35-245, 35-246, 35-247, 35-248, 35-249,
36-243, 36-244, 36-245, 36-246, 36-247, 36-248, 36-249,
37-243, 37-244, 37-245, 37-246, 37-247, 37-248, 37-249,
38-243, 38-244, 38-245, 38-246, 38-247, 38-248, 38-249,
39-243, 39-244, 39-245, 39-246, 39-247, 39-248, 39-249, of SEQ ID NO:13, optionally with one to five amino acids of a signal peptide attached to the N-terminal end. The signal peptide may be any disclosed herein, including the signal peptide contained within SEQ ID NO:13, or may be any signal peptide known in the art.

### E. Variants

### 1. Variant PD-L2 and PD-L1 PD-1

### Antagonists

Additional PD-1 antagonists include PD-L2 and PD-L1, polypeptides and fragments thereof that are mutated so that they retain the ability to bind to PD-1 under physiological conditions, have increased binding to PD-1, or have decreased ability to promote signal transduction through the PD-1 receptor. One embodiment provides isolated PD-L2 and PD-L1 polypeptides that contain one or more amino acid substitutions, deletions, or insertions that inhibit or reduce the ability of the polypeptide to activate PD-1 and transmit an inhibitory signal to a T cell compared to non-mutated PD-L2 or PD-L1. The PD-L2 and PD-L1 polypeptides may be of any species of origin. In one embodiment, the PD-L2 or PD-L1 polypeptide is from a mammalian species. In a preferred embodiment, the PD-L2 or PD-Llpolypeptide is of human or non-human primate origin.

In another embodiment the variant PD-L2 or PD-L1 polypeptide has the same binding activity to PD-1 as wildtype or non-variant PD-L2 or PD-L1 but does not have or has less than 10% ability to stimulate signal transduction through the PD-1 receptor relative to a non-mutated PD-L2 or PD-L1 polypeptide. In other embodiments, the variant PD-L2 or PD-L1 polypeptide has 10%, 20%, 30%, 40%, 50%, 60%, 70%, 80%, 90%, 100% or more binding activity to PD-1 than wildtype PD-L2 or PD-L1 and has less than 50%, 40%, 30%, 20%, or 10% of the ability to stimulate signal transduction through the PD-1 receptor relative to a non-mutated PD-L2 or PD-L1 polypeptide..

A variant PD-L2 or PD-L1 polypeptide can have any combination of amino acid substitutions, deletions or insertions. In one embodiment, isolated PD-L2 or PD-L1 variant polypeptides have an integer number of amino acid alterations such that their amino acid sequence shares at least 60, 70, 80, 85, 90, 95, 97, 98, 99, 99.5 or 100% identity with an amino acid sequence of a wild type PD-L2 or PD-L1 polypeptide. In a preferred embodiment, B7- H1 variant polypeptides have an amino acid sequence sharing at least 60, 70, 80, 85, 90, 95, 97, 98, 99, 99.5 or 100% identity with the amino acid sequence of a wild type murine, non-human primate or human PD-L2 or PD-L1 polypeptide.

Percent sequence identity can be calculated using computer programs or direct sequence comparison. Preferred computer program methods to determine identity between two sequences include, but are not limited to, the GCG program package, FASTA, BLASTP, and TBLASTN (see, e.g., D. W. Mount, 2001, Bioinformatics: Sequence and Genome Analysis, Cold Spring Harbor Laboratory Press, Cold Spring Harbor, N.Y.). The BLASTP and TBLASTN programs are publicly available from NCBI and other sources. The well-known Smith Waterman algorithm may also be used to determine identity.

Exemplary parameters for amino acid sequence comparison include the following: 1) algorithm from Needleman and Wunsch (J. Mol. Biol., 48:443-453 (1970)); 2) BLOSSUM62 comparison matrix from Hentikoff and Hentikoff (Proc. Natl. Acad. Sci. U.S.A., 89:10915-10919 (1992)) 3) gap penalty = 12; and 4) gap length penalty = 4. A program useful with these parameters is publicly available as the "gap" program (Genetics Computer Group, Madison, Wis.). The aforementioned parameters are the default parameters for polypeptide comparisons (with no penalty for end gaps).

Alternatively, polypeptide sequence identity can be calculated using the following equation: % identity = (the number of identical residues)/(alignment length in amino acid residues)*100. For this calculation, alignment length includes internal gaps but does not include terminal gaps.

Amino acid substitutions in PD-L2 or PD-L1 polypeptides may be "conservative" or "non-conservative". As used herein, "conservative" amino acid substitutions are substitutions wherein the substituted amino acid has similar structural or chemical properties, and "non-conservative" amino acid substitutions are those in which the charge, hydrophobicity, or bulk of the substituted amino acid is significantly altered. Non-conservative substitutions will differ more significantly in their effect on maintaining (a) the structure of the peptide backbone in the area of the substitution, for example, as a sheet or helical conformation, (b) the charge or hydrophobicity of the molecule at the target site, or (c) the bulk of the side chain.

Examples of conservative amino acid substitutions include those in which the substitution is within one of the five following groups: 1) small aliphatic, nonpolar or slightly polar residues (Ala, Ser, Thr, Pro, Gly); 2) polar, negatively charged residues and their amides (Asp, Asn, Glu, Gln); polar, positively charged residues (His, Arg, Lys); large aliphatic, nonpolar residues (Met, Leu, Ile, Val, Cys); and large aromatic resides (Phe, Tyr, Trp). Examples of non-conservative amino acid substitutions are those where 1) a hydrophilic residue, e.g., seryl or threonyl, is substituted for (or by) a hydrophobic residue, e.g., leucyl, isoleucyl, phenylalanyl, valyl, or alanyl; 2) a cysteine or proline is substituted for (or by) any other residue; 3) a residue having an electropositive side chain, e.g., lysyl, arginyl, or histidyl, is substituted for (or by) an electronegative residue, e.g., glutamyl or aspartyl; or 4) a residue having a bulky side chain, e.g., phenylalanine, is substituted for (or by) a residue that does not have a side chain, e.g., glycine.

It is understood, however, that substitutions at the recited amino acid positions can be made using any amino acid or amino acid analog. For example, the substitutions at the recited positions can be made with any of the naturally-occurring amino acids (e.g., alanine, aspartic acid, asparagine, arginine, cysteine, glycine, glutamic acid, glutamine, histidine, leucine, valine, isoleucine, lysine, methionine, proline, threonine, serine, phenylalanine, tryptophan, or tyrosine).

While the substitutions described herein are with respect to mouse, non-human primate and human PD-L2 or PD-L1, it is noted that one of ordinary skill in the art could readily make equivalent alterations in the corresponding polypeptides from other species (e.g., rat, hamster, guinea pig, gerbil, rabbit, dog, cat, horse, pig, sheep or cow). However, since binding has a species-specific component, it is preferable to use human when administering PD-1 antagonists to humans.

In one embodiment, the disclosed isolated variant PD-L2 or PD-L1 polypeptides are antagonists of PD-1 and bind to and block PD-1 without triggering signal transduction through PD-1. By preventing the attenuation of T cells by PD-1 signal transduction, more T cells are available to be activated. Preventing T cell inhibition enhances T cell responses, enhances proliferation of T cells, enhances production and/or secretion of cytokines by T cells, stimulates differentiation and effector functions ofT cells or promotes survival ofT cells relative to T cells not contacted with a PD-1 antagonist. The T cell response that results from the interaction typically is greater than the response in the absence of the PD-1 antagonist polypeptide. The response of the T cell in the absence of the PD-1 antagonist polypeptide can be no response or can be a response significantly lower than in the presence of the PD-1 antagonist polypeptide. The response of the T cell can be an effector (e.g., CTL or antibody-producing B cell) response, a helper response providing help for one or more effector (e.g., CTL or antibody-producing B cell) responses, or a suppressive response.

Methods for measuring the binding affinity between two molecules are well known in the art. Methods for measuring the binding affinity of variant PD-L2 or PD-L1 polypeptides for PD-1 include, but are not limited to, fluorescence activated cell sorting (FACS), surface plasmon resonance, fluorescence anisotropy, affinity chromatography and affinity selection-mass spectrometry.

The variant polypeptides disclosed herein can be full-length polypeptides, or can be a fragment of a full length polypeptide. Preferred fragments include all or part of the extracellular domain of effective to bind to PD-1. As used herein, a fragment refers to any subset of the polypeptide that is a shorter polypeptide of the full length protein.

### 2. Variant B7.1 and PD-1 Antagonists

Additional PD-1 antagonists include B7.1 and PD-1 polypeptides and fragments thereof that are modified so that they retain the ability to bind to PD-L2 and/or PD-L1 under physiological conditions, or have increased binding binding to PD-L2 and/or PD-L1. Such variant PD-1 proteins include the soluble ECD portion of the PD-1 protein that includes mutations, such as the A99L mutation, that increases binding to the natural ligands (Molnar et al., Crystal structure of the complex between programmed death-1 (PD-1) and its ligand PD-L2, PNAS, Vol. 105, pp. 10483-10488 (29 July 2008)). The B7.1 and PD-1 polypeptides may be of any species of origin. In one embodiment, the B7.1 or PD-1 polypeptide is from a mammalian species. In a preferred embodiment, the B7.1 or PD-1 polypeptide is of human or non-human primate origin.

A variant B7.1 or PD-1 polypeptide can have any combination of amino acid substitutions, deletions or insertions. In one embodiment, isolated B7.1 or PD-1 variant polypeptides have an integer number of amino acid alterations such that their amino acid sequence shares at least 60, 70, 80, 85, 90, 95, 97, 98, 99, 99.5 or 100% identity with an amino acid sequence of a wild type B7.1 or PD-1 polypeptide. In a preferred embodiment, B7.1 or PD-1 variant polypeptides have an amino acid sequence sharing at least 60, 70, 80, 85, 90, 95, 97, 98, 99, 99.5 or 100% identity with the amino acid sequence of a wild type murine, non-human primate or human B7.1 or PD.-1 polypeptide.

Amino acid substitutions in B7.1 or PD-1 polypeptides may be "conservative" or "non-conservative". Conservative and non-conservative substitutions are described above.

In one embodiment, the disclosed isolated variant B7.1 or PD-1 polypeptides are antagonists of PD-1 and bind to PD-L2 and/or PD-L1, thereby blocking their binding to endogenous PD-1. By preventing the attenuation of T cells by PD-1 signal transduction, more T cells are available to be activated. Preventing T cell inhibition enhances T cell responses, enhances proliferation of T cells, enhances production and/or secretion of cytokines by T cells, stimulates differentiation and effector functions of T cells or promotes survival of T cells relative to T cells not contacted with a PD-1 antagonist. The T cell response that results from the interaction typically is greater than the response in the absence of the PD-1 antagonist polypeptide. The response of the T cell in the absence of the PD-1 antagonist polypeptide can be no response or can be a response significantly lower than in the presence of the PD-1 antagonist polypeptide. The response of the T cell can be an effector (e.g., CTL or antibody-producing B cell) response, a helper response providing help for one or more effector (e.g., CTL or antibody-producing B cell) responses, or a suppressive response.

The variant polypeptides can be full-length polypeptides, or can be a fragment of a full length polypeptide. Preferred fragments include all or part of the extracellular domain of effective to bind to PD-L2 and/or PD-L1. As used herein, a fragment refers to any subset of the polypeptide that is a shorter polypeptide of the full length protein.

### F. Fusion Proteins

In some embodiments, the PD-1 antagonists are fusion proteins that contain a first polypeptide domain and a second domain. The fusion protein can either bind to a T cell receptor and or preferably the fusion protein can bind to and block inhibitory signal transduction into the T cell, for example by competitively binding to PD-1. By interfering with natural inhibitory ligands binding PD-1, the disclosed compositions effectively block signal transduction through PD-1. Suitable costimulatory polypeptides include variant polypeptides and/or fragments thereof that have increased or decreased binding affinity to inhibitory T cell signal transduction receptors such as PD-1.

The fusion proteins also optionally contain a peptide or polypeptide linker domain that separates the first polypeptide domain from the antigen-binding domain.

Fusion proteins disclosed herein are of formula I:

N-R₁-R₂-R₃-C

wherein "N" represents the N-terminus of the fusion protein, "C" represents the C-terminus of the fusion protein, "R₁" is a PD-L2, PD-L1, B7.1, or PD-1 polypeptide or a antigen-binding targeting domain, "R₂" is a peptide/polypeptide linker domain, and "R₃" is a targeting domain or a antigen-binding targeting domain, wherein "R₃" is a polypeptide domain when "R₁" is a antigen-binding targeting domain, and "R₃" is a antigen-binding targeting domain when "R₁" is a PD-L2, PD-L1, B7.1, or PD-1 polypeptide domain. In a preferred embodiment, "R₁" is a PD-L2, PD-L1, B7.1, or PD-1 polypeptide domain and "R₃" is a antigen-binding targeting domain.

Optionally, the fusion proteins additionally contain a domain that functions to dimerize or multimerize two or more fusion proteins. The domain that functions to dimerize or multimerize the fusion proteins can either be a separate domain, or alternatively can be contained within one of one of the other domains (PD-L2, PD-L1, B7.1, or PD-1 polypeptide domain, antigen-binding targeting domain, or peptide/polypeptide linker domain) of the fusion protein.

The fusion proteins can be dimerized or multimerized. Dimerization or multimerization can occur between or among two or more fusion proteins through dimerization or multimerization domains. Alternatively, dimerization or multimerization of fusion proteins can occur by chemical crosslinking. The dimers or multimers that are formed can be homodimeric/homomultimeric or heterodimeric/heteromultimeric.

The modular nature of the fusion proteins and their ability to dimerize or multimerize in different combinations provides a wealth of options for targeting molecules that function to enhance an immune response to the tumor cell microenvironment or to immune regulatory tissues.

### 1. Antigen-binding targeting domain

The fusion proteins also contain antigen-binding targeting domains. In some embodiments, the targeting domains bind to antigens, ligands or receptors that are specific to immune tissue involved in the regulation of T cell activation in response to infectious disease causing agents.

### Targeting domains

### Antigens, ligands and receptors to target

In one embodiment the fusion proteins contain a domain that specifically binds to an antigen that is expressed by immune tissue involved in the regulation of T cell activation in response to infectious disease causing agents.

### Molecular classes of targeting domains

### Ligands and receptors

In one embodiment, disease targeting domains are ligands that bind to cell surface antigens or receptors that are specifically expressed on diseased cells or are overexpressed on diseased cells as compared to normal tissue. Diseased cells also secrete a large number of ligands into the microenvironment that affect growth and development. Receptors that bind to ligands secreted by diseased cells, including, but not limited to growth factors, cytokines and chemokines, including the chemokines provided above, are suitable for use in the disclosed fusion proteins. Ligands secreted by diseased cells can be targeted using soluble fragments of receptors that bind to the secreted ligands. Soluble receptor fragments are fragments polypeptides that may be shed, secreted or otherwise extracted from the producing cells and include the entire extracellular domain, or fragments thereof.

### Single polypeptide antibodies

In another embodiment, disease-associated targeting domains are single polypeptide antibodies that bind to cell surface antigens or receptors that are specifically expressed on diseased cells or are overexpressed on diseased cells as compared to normal tissue. Single domain antibodies are described above with respect to coinhibitory receptor antagonist domains.

### Fc domains

In another embodiment, disease or disease-associated targeting domains are Fc domains of immunoglobulin heavy chains that bind to Fc receptors expressed on diseased cells. The Fc region a includes the polypeptides containing the constant region of an antibody excluding the first constant region immunoglobulin domain. Thus Fc refers to the last two constant region immunoglobulin domains of IgA, IgD, and IgG, and the last three constant region immunoglobulin domains of IgE and IgM. In a preferred embodiment, the Fc domain is derived from a human or murine immunoglobulin. In a more preferred embodiment, the Fc domain is derived from human IgG1 or murine IgG2a including the C_{H}2 and C_{H}3 regions.

In one embodiment, the hinge, C_{H}2 and C_{H}3 regions of a human immunoglobulin Cγ1 chain are encoded by a nucleic acid having at least 80%, 85%, 90%, 95%, 99% or 100% sequence identity to:

gagcctaagt catgtgacaa gacccatacg tgcccaccct gtcccgctcc agaactgctg 60
gggggaccta gcgttttctt gttcccccca aagcccaagg acaccctcat gatctcacgg 120
actcccgaag taacatgcgt agtagtcgac gtgagccacg aggatcctga agtgaagttt 180
aattggtacg tggacggagt cgaggtgcat aatgccaaaa ctaaacctcg ggaggagcag 240
tataacagta cctaccgcgt ggtatccgtc ttgacagtgc tccaccagga ctggctgaat 300

ggtaaggagt ataaatgcaa ggtcagcaac aaagctcttc ccgcccccat tgaaaagact 360
atcagcaagg ccaagggaca accccgcgag ccccaggttt acacccttcc accttcacga 420
gacgagctga ccaagaacca ggtgtctctg acttgtctgg tcaaaggttt ctatccttcc 480
gacatcgcag tggagtggga gtcaaacggg cagcctgaga ataactacaa gaccacaccc 540
ccagtgcttg atagcgatgg gagctttttc ctctacagta agctgactgt ggacaaatcc 600
cgctggcagc agggaaacgt tttctcttgt agcgtcatgc atgaggccct ccacaaccat 660
tatactcaga aaagcctgag tctgagtccc ggcaaa 696
(SEQ ID NO:44)

The hinge, C_{H}2 and C_{H}3 regions of a human immunoglobulin Cγ1 chain encoded by SEQ ID NO:44 has the following amino acid sequence:

EPKSCDKTHT CPPCPAPELL GGPSVFLFPP KPKDTLMISR TPEVTCVVVD VSHEDPEVKF 60
NWYVDGVEVH NAKTKPREEQ YNSTYRVVSV LTVLHQDWLN GKEYKCKVSN KALPAPIEKT 120
ISKAKGQPRE PQVYTLPPSR DELTKQVSL TCLVKGFYPS DIAVEWESNG QPENNYKTTP 180
PVLDSDGSFF LYSKLTVDKS RWQQGNVFSC SVMHEALHNH YTQKSLSLSP GK 232
(SEQ ID NO:45)

In another embodiment, the hinge, C_{H}2 and C_{H}3 regions of a murine immunoglobulin Cγ2a chain are encoded by a nucleic acid having at least 80%, 85%, 90%, 95%, 99% or 100% sequence identity to:

gagccaagag gtcctacgat caagccctgc ccgccttgta aatgcccagc tccaaatttg 60
ctgggtggac cgtcagtctt tatcttcccg ccaaagataa aggacgtctt gatgattagt 120
ctgagcccca tcgtgacatg cgttgtggtg gatgtttcag aggatgaccc cgacgtgcaa 180
atcagttggt tcgttaacaa cgtggaggtg cataccgctc aaacccagac ccacagagag 240
gattataaca gcaccctgcg ggtagtgtcc gccctgccga tccagcatca ggattggatg 300
agcgggaaag agttcaagtg taaggtaaac aacaaagatc tgccagcgcc gattgaacga 360
accattagca agccgaaagg gagcgtgcgc gcacctcagg tttacgtcct tcctccacca 420
gaagaggaga tgacgaaaaa gcaggtgacc ctgacatgca tggtaactga ctttatgcca 480
gaagatattt acgtggaatg gactaataac ggaaagacag agctcaatta caagaacact 540
gagcctgttc tggattctga tggcagctac tttatgtact ccaaattgag ggtcgagaag 600
aagaattggg tcgagagaaa cagttatagt tgctcagtgg tgcatgaggg cctccataat 660
catcacacca caaagtcctt cagccgaacg cccgggaaa 699
(SEQ ID NO:46)

The hinge, C_{H}2 and C_{H}3 regions of a murine immunoglobulin Cγ2a chain encoded by SEQ ID NO:46 has the following amino acid sequence:

EPRGPTIKPC PPCKCPAPNL LGGPSVFIFP PKIKDVLMIS LSPIVTCVVV DVSEDDPDVQ 60
ISWFVNNVEV HTAQTQTHRE DYNSTLRVVS ALPIQHQDWM SGKEFKCKVN NKDLPAPIER 120
TISKPKGSVR APQVYVLPPP EEEMTKKQVT LTCMVTDFMP EDIYVEWTNN GKTELNYKNT 180
EPVLDSDGSY FMYSKLRVEK KNWVERNSYS CSVVHEGLHN HHTTKSFSRT PGK 233
(SEQ ID NO:47)

In one embodiment, the Fc domain may contain one or more amino acid insertions, deletions or substitutions that enhance binding to specific Fc receptors that specifically expressed on tumors or tumor-associated neovasculature or are overexpressed on tumors or tumor-associated neovasculature relative to normal tissue. Suitable amino acid substitutions include conservative and non-conservative substitutions, as described above.

The therapeutic outcome in patients treated with rituximab (a chimeric mouse/human IgG1 monoclonal antibody against CD20) for non-Hodgkin's lymphoma or Waldenstrom's macroglobulinemia correlated with the individual's expression of allelic variants of Fcγ receptors with distinct intrinsic affinities for the Fc domain of human IgG1. In particular, patients with high affinity alleles of the low affinity activating Fc receptor CD16A (FcγRIIIA) showed higher response rates and, in the cases of non-Hodgkin's lymphoma, improved progression-free survival. In another embodiment, the Fc domain may contain one or more amino acid insertions, deletions or substitutions that reduce binding to the low affinity inhibitory Fc receptor CD32B (FcγRIIB) and retain wild-type levels of binding to or enhance binding to the low affinity activating Fc receptor CD16A (FcγRIIIA). In a preferred embodiment, the Fc domain contains amino acid insertions, deletions or substitutions that enhance binding to CD16A. A large number of substitutions in the Fc domain of human IgG1 that increase binding to CD16A and reduce binding to CD32B are known in the art and are described in Stavenhagen, et al., Cancer Res., 57(18):8882-90 (2007). Exemplary variants of human IgG1 Fc domains with reduced binding to CD32B and/or increased binding to CD16A contain F243L, R929P, Y300L, V305I or P296L substitutions. These amino acid substitutions may be present in a human IgG1 Fc domain in any combination. In one embodiment, the human IgG1 Fc domain variant contains a F243L, R929P and Y300L substitution. In another embodiment, the human IgG1 Fc domain variant contains a F243L, R929P, Y300L, V305I and P296L substitution.

### Glycophosphatidylinositol anchor domain

In another embodiment, disease or disease-associated neovasculature targeting domains are polypeptides that provide a signal for the posttranslational addition of a glycosylphosphatidylinositol (GPI) anchor. GPI anchors are glycolipid structures that are added posttranslationally to the C-terminus of many eukaryotic proteins. This modification anchors the attached protein in the outer leaflet of cell membranes. GPI anchors can be used to attach T cell receptor binding domains to the surface of cells for presentation to T cells. In this embodiment, the GPI anchor domain is C-terminal to the T cell receptor binding domain.

In one embodiment, the GPI anchor domain is a polypeptide that signals for the posttranslational addition addition of a GPI anchor when the polypeptide is expressed in a eukaryotic system. Anchor addition is determined by the GPI anchor signal sequence, which consists of a set of small amino acids at the site of anchor addition (the ω site) followed by a hydrophilic spacer and ending in a hydrophobic stretch (Low, FASEB J., 3:1600-1608 (1989)). Cleavage of this signal sequence occurs in the ER before the addition of an anchor with conserved central components (Low, FASEB J., 3:1600-1608 (1989)) but with variable peripheral moieties (Homans et al., Nature, 333:269-272 (1988)). The C-terminus of a GPI-anchored protein is linked through a phosphoethanolamine bridge to the highly conserved core glycan, mannose(α1-2)mannose(α1-6)mannose(α1-4)glucosamine(α1-6)*myo-*inositol. A phospholipid tail attaches the GPI anchor to the cell membrane. The glycan core can be variously modified with side chains, such as a phosphoethanolamine group, mannose, galactose, sialic acid, or other sugars. The most common side chain attached to the first mannose residue is another mannose. Complex side chains, such as the N-acetylgalactosamine-containing polysaccharides attached to the third mannose of the glycan core, are found in mammalian anchor structures. The core glucosamine is rarely modified. Depending on the protein and species of origin, the lipid anchor of the phosphoinositol ring is a diacylglycerol, an alkylacylglycerol, or a ceramide. The lipid species vary in length, ranging from 14 to 28 carbons, and can be either saturated or unsaturated. Many GPI anchors also contain an additional fatty acid, such as palmitic acid, on the 2-hydroxyl of the inositol ring. This extra fatty acid renders the GPI anchor resistant to cleavage by PI-PLC.

GPI anchor attachment can be achieved by expression of a fusion protein containing a GPI anchor domain in a eukaryotic system capable of carrying out GPI posttranslational modifications. GPI anchor domains can be used as the tumor or tumor vasculature targeting domain, or can be additionally added to fusion proteins already containing separate tumor or tumor vasculature targeting domains.

In another embodiment, GPI anchor moieties are added directly to isolated T cell receptor binding domains through an *in vitro* enzymatic or chemical process. In this embodiment, GPI anchors can be added to polypeptides without the requirement for a GPI anchor domain. GPI anchor moieties can be added to fusion proteins described herein having a T cell receptor binding domain and a tumor or tumor vasculature targeting domain. Alternatively, GPI anchors can be added directly to T cell receptor binding domain polypeptides without the requirement for fusion partners encoding tumor or tumor vasculature targeting domains.

### 2. Peptide or polypeptide linker domain

Fusion proteins optionally contain a peptide or polypeptide linker domain that separates the costimulatory polypeptide domain from the antigen-binding targeting domain.

### Hinge region of antibodies

In one embodiment, the linker domain contains the hinge region of an immunoglobulin. In a preferred embodiment, the hinge region is derived from a human immunoglobulin. Suitable human immunoglobulins that the hinge can be derived from include IgG, IgD and IgA. In a preferred embodiment, the hinge region is derived from human IgG.

In another embodiment, the linker domain contains a hinge region of an immunoglobulin as described above, and further includes one or more additional immunoglobulin domains. In one embodiment, the additional domain includes the Fc domain of an immunoglobulin. The Fc region as used herein includes the polypeptides containing the constant region of an antibody excluding the first constant region immunoglobulin domain. Thus Fc refers to the last two constant region immunoglobulin domains of IgA, IgD, and IgG, and the last three constant region immunoglobulin domains of IgE and IgM. In a preferred embodiment, the Fc domain is derived from a human immunoglobulin. In a more preferred embodiment, the Fc domain is derived from human IgG including the C_{H}2 and C_{H}3 regions.

In another embodiment, the linker domain contains a hinge region of an immunoglobulin and either the C_{H}1 domain of an immunoglobulin heavy chain or the C_{L} domain of an immunoglobulin light chain. In a preferred embodiment, the C_{H}1 or C_{L} domain is derived from a human immunoglobulin. The C_{L} domain may be derived from either a κ light chain or a λ light chain. In a more preferred embodiment, the C_{H}1 or C_{L} domain is derived from human IgG.

Amino acid sequences of immunoglobulin hinge regions and other domains are well known in the art.

### Other peptide/polypeptide linker domains

Other suitable peptide/polypeptide linker domains include naturally occurring or non-naturally occurring peptides or polypeptides. Peptide linker sequences are at least 2 amino acids in length. Preferably the peptide or polypeptide domains are flexible peptides or polypeptides. A "flexible linker" refers to a peptide or polypeptide containing two or more amino acid residues joined by peptide bond(s) that provides increased rotational freedom for two polypeptides linked thereby than the two linked polypeptides would have in the absence of the flexible linker. Such rotational freedom allows two or more antigen binding sites joined by the flexible linker to each access target antigen(s) more efficiently. Exemplary flexible peptides/polypeptides include, but are not limited to, the amino acid sequences Gly-Ser, Gly-Ser-Gly-Ser (SEQ ID NO:74), Ala-Ser, Gly-Gly-Gly-Ser (SEQ ID NO:75), (Gly₄-Ser)₃ (SEQ ID NO:76), and (Gly₄-Ser)₄ (SEQ ID NO:77). Additional flexible peptide/polypeptide sequences are well known in the art.

### 3. Dimerization and multimerization domains

The fusion proteins optionally contain a dimerization or multimerization domain that functions to dimerize or multimerize two or more fusion proteins. The domain that functions to dimerize or multimerize the fusion proteins can either be a separate domain, or alternatively can be contained within one of the other domains (T cell costimulatory/coinhibitory receptor binding domain, tumor/tumor neovasculature antigen-binding domain, or peptide/polypeptide linker domain) of the fusion protein.

### Dimerization domains

A "dimerization domain" is formed by the association of at least two amino acid residues or of at least two peptides or polypeptides (which may have the same, or different, amino acid sequences). The peptides or polypeptides may interact with each other through covalent and/or non-covalent association(s). Preferred dimerization domains contain at least one cysteine that is capable of forming an intermolecular disulfide bond with a cysteine on the partner fusion protein. The dimerization domain can contain one or more cysteine residues such that disulfide bond(s) can form between the partner fusion proteins. In one embodiment, dimerization domains contain one, two or three to about ten cysteine residues. In a preferred embodiment, the dimerization domain is the hinge region of an immunoglobulin. In this particular embodiment, the dimerization domain is contained within the linker peptide/polypeptide of the fusion protein.

Additional exemplary dimerization domain can be any known in the art and include, but not limited to, coiled coils, acid patches, zinc fingers, calcium hands, a C_{H}1-C_{L} pair, an "interface" with an engineered "knob" and/or "protruberance" as described in U.S. Pat. No. 5,821,333, leucine zippers (e.g., from jun and/or fos) (U.S. Pat. No. 5,932,448), SH2 (src homology 2), SH3 (src Homology 3) (Vidal, et al., Biochemistry, 43, 7336-44 ((2004)), phosphotyrosine binding (PTB) (Zhou, et al., Nature, 378:584-592 (1995)), WW (Sudol, Prog. Biochys. Mol. Bio., 65:113-132 (1996)), PDZ (Kim, et al., Nature, 378: 85-88 (1995); Komau, et al., Science, 269:1737-1740 (1995)) 14-3-3, WD40 (Hu, et al., J Biol Chem., 273, 33489-33494 (1998)) EH, Lim, an isoleucine zipper, a receptor dimer pair (e.g., interleukin-8 receptor (IL-8R); and integrin heterodimers such as LFA-1 and GPIIIb/IIIa), or the dimerization region(s) thereof, dimeric ligand polypeptides (e.g. nerve growth factor (NGF), neurotrophin-3 (NT-3), interleukin-8 (IL-8), vascular endothelial growth factor (VEGF), VEGF-C, VEGF-D, PDGF members, and brain-derived neurotrophic factor (BDNF) (Arakawa, et al., J Biol. Chem., 269(45): 27833-27839 (1994) and Radziejewski, et al., Biochem., 32(48): 1350 (1993)) and can also be variants of these domains in which the affinity is altered. The polypeptide pairs can be identified by methods known in the art, including yeast two hybrid screens. Yeast two hybrid screens are described in U.S. Pat. Nos. 5,283,173 and 6,562,576, both of which are herein incorporated by reference in their entireties. Affinities between a pair of interacting domains can be determined using methods known in the art, including as described in Katahira, et al., J. Biol. Chem., 277, 9242-9246 (2002)). Alternatively, a library of peptide sequences can be screened for heterodimerization, for example, using the methods described in WO 01/00814. Useful methods for protein-protein interactions are also described in U.S. Pat. No. 6,790,624.

### Multimerization domains

A "multimerization domain" is a domain that causes three or more peptides or polypeptides to interact with each other through covalent and/or non-covalent association(s). Suitable multimerization domains include, but are not limited to, coiled-coil domains. A coiled-coil is a peptide sequence with a contiguous pattern of mainly hydrophobic residues spaced 3 and 4 residues apart, usually in a sequence of seven amino acids (heptad repeat) or eleven amino acids (undecad repeat), which assembles (folds) to form a multimeric bundle of helices. Coiled-coils with sequences including some irregular distribution of the 3 and 4 residues spacing are also contemplated. Hydrophobic residues are in particular the hydrophobic amino acids Val, Ile, Leu, Met, Tyr, Phe and Trp. Mainly hydrophobic means that at least 50% of the residues must be selected from the mentioned hydrophobic amino acids.

The coiled coil domain may be derived from laminin. In the extracellular space, the heterotrimeric coiled coil protein laminin plays an important role in the formation of basement membranes. Apparently, the multifunctional oligomeric structure is required for laminin function. Coiled coil domains may also be derived from the thrombospondins in which three (TSP-1 and TSP-2) or five (TSP-3, TSP-4 and TSP-5) chains are connected, or from COMP (COMPcc) (Guo, et at., EMBO J., 1998, 17: 5265-5272) which folds into a parallel five-stranded coiled coil (Malashkevich ,et al., Science, 274: 761-765 (1996)).

Additional coiled-coil domains derived from other proteins, and other domains that mediate polypeptide multimerization are known in the art and are suitable for use in the disclosed fusion proteins.

### 4. Exemplary fusion proteins

### PD-L2

A representative murine PD-L2 fusion protein is encoded by a nucleic acid having at least 80%, 85%, 90%, 95%, 99% or 100% sequence identity to:

atgctgctcc tgctgccgat actgaacctg agcttacaac ttcatcctgt agcagcttta 60
ttcaccgtga cagcccctaa agaagtgtac accgtagacg tcggcagcag tgtgagcctg 120
gagtgcgatt ttgaccgcag agaatgcact gaactggaag ggataagagc cagtttgcag 180
aaggtagaaa atgatacgtc tctgcaaagt gaaagagcca ccctgctgga ggagcagctg 240
cccctgggaa aggctttgtt ccacatccct agtgtccaag tgagagattc cgggcagtac 300
cgttgcctgg tcatctgcgg ggccgcctgg gactacaagt acctgacggt gaaagtcaaa 360
gcttcttaca tgaggataga cactaggatc ctggaggttc caggtacagg ggaggtgcag 420
cttacctgcc aggctagagg ttatccccta gcagaagtgt cctggcaaaa tgtcagtgtt 480
cctgccaaca ccagccacat caggaccccc gaaggcctct accaggtcac cagtgttctg 540
cgcctcaagc ctcagcctag cagaaacttc agctgcatgt tctggaatgc tcacatgaag 600
gagctgactt cagccatcat tgaccctctg agtcggatgg aacccaaagt ccccagaacg 660
tgggagccaa gaggtcctac gatcaagccc tgcccgcctt gtaaatgccc agctccaaat 720
ttgctgggtg gaccgtcagt ctttatcttc ccgccaaaga taaaggacgt cttgatgatt 780
agtctgagcc ccatcgtgac atgcgttgtg gtggatgttt cagaggatga ccccgacgtg 840
caaatcagtt ggttcgttaa caacgtggag gtgcataccg ctcaaaccca gacccacaga 900
gaggattata acagcaccct gcgggtagtg tccgccctgc cgatccagca tcaggattgg 960
atgagcggga aagagttcaa gtgtaaggta aacaacaaag atctgccagc gccgattgaa 1020
cgaaccatta gcaagccgaa agggagcgtg cgcgcacctc aggtttacgt ccttcctcca 1080
ccagaagagg agatgacgaa aaagcaggtg accctgacat gcatggtaac tgactttatg 1140
ccagaagata tttacgtgga atggactaat aacggaaaga cagagctcaa ttacaagaac 1200
actgagcctg ttctggattc tgatggcagc tactttatgt actccaaatt gagggtcgag 1260
aagaagaatt gggtcgagag aaacagttat agttgctcag tggtgcatga gggcctccat 1320
aatcatcaca ccacaaagtc cttcagccga acgcccggga aatga 1365
(SEQ ID NO:52)

The murine PD-L2 fusion protein encoded by SEQ ID NO:79 has the following amino acid sequence:

MLLLLPILNL SLQLHPVAAL FTVTAPKEVY TVDVGSSVSL ECDFDRRECT ELEGIRASLQ 60
KVENDTSLQS ERATLLEEQL PLGKALFHIP SVQVRDSGQY RCLVICGAAW DYKYLTVKVK 120

ASYMRIDTRI LEVPGTGEVQ LTCQARGYPL AEVSWQNVSV PANTSHIRTP EGLYQVTSVL 180
RLKPQPSRNF SCMFWNAHMK ELTSAIIDPL SRMEPKVPRT WEPRGPTIKP CPPCKCPAPN 240
LLGGPSVFIF PPKIKDVLMI SLSPIVTCVV VDVSEDDPDV QISWFVNNVE VHTAQTQTHR 300
EDYNSTLRVV SALPIQHQDW MSGKEFKCKV NNKDLPAPIE RTISKPKGSV RAPQVYVLPP 360
PEEEMTKKQV TLTCMVTDFM PEDIYVEWTN NGKTELNYKN TEPVLDSDGS YFMYSKLRVE 420
KKNWVERNSY SCSVVHEGLH NHHTTKSFSR TPGK 454
(SEQ ID NO:53)

The amino acid sequence of the murine PD-L2 fusion protein of SEQ ID NO:53 without the signal sequence is:

LFTVTAPKEV YTVDVGSSVS LECDFDRREC TELEGIRASL QKVENDTSLQ SERATLLEEQ 60
LPLGKALFHI PSVQVRDSGQ YRCLVICGAA WDYKYLTVKV KASYMRIDTR ILEVPGTGEV 120
QLTCQARGYP LAEVSWQNVS VPANTSHIRT PEGLYQVTSV LRLKPQPSRN FSCMFWNAHM 180
KELTSAIIDP LSRMEPKVPR TWEPRGPTIK PCPPCKCPAP NLLGGPSVFI FPPKIKDVLM 240
ISLSPIVTCV VVDVSEDDPD VQISWFVNNV EVHTAQTQTH REDYNSTLRV VSALPIQHQD 300
WMSGKEFKCK VNNKDLPAPI ERTISKPKGS VRAPQVYVLP PPEEEMTKKQ VTLTCMVTDF 360
MPEDIYVEWT NNGKTELNYK NTEPVLDSDG SYFMYSKLRV EKKNWVERNS YSCSVVHEGL 420
HNHHTTKSFS RTPGK 435
(SEQ ID NO:54).

A representative human PD-L2 fusion protein is encoded by a nucleic acid having at least 80%, 85%, 90%, 95%, 99% or 100% sequence identity to:

atgatctttc ttctcttgat gctgtctttg gaattgcaac ttcaccaaat cgcggccctc 60
tttactgtga ccgtgccaaa agaactgtat atcattgagc acgggtccaa tgtgaccctc 120
gaatgtaact ttgacaccgg cagccacgtt aacctggggg ccatcactgc cagcttgcaa 180
aaagttgaaa acgacacttc acctcaccgg gagagggcaa ccctcttgga ggagcaactg 240
ccattgggga aggcctcctt tcatatccct caggtgcagg ttcgggatga gggacagtac 300
cagtgcatta ttatctacgg cgtggcttgg gattacaagt atctgaccct gaaggtgaaa 360
gcgtcctatc ggaaaattaa cactcacatt cttaaggtgc cagagacgga cgaggtggaa 420
ctgacatgcc aagccaccgg ctacccgttg gcagaggtca gctggcccaa cgtgagcgta 480
cctgctaaca cttctcattc taggacaccc gagggcctct accaggttac atccgtgctc 540
cgcctcaaac cgcccccagg ccggaatttt agttgcgtgt tttggaatac ccacgtgcga 600
gagctgactc ttgcatctat tgatctgcag tcccagatgg agccacggac tcatccaact 660
tgggaaccta aatcttgcga taaaactcat acctgtcccc cttgcccagc ccccgagctt 720
ctgggaggtc ccagtgtgtt tctgtttccc ccaaaaccta aggacacact tatgatatcc 780
cgaacgccgg aagtgacatg cgtggttgtg gacgtctcac acgaagaccc ggaggtgaaa 840
ttcaactggt acgttgacgg agttgaggtt cataacgcta agaccaagcc cagagaggag 900
caatacaatt ccacctatcg agtggttagt gtactgaccg ttttgcacca agactggctg 960
aatggaaaag aatacaagtg caaagtatca aacaaggctt tgcctgcacc catcgagaag 1020
acaatttcta aagccaaagg gcagcccagg gaaccgcagg tgtacacact cccaccatcc 1080
cgcgacgagc tgacaaagaa tcaagtatcc ctgacctgcc tggtgaaagg cttttaccca 1140
tctgacattg ccgtggaatg ggaatcaaat ggacaacctg agaacaacta caaaaccact 1200
ccacctgtgc ttgacagcga cgggtccttt ttcctgtaca gtaagctcac tgtcgataag 1260
tctcgctggc agcagggcaa cgtcttttca tgtagtgtga tgcacgaagc tctgcacaac 1320
cattacaccc agaagtctct gtcactgagc ccaggtaaat ga 1362

(SEQ ID NO:55)

The human PD-L2 fusion protein encoded by SEQ ID NO:82 has the following amino acid sequence:

MIFLLLMLSL ELQLHQIAAL FTVTVPKELY IIEHGSNVTL ECNFDTGSHV NLGAITASLQ 60
KVENDTSPHR ERATLLEEQL PLGKASFHIP QVQVRDEGQY QCIIIYGVAW DYKYLTLKVK 120
ASYRKINTHI LKVPETDEVE LTCQATGYPL AEVSWPNVSV PANTSHSRTP EGLYQVTSVL 180
RLKPPPGRNF SCVFWNTHVR ELTLASIDLQ SQMEPRTHPT WEPKSCDKTH TCPPCPAPEL 240
LGGPSVFLFP PKPKDTLMIS RTPEVTCVVV DVSHEDPEVK FNWYVDGVEV HNAKTKPREE 300
QYNSTYRVVS VLTVLHQDWL NGKEYKCKVS NKALPAPIEK TISKAKGQPR EPQVYTLPPS 360
RDELTKNQVS LTCLVKGFYP SDIAVEWESN GQPENNYKTT PPVLDSDGSF FLYSKLTVDK 420
SRWQQGNVFS CSVMHEALHN HYTQKSLSLS PGK 453
(SEQ ID NO:56)

The amino acid sequence of the human PD-L2 fusion protein of SEQ ID NO:83 without the signal sequence is:

LFTVTVPKEL YIIEHGSNVT LECNFDTGSH VNLGAITASL QKVENDTSPH RERATLLEEQ 60
LPLGKASFHI PQVQVRDEGQ YQCIIIYGVA WDYKYLTLKV KASYRKINTH ILKVPETDEV 120
ELTCQATGYP LAEVSWPNVS VPANTSHSRT PEGLYQVTSV LRLKPPPGRN FSCVFWNTHV 180
RELTLASIDL QSQMEPRTHP TWEPKSCDKT HTCPPCPAPE LLGGPSVFLF PPKPKDTLMI 240
SRTPEVTCVV VDVSHEDPEV KFNWYVDGVE VHNAKTKPRE EQYNSTYRVV SVLTVLHQDW 300
LNGKEYKCKV SNKALPAPIE KTISKAKGQP REPQVYTLPP SRDELTKNQV SLTCLVKGFY 360
PSDIAVEWES NGQPENNYKT TPPVLDSDGS FFLYSKLTVD KSRWQQGNVF SCSVMHEALH 420
NHYTQKSLSL SPGK 434
(SEQ ID NO:57).

A representative non-human primate (*Cynomolgus*) PD-L2 fusion protein has the following amino acid sequence:

MIFLLLMLSLELQLHQIAA
LFTVTVPKELYIIEHGSNVTLECNFDTGSHVNLGAITASLQKVENDTSPHRERATLLEEQLPLGKASFHIPQ
VQVRDEGQYQCIIIYGVAWDYKYLTLKVKASYRKINTHILKVPETDEVELTCQATGYPLAEVSWPNVSVPAN
TSHSRTPEGLYQVTSVLRLKPPPGRNFSCVFWNTHVRELTLASIDLQSQMEPRTHPTWEPKSCDKTHTCPPC
PAPELLGGPSVFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSVMHEALHNHYTQKSLSLSP
GK

(SEQ ID NO:86)

The amino acid sequence of the non-human primate (*Cynomolgus*) PD-L2 fusion protein of SEQ ID NO:86 without the signal sequence is:

LFTVTVPKELYIIEHGSNVTLECNFDTGSHVNLGAITASLQKVENDTSPHRERATLLEEQLPLGKASFHIPQ
VQVRDEGQYQCIIIYGVAWDYKYLTLKVKASYRKINTHILKVPETDEVELTCQATGYPLAEVSWPNVSVPAN

TSHSRTPEGLYQVTSVLRLKPPPGRNFSCVFWNTHVRELTLASIDLQSQMEPRTHPTWEPKSCDKTHTCPPC
PAPELLGGPSVFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSVMHEALHNHYTQKSLSLSP
GK

(SEQ ID NO:87).

### G. Isolated Nucleic Acid Molecules Encoding PD-1 Receptor Antagonists

Isolated nucleic acid sequences encoding PD-1 antagonist polypeptides, variants thereof and fusion proteins thereof are disclosed. As used herein, "isolated nucleic acid" refers to a nucleic acid that is separated from other nucleic acid molecules that are present in a mammalian genome, including nucleic acids that normally flank one or both sides of the nucleic acid in a mammalian genome.

An isolated nucleic acid can be, for example, a DNA molecule, provided one of the nucleic acid sequences normally found immediately flanking that DNA molecule in a naturally-occurring genome is removed or absent. Thus, an isolated nucleic acid includes, without limitation, a DNA molecule that exists as a separate molecule independent of other sequences (e.g., a chemically synthesized nucleic acid, or a cDNA or genomic DNA fragment produced by PCR or restriction endonuclease treatment), as well as recombinant DNA that is incorporated into a vector, an autonomously replicating plasmid, a virus (e.g., a retrovirus, lentivirus, adenovirus, or herpes virus), or into the genomic DNA of a prokaryote or eukaryote. In addition, an isolated nucleic acid can include an engineered nucleic acid such as a recombinant DNA molecule that is part of a hybrid or fusion nucleic acid. A nucleic acid existing among hundreds to millions of other nucleic acids within, for example, a cDNA library or a genomic library, or a gel slice containing a genomic DNA restriction digest, is not to be considered an isolated nucleic acid.

Nucleic acids can be in sense or antisense orientation, or can be complementary to a reference sequence encoding a PD-L2, PD-L1, PD-1 or B7.1 polypeptide or variant thereof. Reference sequences include, for example, the nucleotide sequence of human PD-L2, human PD-L1 or murine PD-L2 and murine PD-L1 which are known in the art and discussed above.

Nucleic acids can be DNA, RNA, or nucleic acid analogs. Nucleic acid analogs can be modified at the base moiety, sugar moiety, or phosphate backbone. Such modification can improve, for example, stability, hybridization, or solubility of the nucleic acid. Modifications at the base moiety can include deoxyuridine for deoxythymidine, and 5-methyl-2'-deoxycytidine or 5-bromo-2'-deoxycytidine for deoxycytidine. Modifications of the sugar moiety can include modification of the 2' hydroxyl of the ribose sugar to form 2'-O-methyl or 2'-O-allyl sugars. The deoxyribose phosphate backbone can be modified to produce morpholino nucleic acids, in which each base moiety is linked to a six membered, morpholino ring, or peptide nucleic acids, in which the deoxyphosphate backbone is replaced by a pseudopeptide backbone and the four bases are retained. See, for example, Summerton and Weller (1997) Antisense Nucleic Acid Drug Dev. 7:187-195; and Hyrup et al. (1996) Bioorgan. Med Chem. 4:5-23. In addition, the deoxyphosphate backbone can be replaced with, for example, a phosphorothioate or phosphorodithioate backbone, a phosphoroamidite, or an alkyl phosphotriester backbone.

### H. Vectors and Host Cells Expressing PD-1 Receptor Antagonists

Nucleic acids, such as those described above, can be inserted into vectors for expression in cells. As used herein, a "vector" is a replicon, such as a plasmid, phage, or cosmid, into which another DNA segment may be inserted so as to bring about the replication of the inserted segment. Vectors can be expression vectors. An "expression vector" is a vector that includes one or more expression control sequences, and an "expression control sequence" is a DNA sequence that controls and regulates the transcription and/or translation of another DNA sequence.

Nucleic acids in vectors can be operably linked to one or more expression control sequences. As used herein, "operably linked" means incorporated into a genetic construct so that expression control sequences effectively control expression of a coding sequence of interest. Examples of expression control sequences include promoters, enhancers, and transcription terminating regions. A promoter is an expression control sequence composed of a region of a DNA molecule, typically within 100 nucleotides upstream of the point at which transcription starts (generally near the initiation site for RNA polymerase II). To bring a coding sequence under the control of a promoter, it is necessary to position the translation initiation site of the translational reading frame of the polypeptide between one and about fifty nucleotides downstream of the promoter. Enhancers provide expression specificity in terms of time, location, and level. Unlike promoters, enhancers can function when located at various distances from the transcription site. An enhancer also can be located downstream from the transcription initiation site. A coding sequence is "operably linked" and "under the control" of expression control sequences in a cell when RNA polymerase is able to transcribe the coding sequence into mRNA, which then can be translated into the protein encoded by the coding sequence.

Suitable expression vectors include, without limitation, plasmids and viral vectors derived from, for example, bacteriophage, baculoviruses, tobacco mosaic virus, herpes viruses, cytomegalo virus, retroviruses, vaccinia viruses, adenoviruses, and adeno-associated viruses. Numerous vectors and expression systems are commercially available from such corporations as Novagen (Madison, WI), Clontech (Palo Alto, CA), Stratagene (La Jolla, CA), and Invitrogen Life Technologies (Carlsbad, CA).

An expression vector can include a tag sequence. Tag sequences, are typically expressed as a fusion with the encoded polypeptide. Such tags can be inserted anywhere within the polypeptide including at either the carboxyl or amino terminus. Examples of useful tags include, but are not limited to, green fluorescent protein (GFP), glutathione S-transferase (GST), polyhistidine, c-myc, hemagglutinin, Flag™ tag (Kodak, New Haven, CT), maltose E binding protein and protein A. In one embodiment, the variant PD-L2 fusion protein is present in a vector containing nucleic acids that encode one or more domains of an Ig heavy chain constant region, preferably having an amino acid sequence corresponding to the hinge, C_{H2} and C_{H3} regions of a human immunoglobulin Cγ1 chain.

Vectors containing nucleic acids to be expressed can be transferred into host cells. The term "host cell" is intended to include prokaryotic and eukaryotic cells into which a recombinant expression vector can be introduced. As used herein, "transformed" and "transfected" encompass the introduction of a nucleic acid molecule (e.g., a vector) into a cell by one of a number of techniques. Although not limited to a particular technique, a number of these techniques are well established within the art. Prokaryotic cells can be transformed with nucleic acids by, for example, electroporation or calcium chloride mediated transformation. Nucleic acids can be transfected into mammalian cells by techniques including, for example, calcium phosphate co-precipitation, DEAE-dextran-mediated transfection, lipofection, electroporation, or microinjection. Host cells (e.g., a prokaryotic cell or a eukaryotic cell such as a CHO cell) can be used to, for example, produce the PD-1 antagonist polypeptides described herein.

### I. Antibody PD-1 antagonists

Monoclonal and polyclonal antibodies that are reactive with epitopes of the PD-1 antagonists, or PD-1, are disclosed. Monoclonal antibodies (mAbs) and methods for their production and use are described in Kohler and Milstein, Nature 256:495-497 (1975); U.S. Pat. No. 4,376,110; Hartlow, E. et al., Antibodies: A Laboratory Manual, Cold Spring Harbor Laboratory Press, Cold Spring Harbor, N.Y., 1988); Monoclonal Antibodies and Hybridomas: A New Dimension in Biological Analyses, Plenum Press, New York, N.Y. (1980); H. Zola et al., in Monoclonal Hybridoma Antibodies: Techniques and Applications, CRC Press, 1982)).

Antibodies that bind to PD-1 and block signal transduction through PD-1, and which have a lower affinity than those currently in use, allowing the antibody to dissociated in a period of less than three months, two months, one month, three weeks, two weeks, one week, or a few days after administration, are preferred for enhancement, augmentation or stimulation of an immune response.

Another embodiment of the invention includes a bi-specific antibody that comprises an antibody that binds to the PD-1 receptor bridged to an antibody that binds to a ligand of PD-1, such as B7-H1. In a preferred embodiment, the PD-1 binding portion reduces or inhibits signal transduction through the PD-1 receptor

Immunoassay methods are described in Coligan, J. E. et al., eds., Current Protocols in Immunology, Wiley-Interscience, New York 1991 (or current edition); Butt, W. R. (ed.) Practical Immunoassay: The State of the Art, Dekker, N.Y., 1984; Bizollon, Ch. A., ed., Monoclonal Antibodies and New Trends in Immunoassays, Elsevier, N.Y., 1984; Butler, J. E., ELISA (Chapter 29), In: van Oss, C. J. et al., (eds), Immunochemistry, Marcel Dekker, Inc., New York, 1994, pp. 759-803; Butler, J. E. (ed.), Immunochemistry of Solid-Phase Immunoassay, CRC Press, Boca Raton, 1991; Weintraub, B., Principles of Radioimmunoassays, Seventh Training Course on Radioligand Assay Techniques, The Endocrine Society, March, 1986; Work, T. S. et al., Laboratory Techniques and Biochemistry in Molecular Biology, North Holland Publishing Company, NY, (1978) (Chapter by Chard, T., "An Introduction to Radioimmune Assay and Related Techniques").

Anti-idiotypic antibodies are described, for example, in Idiotypy in Biology and Medicine, Academic Press, New York, 1984; Immunological Reviews Volume 79, 1984; Immunological Reviews Volume 90, 1986; Curr. Top. Microbiol., Immunol. Volume 119, 1985; Bona, C. et al., CRC Crit. Rev. Immunol., pp. 33-81 (1981); Jerme, N K, Ann. Immunol. 125C:373-389 (1974); Jeme, N K, In: Idiotypes--Antigens on the Inside, Westen-Schnurr, I., ed., Editiones Roche, Basel, 1982, Urbain, J. et al., Ann. Immunol. 133D:179-(1982); Rajewsky, K. et al., Ann. Rev. Immunol. 1:569-607 (1983).

The antibodies may be xenogeneic, allogeneic, syngeneic, or modified forms thereof, such as humanized or chimeric antibodies. Antiidiotypic antibodies specific for the idiotype of a specific antibody, for example an anti-PD-L2 antibody, are also included. The term "antibody" is meant to include both intact molecules as well as fragments thereof that include the antigen-binding site and are capable of binding to a PD-1 antagonist epitope. These include, Fab and F(ab')₂ fragments which lack the Fc fragment of an intact antibody, clear more rapidly from the circulation, and may have less non-specific tissue binding than an intact antibody (Wahl et al., J. Nuc. Med. 24:316-325 (1983)). Also included are Fv fragments (Hochman, J. et al. (1973) Biochemistry 12:1130-1135; Sharon, J. et al.(1976) Biochemistry 15:1591-1594). These various fragments are produced using conventional techniques such as protease cleavage or chemical cleavage (see, e.g., Rousseaux et al., Meth. Enzymol., 121:663-69 (1986)).

Polyclonal antibodies are obtained as sera from immunized animals such as rabbits, goats, rodents, etc. and may be used directly without further treatment or may be subjected to conventional enrichment or purification methods such as ammonium sulfate precipitation, ion exchange chromatography, and affinity chromatography.

The immunogen may include the complete PD-1 antagonist, PD-1, or fragments or derivatives thereof. Preferred immunogens include all or a part of the extracellular domain (ECD) of PD-1 antagonist or PD-1, where these residues contain the post-translation modifications, such as glycosylation. Immunogens including the extracellular domain are produced in a variety of ways known in the art, e.g., expression of cloned genes using conventional recombinant methods or isolation from cells of origin.

Monoclonal antibodies may be produced using conventional hybridoma technology, such as the procedures introduced by Kohler and Milstein, Nature, 256:495-97 (1975), and modifications thereof (see above references). An animal, preferably a mouse is primed by immunization with an immunogen as above to elicit the desired antibody response in the primed animal. B lymphocytes from the lymph nodes, spleens or peripheral blood of a primed, animal are fused with myeloma cells, generally in the presence of a fusion promoting agent such as polyethylene glycol (PEG). Any of a number of murine myeloma cell lines are available for such use: the P3-NS1/1-Ag4-1, P3-x63-k0Ag8.653, Sp2/0-Agl4, or HL1-653 myeloma lines (available from the ATCC, Rockville, Md.). Subsequent steps include growth in selective medium so that unfused parental myeloma cells and donor lymphocyte cells eventually die while only the hybridoma cells survive. These are cloned and grown and their supernatants screened for the presence of antibody of the desired specificity, e.g. by immunoassay techniques using PD-L2 or PD-L1 fusion proteins. Positive clones are subcloned, e.g., by limiting dilution, and the monoclonal antibodies are isolated.

Hybridomas produced according to these methods can be propagated *in vitro* or *in vivo* (in ascites fluid) using techniques known in the art (see generally Fink et al., Prog. Clin. Pathol., 9:121-33 (1984)). Generally, the individual cell line is propagated in culture and the culture medium containing high concentrations of a single monoclonal antibody can be harvested by decantation, filtration, or centrifugation.

The antibody may be produced as a single chain antibody or scFv instead of the normal multimeric structure. Single chain antibodies include the hypervariable regions from an Ig of interest and recreate the antigen binding site of the native Ig while being a fraction of the size of the intact Ig (Skerra, A. et al. Science, 240: 1038-1041 (1988); Pluckthun, A. et al. Methods Enzymol. 178: 497-515 (1989); Winter, G. et al. Nature, 349: 293-299 (1991)). In a preferred embodiment, the antibody is produced using conventional molecular biology techniques.

### III. Methods of Manufacture

### A. Methods for Producing PD-1 Antagonist Polypeptides and Variants Thereof

Isolated PD-1 antagonists or variants thereof can be obtained by, for example, chemical synthesis or by recombinant production in a host cell. To recombinantly produce a PD-1 antagonist polypeptide, a nucleic acid containing a nucleotide sequence encoding the polypeptide can be used to transform, transduce, or transfect a bacterial or eukaryotic host cell (e.g., an insect, yeast, or mammalian cell). In general, nucleic acid constructs include a regulatory sequence operably linked to a nucleotide sequence encoding a PD-1 antagonist polypeptide. Regulatory sequences (also referred to herein as expression control sequences) typically do not encode a gene product, but instead affect the expression of the nucleic acid sequences to which they are operably linked.

Useful prokaryotic and eukaryotic systems for expressing and producing polypeptides are well know in the art include, for example, *Escherichia coli* strains such as BL-21, and cultured mammalian cells such as CHO cells.

In eukaryotic host cells, a number of viral-based expression systems can be utilized to express PD-1 antagonist polypeptide. Viral based expression systems are well known in the art and include, but are not limited to, baculoviral, SV40, retroviral, or vaccinia based viral vectors.

Mammalian cell lines that stably express PD-1 antagonist polypeptides can be produced using expression vectors with appropriate control elements and a selectable marker. For example, the eukaryotic expression vectors pCR3.1 (Invitrogen Life Technologies) and p91023(B) (see Wong et al. (1985) Science 228:810-815) are suitable for expression of variant costimulatory polypeptides in, for example, Chinese hamster ovary (CHO) cells, COS-1 cells, human embryonic kidney 293 cells, NIH3T3 cells, BHK21 cells, MDCK cells, and human vascular endothelial cells (HUVEC). Following introduction of an expression vector by electroporation, lipofection, calcium phosphate, or calcium chloride co-precipitation, DEAE dextran, or other suitable transfection method, stable cell lines can be selected (e.g., by antibiotic resistance to G418, kanamycin, or hygromycin). The transfected cells can be cultured such that the polypeptide of interest is expressed, and the polypeptide can be recovered from, for example, the cell culture supernatant or from lysed cells. Alternatively, a PD-1 antagonist polypeptide can be produced by (a) ligating amplified sequences into a mammalian expression vector such as pcDNA3 (Invitrogen Life Technologies), and (b) transcribing and translating *in vitro* using wheat germ extract or rabbit reticulocyte lysate.

PD-1 antagonist polypeptides can be isolated using, for example, chromatographic methods such as DEAE ion exchange, gel filtration, and hydroxylapatite chromatography. For example, PD-1 antagonist polypeptides in a cell culture supernatant or a cytoplasmic extract can be isolated using a protein G column. In some embodiments, variant PD-1 antagonist polypeptides can be "engineered" to contain an amino acid sequence that allows the polypeptides to be captured onto an affinity matrix. For example, a tag such as c-myc, hemagglutinin, polyhistidine, or Flag™ (Kodak) can be used to aid polypeptide purification. Such tags can be inserted anywhere within the polypeptide, including at either the carboxyl or amino terminus. Other fusions that can be useful include enzymes that aid in the detection of the polypeptide, such as alkaline phosphatase. Immunoaffinity chromatography also can be used to purify costimulatory polypeptides.

Methods for introducing random mutations to produce variant polypeptides are known in the art. Random peptide display libraries can be used to screen for peptides which interact with PD-1, PD-L1 or PD-L2. Techniques for creating and screening such random peptide display libraries are known in the art (Ladner et al., U.S. Patent No. 5,223,409; Ladner et al., U.S. Patent No. 4,946,778; Ladner et aL, U.S. Patent No. 5,403,484 and Ladner et al., U.S. Patent No. 5,571,698) and random peptide display libraries and kits for screening such libraries are available commercially.

### B. Methods for Producing Isolated Nucleic Acid Molecules Encoding PD-1 Antagonist Polypeptides

Isolated nucleic acid molecules encoding PD-1 antagonist polypeptides can be produced by standard techniques, including, without limitation, common molecular cloning and chemical nucleic acid synthesis techniques. For example, polymerase chain reaction (PCR) techniques can be used to obtain an isolated nucleic acid encoding a variant costimulatory polypeptide. PCR is a technique in which target nucleic acids are enzymatically amplified. Typically, sequence information from the ends of the region of interest or beyond can be employed to design oligonucleotide primers that are identical in sequence to opposite strands of the template to be amplified. PCR can be used to amplify specific sequences from DNA as well as RNA, including sequences from total genomic DNA or total cellular RNA. Primers typically are 14 to 40 nucleotides in length, but can range from 10 nucleotides to hundreds of nucleotides in length. General PCR techniques are described, for example in PCR Primer: A Laboratory Manual, ed. by Dieffenbach and Dveksler, Cold Spring Harbor Laboratory Press, 1995. When using RNA as a source of template, reverse transcriptase can be used to synthesize a complementary DNA (cDNA) strand. Ligase chain reaction, strand displacement amplification, self-sustained sequence replication or nucleic acid sequence-based amplification also can be used to obtain isolated nucleic acids. See, for example, Lewis (1992) Genetic Engineering News 12:1; Guatelli et al. (1990) Proc. Natl, Acad. Sci. USA 87:1874-1878; and Weiss (1991) Science 254:1292-1293.

Isolated nucleic acids can be chemically synthesized, either as a single nucleic acid molecule or as a series of oligonucleotides (e.g., using phosphoramidite technology for automated DNA synthesis in the 3' to 5' direction). For example, one or more pairs of long oligonucleotides (e.g., >100 nucleotides) can be synthesized that contain the desired sequence, with each pair containing a short segment of complementarity (e.g., about 15 nucleotides) such that a duplex is formed when the oligonucleotide pair is annealed. DNA polymerase can be used to extend the oligonucleotides, resulting in a single, double-stranded nucleic acid molecule per oligonucleotide pair, which then can be ligated into a vector. Isolated nucleic acids can also obtained by mutagenesis. PD-1 antagonist polypeptide encoding nucleic acids can be mutated using standard techniques, including oligonucleotide-directed mutagenesis and/or site-directed mutagenesis through PCR. See, Short Protocols in Molecular Biology. Chapter 8, Green Publishing Associates and John Wiley & Sons, edited by Ausubel et al, 1992. Examples of amino acid positions that can be modified include those described herein.

### IV. Formulations

### A. PD-1 Antagonist Formulations

Pharmaceutical compositions including PD-1 antagonists are provided. Pharmaceutical compositions containing peptides or polypeptides may be for administration by parenteral (intramuscular, intraperitoneal, intravenous (IV) or subcutaneous injection), transdermal (either passively or using iontophoresis or electroporation), or transmucosal (nasal, vaginal, rectal, or sublingual) routes of administration. The compositions may also be administered using bioerodible inserts and may be delivered directly to an appropriate lymphoid tissue (e.g., spleen, lymph node, or mucosal-associated lymphoid tissue) or directly to an organ or tumor. The compositions can be formulated in dosage forms appropriate for each route of administration. Compositions containing antagonists of PD-1 receptors that are not peptides or polypeptides can additionally be formulated for enteral administration.

As used herein the term "effective amount" or "therapeutically effective amount" means a dosage sufficient to treat, inhibit, or alleviate one or more symptoms of the disorder being treated or to otherwise provide a desired pharmacologic and/or physiologic effect. The precise dosage will vary according to a variety of factors such as subject-dependent variables (e.g., age, inunune system health, etc.), the disease, and the treatment being effected. Therapeutically effective amounts of PD-1 antagonist cause an immune response to be activated, enhanced, augmented, or sustained, and/or overcome or alleviate T cell exhaustion and/or T cell anergy, and/or activate monocytes, macrophages, dendritic cells and other antigen presenting cells ("APCs").

In a preferred embodiment, the PD-1 antagonist is administered in a range of 0.1 - 20 mg/kg based on extrapolation from tumor modeling and bioavailability. A most preferred range is 5-20 mg of PD-1 antagonist/kg. Generally, for intravenous injection or infusion, dosage may be lower than when administered by an alternative route.

### 1. Formulations for Parenteral Administration

In a preferred embodiment, the disclosed compositions, including those containing peptides and polypeptides, are administered in an aqueous solution, by parenteral injection. The formulation may also be in the form of a suspension or emulsion. In general, pharmaceutical compositions are provided including effective amounts of a peptide or polypeptide, and optionally include pharmaceutically acceptable diluents, preservatives, solubilizers, emulsifiers, adjuvants and/or carriers. Such compositions include sterile water, buffered saline (e.g., Tris-HCl, acetate, phosphate), pH and ionic strength; and optionally, additives such as detergents and solubilizing agents (e.g., TWEEN® 20, TWEEN 80, Polysorbate 80), antioxidants (e.g., ascorbic acid, sodium metabisulfite), and preservatives (e.g., Thimersol, benzyl alcohol) and bulking substances (e.g., lactose, mannitol). Examples of non-aqueous solvents or vehicles are propylene glycol, polyethylene glycol, vegetable oils, such as olive oil and corn oil, gelatin, and injectable organic esters such as ethyl oleate. The formulations may be lyophilized and redissolved/resuspended immediately before use. The formulation may be sterilized by, for example, filtration through a bacteria retaining filter, by incorporating sterilizing agents into the compositions, by irradiating the compositions, or by heating the compositions.

### 2. Controlled Delivery Polymeric Matrices

Compositions containing one or more PD-1 antagonist or nucleic acids encoding the PD-1 antagonist can be administered in controlled release formulations. Controlled release polymeric devices can be made for long term release systemically following implantation of a polymeric device (rod, cylinder, film, disk) or injection (microparticles). The matrix can be in the form of microparticles such as microspheres, where peptides are dispersed within a solid polymeric matrix or microcapsules, where the core is of a different material than the polymeric shell, and the peptide is dispersed or suspended in the core, which may be liquid or solid in nature. Unless specifically defined herein, microparticles, microspheres, and microcapsules are used interchangeably. Alternatively, the polymer may be cast as a thin slab or film, ranging from nanometers to four centimeters, a powder produced by grinding or other standard techniques, or even a gel such as a hydrogel. The matrix can also be incorporated into or onto a medical device to modulate an immune response, to prevent infection in an immunocompromised patient (such as an elderly person in which a catheter has been inserted or a premature child) or to aid in healing, as in the case of a matrix used to facilitate healing of pressure sores, decubitis ulcers, etc.

Either non-biodegradable or biodegradable matrices can be used for delivery of PD-1 antagonist or nucleic acids encoding them, although biodegradable matrices are preferred. These may be natural or synthetic polymers, although synthetic polymers are preferred due to the better characterization of degradation and release profiles. The polymer is selected based on the period over which release is desired. In some cases linear release may be most useful, although in others a pulse release or "bulk release" may provide more effective results. The polymer may be in the form of a hydrogel (typically in absorbing up to about 90% by weight of water), and can optionally be crosslinked with multivalent ions or polymers.

The matrices can be formed by solvent evaporation, spray drying, solvent extraction and other methods known to those skilled in the art. Bioerodible microspheres can be prepared using any of the methods developed for making microspheres for drug delivery, for example, as described by Mathiowitz and Langer, J. Controlled Release, 5:13-22 (1987); Mathiowitz, et al., Reactive Polymers, 6:275-283 (1987); and Mathiowitz, et al., J. Appl. Polymer Sci., 35:755-774 (1988).

Controlled release oral formulations may be desirable. Antagonists of PD-1 inhibitory signaling can be incorporated into an inert matrix which permits release by either diffusion or leaching mechanisms, e.g., films or gums. Slowly disintegrating matrices may also be incorporated into the formulation. Another form of a controlled release is one in which the drug is enclosed in a semipermeable membrane which allows water to enter and push drug out through a single small opening due to osmotic effects. For oral formulations, the location of release may be the stomach, the small intestine (the duodenum, the jejunem, or the ileum), or the large intestine. Preferably, the release will avoid the deleterious effects of the stomach environment, either by protection of the active agent (or derivative) or by release of the active agent beyond the stomach environment, such as in the intestine. To ensure full gastric resistance an enteric coating (i.e, impermeable to at least pH 5.0) is essential. These coatings may be used as mixed films or as capsules such as those available from Banner Pharmacaps.

The devices can be formulated for local release to treat the area of implantation or injection and typically deliver a dosage that is much less than the dosage for treatment of an entire body. The devices can also be formulated for systemic delivery. These can be implanted or injected subcutaneously.

### 3. Formulations for Enteral Administration

Antagonists of PD-1 can also be formulated for oral delivery. Oral solid dosage forms are known to those skilled in the art. Solid dosage forms include tablets, capsules, pills, troches or lozenges, cachets, pellets, powders, or granules or incorporation of the material into particulate preparations of polymeric compounds such as polylactic acid, polyglycolic acid, etc. or into liposomes. Such compositions may influence the physical state, stability, rate of *in vivo* release, and rate of *in vivo* clearance of the present proteins and derivatives. See, e.g., Remington's Pharmaceutical Sciences, 21 st Ed. (2005, Lippincott, Williams & Wilins, Baltimore, Md. 21201) pages 889-964. The compositions may be prepared in liquid form, or may be in dried powder (e.g., lyophilized) form. Liposomal or polymeric encapsulation may be used to formulate the compositions. See also Marshall, K. In: Modem Pharmaceutics Edited by G. S. Banker and C. T. Rhodes Chapter 10, 1979. In general, the formulation will include the active agent and inert ingredients which protect the PD-1 antagonist in the stomach environment, and release of the biologically active material in the intestine.

Liquid dosage forms for oral administration, including pharmaceutically acceptable emulsions, solutions, suspensions, and syrups, may contain other components including inert diluents; adjuvants such as wetting agents, emulsifying and suspending agents; and sweetening, flavoring, and perfuming agents.

### B. Vaccines Including PD-1 Antagonists

Vaccines require strong T cell response to eliminate infected cells. PD-1 antagonists can be administered as a component of a vaccine to promote, augment, or enhance the primary immune response and effector cell activity and numbers. Vaccines include antigens, the PD-1 antagonist (or a source thereof) and optionally other adjuvants and targeting molecules. Sources of PD-1 antagonist include any of the disclosed PD-L2 polypeptides, PD-L2 fusion proteins, variants thereof, PD-L1 fragments, PD-1 fragments, nucleic acids encoding PD-L2 polypeptides, PD-L2 fusion proteins, variants thereof, PD-L1 fragments or PD-1 fragments, or host cells containing vectors that express polypeptide ligands of PD-1 described above.

### 1. Antigens

Antigens can be peptides, proteins, polysaccharides, saccharides, lipids, nucleic acids, or combinations thereof. The antigen can be derived from a virus, bacterium, parasite, protozoan, fungus, histoplasma, tissue or transformed cell and can be a whole cell or immunogenic component thereof, e.g., cell wall components or molecular components thereof.

Suitable antigens are known in the art and are available from commercial, government and scientific sources. In one embodiment, the antigens are whole inactivated or attenuated organisms. These organisms may be infectious organisms, such as viruses, parasites and bacteria. The organisms may be tumor cells or cells infected with a virus or intracellular pathogen such as gonorrhea or malaria. The antigens may be purified or partially purified polypeptides derived from tumors or viral or bacterial sources. The antigens can be recombinant polypeptides produced by expressing DNA encoding the polypeptide antigen in a heterologous expression system. The antigens can be DNA encoding all or part of an antigenic protein. The DNA may be in the form of vector DNA such as plasmid DNA.

Antigens may be provided as single antigens or may be provided in combination. Antigens may also be provided as complex mixtures of polypeptides or nucleic acids.

### i. Viral Antigens

A viral antigen can be isolated from any virus including, but not limited to, a virus from any of the following viral families: *Arenaviridae, Arterivirus, Astroviridae, Baculoviridae, Badnavirus, Barnaviridae, Birnaviridae, Bromoviridae, Bunyaviridae, Caliciviridae, Capillovirus, Carlavirus, Caulimovirus, Circoviridae, Closterovirus, Comoviridae, Coronaviridae (e.g., Coronavirus,* such as severe acute respiratory syndrome (SARS) virus), *Corticoviridae, Cystoviridae, Deltavirus, Dianthovirus, Enamovirus, Filoviridae* (e.g., Marburg virus and Ebola virus (e.g., Zaire, Reston, Ivory Coast, or Sudan strain)), *Flaviviridae,* (e.g., Hepatitis C virus, Dengue virus 1, Dengue virus 2, Dengue virus 3, and Dengue virus 4), *Hepadnaviridae, Herpesviridae (e.g.,* Human herpesvirus 1, 3, 4, 5, and 6, and Cytomegalovirus), *Hypoviridae, Iridoviridae, Leviviridae, Lipothrixviridae, Microviridae, Orthomyxoviridae* (e.g., Influenzavirus A and B and C), *Papovaviridae, Paramyxoviridae (e.g.,* measles, mumps, and human respiratory syncytial virus), *Parvoviridae, Picornaviridae (e.g.,* poliovirus, rhinovirus, hepatovirus, and aphthovirus), *Poxviridae (e.g.,* vaccinia and smallpox virus), *Reoviridae* (e.g., rotavirus), *Retroviridae (e.g.,* lentivirus, such as human immunodeficiency virus (HIV) 1 and HIV 2), *Rhabdoviridae* (for example, rabies virus, measles virus, respiratory syncytial virus, etc.), *Togaviridae* (for example, rubella virus, dengue virus, etc.), and *Totiviridae.* Suitable viral antigens also include all or part of Dengue protein M, Dengue protein E, Dengue D1NS1, Dengue D1NS2, and Dengue D1NS3.

Viral antigens may be derived from a particular strain, or a combination of strains, such as a papilloma virus, a herpes virus, i.e. herpes simplex 1 and 2; a hepatitis virus, for example, hepatitis A virus (HAV), hepatitis B virus (HBV), hepatitis C virus (HCV), the delta hepatitis D virus (HDV), hepatitis E virus (HEV) and hepatitis G virus (HGV), the tick-borne encephalitis viruses; parainfluenza, varicella-zoster, cytomeglavirus, Epstein-Barr, rotavirus, rhinovirus, adenovirus, coxsackieviruses, equine encephalitis, Japanese encephalitis, yellow fever, Rift Valley fever,and lymphocytic choriomeningitis.

### ii. Bacterial Antigens

Bacterial antigens can originate from any bacteria including, but not limited to, *Actinomyces, Anabaena, Bacillus, Bacteroides, Bdellovibrio, Bordetella, Borrelia, Campylobacter, Caulobacter, Chlamydia, Chlorobium, Chromatium, Clostridium, Corynebacterium, Cytophaga, Deinococcus, Escherichia, Francisella, Halobacterium, Heliobacter, Haemophilus, Hemophilus influenza type B (HIB), Hyphomicrobium, Legionella, Leptspirosis, Listeria, Meningococcus A, B and C, Methanobacterium, Mieroeoceus, Myobacterium, Mycoplasma, Myxococcus, Neisseria, Nitrobacter, Oscillatoria, Prochloron, Proteus, Pseudomonas, Phodospirillum, Rickettsia, Salmonella, Shigella, Spirillum, Spirochaeta, Staphylococcus, Streptococcus, Streptomyces, Sulfolobus, Thermoplasma, Thiobacillus, and Treponema, Vibrio,* and *Yersinia.*

### iii. Parasitic Antigens

Antigens of parasites can be obtained from parasites such as, but not limited to, antigens derived from *Cryptococcus neoformans, Histoplasma capsulatum, Candida albicans, Candida tropicalis, Nocardia asteroides, Rickettsia ricketsii, Rickettsia typhi, Mycoplasma pneumoniae, Chlamydial psittaci, Chlamydial trachomatis, Plasmodium falciparum, Trypanosoma brucei, Entamoeba histolytica, Toxoplasma gondit, Trichomonas vaginalis* and *Schistosoma mansoni.* These include Sporozoan antigens, Plasmodian antigens, such as all or part of a Circumsporozoite protein, a Sporozoite surface protein, a liver stage antigen, an apical membrane associated protein, or a Merozoite surface protein.

### iv. Tumor Antigens

The antigen can be a tumor antigen, including a tumor-associated or tumor-specific antigen, such as, but not limited to, alpha-actinin-4, Bcr-Abl fusion protein, Casp-8, beta-catenin, cdc27, cdk4, cdkn2a, coa-1, dek-can fusion protein, EF2, ETV6-AML1 fusion protein, LDLR-fucosyltransferaseAS fusion protein, HLA-A2, HLA-A11, hsp70-2, KIAAO205, Mart2, Mum-1, 2, and 3, neo-PAP, myosin class I, OS-9, pml-RARα fusion protein, PTPRK, K-ras, N-ras, Triosephosphate isomeras, Bage-1, Gage 3,4,5,6,7, GnTV, Herv-K-mel, Lage-1, Mage-A1,2,3,4,6,10,12, Mage-C2, NA-88, NY-Eso-1/Lage-2, SP17, SSX-2, and TRP2-Int2, MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, α-fetoprotein, 13HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB\70K, NY-CO-1, RCAS1, SDCCAG16, TA-90 (Mac-2 binding protein\cyclophilin C-associated protein), TAAL6, TAG72, TLP, and TPS. Tumor antigens, such as BCG, may also be used as an immunostimulant to adjuvant.

### 2. Adjuvants

Optionally, the vaccines may include an adjuvant. The adjuvant can be, but is not limited to, one or more of the following: oil emulsions (e.g., Freund's adjuvant); saponin formulations; virosomes and viral-like particles; bacterial and microbial derivatives; immunostimulatory oligonucleotides; ADP-ribosylating toxins and detoxified derivatives; alum; BCG; mineral-containing compositions (e.g., mineral salts, such as aluminium salts and calcium salts, hydroxides, phosphates, sulfates, etc.); bioadhesives and/or mucoadhesives; microparticles; liposomes; polyoxyethylene ether and polyoxyethylene ester formulations; polyphosphazene; muramyl peptides; imidazoquinolone compounds; and surface active substances (e.g. lysolecithin, pluronic polyols, polyanions, peptides, oil emulsions, keyhole limpet hemocyanin, and dinitrophenol).

Adjuvants may also include immunomodulators such as cytokines, interleukins (e.g., IL-1, IL-2, IL-4, IL-5, IL-6, IL-7, IL-12, etc.), interferons (e.g., interferon-.gamma.), macrophage colony stimulating factor, and tumor necrosis factor. In addition to variant PD-L2 polypeptides, other costimulatory molecules, including other polypeptides of the B7 family, may be administered. Such proteinaceous adjuvants may be provided as the full-length polypeptide or an active fragment thereof, or in the form of DNA, such as plasmid DNA.

### IV. Methods of Use

PD-1 antagonists and variants thereof, as well as nucleic acids encoding these polypeptides and fusion proteins, or cells expressing PD-1 antagonist can be used to enhance a primary immune response to an antigen as well as increase effector cell function such as increasing antigen-specific proliferation of T cells, enhancing cytokine production by T cells, and stimulating differentiation. The PD-1 antagonist compositions can be administered to a subject in need thereof in an effective amount to overcome T cell exhaustion and/or T cell anergy. Overcoming T cell exhaustion or T cell anergy can be determined by measuring T cell function using known techniques. Preferred PD-1 antagonist polypeptides are engineered to bind to PD-1 without triggering inhibitory signal transduction through PD-1 and retain the ability to costimulate T cells.

*In vitro* application of the PD-1 antagonist can be useful, for example, in basic scientific studies of immune mechanisms or for production of activated T cells for use in studies of T cell function or, for example, passive immunotherapy. Furthermore, PD-1 antagonist can be added to *in vitro* assays (e.g., T cell proliferation assays) designed to test for immunity to an antigen of interest in a subject from which the T cells were obtained. Addition of a PD-1 antagonist to such assays would be expected to result in a more potent, and therefore more readily detectable, *in vitro* response.

### A. Administration of PD-1 Antagonists for

### Immunoenhancement

The PD-1 antagonists are generally useful *in vivo* and *ex vivo* as immune response-stimulating therapeutics. In a preferred embodiment, the compositions are useful for treating infections in which T cell exhaustion or T cell anergy has occurred causing the infection to remain with the host over a prolonged period of time. Exemplary infections to be treated are chronic infections cause by a hepatitis virus, a human immunodeficiency virus (HIV), a human T-lymphotrophic virus (HTLV), a herpes virus, an Epstein-Barr virus, or a human papilloma virus. It will be appreciated that other infections can also be treated using the PD-1 antagonists. The disclosed compositions are also useful as part of a vaccine. In a preferred embodiment, the type of disease to be treated or prevented is a chronic infectious disease caused by a bacterium, virus, protozoan, helminth, or other microbial pathogen that enters intracellularly and is attacked, i.e., by cytotoxic T lymphocytes.

Chronic infections in human and animal models are associated with a failure of the host immune response to generate and sustain functional CD8⁺ and CD4⁺ T-cell populations, which also results in poor antibody responses to neutralize infectivity. This loss of function is referred to as T cell exhaustion. T cell anergy is a tolerance mechanism in which the lymphocyte is intrinsically functionally inactivated following an antigen encounter, but remains alive for an extended period of time in a hyporesponsive state. One method for treating chronic infection is to revitalize exhausted T cells or to reverse T cell exhaustion in a subject as well as overcoming T cell anergy. Reversal of T cell exhaustion can be achieved by interfering with the interaction between PD-1 and its ligands PD-L1 (B7-H1) and PD-L2 (PD-L2). Acute, often lethal, effects of pathogens can be mediated by toxins or other factors that fail to elicit a sufficient immune response prior to the damage caused by the toxin. This may be overcome by interfering with the interaction between PD-1 and its ligands, allowing for a more effective, rapid immune response.

Because viral infections are cleared primarily by T-cells, an increase in T-cell activity is therapeutically useful in situations where more rapid or thorough clearance of an infective viral agent would be beneficial to an animal or human subject. Thus, the PD-1 antagonists can be administered for the treatment of local or systemic viral infections, including, but not limited to, immunodeficiency (e.g., HIV), papilloma (e.g., HPV), herpes (e.g., HSV), encephalitis, influenza (e.g., human influenza virus A), and common cold (e.g., human rhinovirus) viral infections. For example, pharmaceutical formulations including the PD-1 antagonist compositions can be administered topically to treat viral skin diseases such as herpes lesions or shingles, or genital warts. Pharmaceutical formulations of PD-1 antagonist compositions can also be administered to treat systemic viral diseases, including, but not limited to, AIDS, influenza, the common cold, or encephalitis.

Representative infections that can be treated, include but are not limited to infections cause by microoganisms including, but not limited to, *Actinomyces, Anabaena, Bacillus, Bacteroides, Bdellovibrio, Bordetella, Borrelia, Campylobacter, Caulobacter, Chlamydia, Chlorobium, Chromatium, Clostridium, Corynebacterium, Cytophaga, Deinococcus, Escherichia, Francisella, Halobacterium, Heliobacter, Haemophilus, Hemophilus influenza type B (HIB), Histoplasma, Hyphomicrobium, Legionella, Leishmania, Leptspirosis, Listeria, Meningococcus A, B and C, Methanobacterium, Micrococcus, Myobacterium, Mycoplasma, Myxococcus, Neisseria, Nitrobacter, Oscillatoria, Prochloron, Proteus, Pseudomonas, Phodospirillum, Rickettsia, Salmonella, Shigella, Spirillum, Spirochaeta, Staphylococcus, Streptococcus, Streptomyces, Sulfolobus, Thermoplasma, Thiobacillus, and Treponema, Vibrio, Yersinia, Cryptococcus neoformans, Histoplasma capsulatum, Candida albicans, Candida tropicalis, Nocardia asteroides, Rickettsia ricketsii, Rickettsia typhi, Mycoplasma pneumoniae, Chlamydial psittaci, Chlamydial trachomatis, Plasmodium falciparum, Plasmodium vivax, Trypanosoma brucei, Entamoeba histolytica, Toxoplasma gondii, Trichomonas vaginalis* and *Schistosoma mansoni.*

### B. Use of PD-1 Antagonists in Vaccines

The PD-1 antagonists or nucleic acids encoding the same may be administered alone or in combination with any other suitable treatment. In one embodiment the PD-1 antagonist can be administered in conjunction with, or as a component of a vaccine composition as described above. Suitable components of vaccine compositions are described above. The disclosed PD-1 antagonist can be administered prior to, concurrently with, or after the administration of a vaccine. In one embodiment the PD-1 antagonist composition is administered at the same time as administration of a vaccine.

PD-1 antagonist compositions may be administered in conjunction with prophylactic vaccines, which confer resistance in a subject to subsequent exposure to infectious agents, or in conjunction with therapeutic vaccines, which can be used to initiate or enhance a subject's immune response to a pre-existing antigen, such as a viral antigen in a subject infected with a virus.

The desired outcome of a prophylactic, therapeutic or de-sensitized immune response may vary according to the disease, according to principles well known in the art. For example, an immune response against an infectious agent may completely prevent colonization and replication of an infectious agent, affecting "sterile immunity" and the absence of any disease symptoms. However, a vaccine against infectious agents may be considered effective if it reduces the number, severity or duration of symptoms; if it reduces the number of individuals in a population with symptoms; or reduces the transmission of an infectious agent. Similarly, immune responses against cancer, allergens or infectious agents may completely treat a disease, may alleviate symptoms, or may be one facet in an overall therapeutic intervention against a disease.

The PD-1 antagonists induce an improved effector cell response such as a CD4 T-cell immune response, against at least one of the component antigen(s) or antigenic compositions compared to the effector cell response obtained with the corresponding composition without the PD-1 antagonist. The term "improved effector cell response" refers to a higher effector cell response such as a CD4 response obtained in a human patient after administration of the vaccine composition than that obtained after administration of the same composition without a PD-1 antagonist. For example, a higher CD4 T-cell response is obtained in a human patient upon administration of an immunogenic composition containing an PD-1 antagonist, preferably PD-L2-Ig, and an antigenic preparation compared to the response induced after administration of an immunogenic composition containing the antigenic preparation thereof which is un-adjuvanted. Such a formulation will advantageously be used to induce anti-antigen effector cell response capable of detection of antigen epitopes presented by MHC class II molecules.

The improved effector cell response can be obtained in an immunologically unprimed patient, i.e. a patient who is seronegative to the antigen. This seronegativity may be the result of the patient having never faced the antigen (so-called "naïve" patient) or, alternatively, having failed to respond to the antigen once encountered. Preferably the improved effector cell response is obtained in an immunocompromised subject such as an elderly, typically 65 years of age or above, or an adult younger than 65 years of age with a high risk medical condition ("high risk" adult), or a child under the age of two.

The improved effector cell response can be assessed by measuring the number of cells producing any of the following cytokines: (1) cells producing at least two different cytokines (CD40L, IL-2, IFN-gamma, TNF-alpha); (2) cells producing at least CD40L and another cytokine (IL-2, TNF-alpha, IFN-gamma); (3) cells producing at least IL-2 and another cytokine (CD40L, TNF-alpha, IFN-gamma); (4) cells producing at least IFN-gamma. and another cytokine (IL-2, TNF-alpha., CD40L); (5) and cells producing at least TNF-alpha and another cytokine (IL-2, CD40L, IFN-gamma)

An improved effector cell response is present when cells producing any of the above cytokines will be in a higher amount following administration of the vaccine composition compared to the administration of the composition without a PD-1 antagonist. Typically at least one, preferably two of the five conditions mentioned above will be fulfilled. In a particular embodiment, cells producing all four cytokines will be present at a higher number in the vaccinated group compared to the un-vaccinated group.

The immunogenic compositions may be administered by any suitable delivery route, such as intradermal, mucosal e.g. intranasal, oral, intramuscular or subcutaneous. Other delivery routes are well known in the art. The intramuscular delivery route is preferred for the immunogenic compositions. Intradermal delivery is another suitable route. Any suitable device may be used for intradermal delivery, for example short needle devices. Intradermal vaccines may also be administered by devices which limit the effective penetration length of a needle into the skin. Jet injection devices which deliver liquid vaccines to the dermis via a liquid jet injector or via a needle which pierces the stratum corneum and produces a jet which reaches the dermis can also be used. Jet injection devices are known in the art. Ballistic powder/particle delivery devices which use compressed gas to accelerate vaccine in powder form through the outer layers of the skin to the dermis can also be used. Additionally, conventional syringes can be used in the classical Mantoux method of intradermal administration.

Another suitable administration route is the subcutaneous route. Any suitable device may be used for subcutaneous delivery, for example classical needle. Preferably, a needle-free jet injector service is used. Needle-free injectors are known in the art. More preferably the device is pre-filled with the liquid vaccine formulation.

Alternatively the vaccine is administered intranasally. Typically, the vaccine is administered locally to the nasopharyngeal area, preferably without being inhaled into the lungs. It is desirable to use an intranasal delivery device which delivers the vaccine formulation to the nasopharyngeal area, without or substantially without it entering the lungs. Preferred devices for intranasal administration of the vaccines are spray devices. Nasal spray devices are commercially available. Nebulizers produce a very fine spray which can be easily inhaled into the lungs and therefore does not efficiently reach the nasal mucosa. Nebulizers are therefore not preferred. Preferred spray devices for intranasal use are devices for which the performance of the device is not dependent upon the pressure applied by the user. These devices are known as pressure threshold devices. Liquid is released from the nozzle only when a threshold pressure is applied. These devices make it easier to achieve a spray with a regular droplet size. Pressure threshold devices suitable for use with the present invention are known in the art and are commercially available.

Preferred intranasal devices produce droplets (measured using water as the liquid) in the range 1 to 200 µm, preferably 10 to 120 µm. Below 10 µm there is a risk of inhalation, therefore it is desirable to have no more than about 5% of droplets below 10 µm. Droplets above 120 µm do not spread as well as smaller droplets, so it is desirable to have no more than about 5% of droplets exceeding 120 µm.

Bi-dose delivery is another feature of an intranasal delivery system for use with the vaccines. Bi-dose devices contain two sub-doses of a single vaccine dose, one sub-dose for administration to each nostril. Generally, the two sub-doses are present in a single chamber and the construction of the device allows the efficient delivery of a single sub-dose at a time. Alternatively, a monodose device may be used for administering the vaccines.

The immunogenic composition may be given in two or more doses, over a time period of a few days, weeks or months. In one embodiment, different routes of administration are utilized, for example, for the first administration may be given intramuscularly, and the boosting composition, optionally containing a PD-1 antagonist, may be administered through a different route, for example intradermal, subcutaneous or intranasal.

The improved effector cell response conferred by the immunogenic composition may be ideally obtained after one single administration. The single dose approach is extremely relevant in a rapidly evolving outbreak situation including bioterrorist attacks and epidemics. In certain circumstances, especially for the elderly population, or in the case of young children (below 9 years of age) who are vaccinated for the first time against a particular antigen, it may be beneficial to administer two doses of the same composition. The second dose of the same composition (still considered as 'composition for first vaccination') can be administered during the on-going primary immune response and is adequately spaced in time from the first dose. Typically the second dose of the composition is given a few weeks, or about one month, e.g. 2 weeks, 3 weeks, 4 weeks, 5 weeks, or 6 weeks after the first dose, to help prime the immune system in unresponsive or poorly responsive individuals.

In a specific embodiment, the administration of the immunogenic composition alternatively or additionally induces an improved B-memory cell response in patients administered with the adjuvanted immunogenic composition compared to the B-memory cell response induced in individuals immunized with the un-adj uvanted composition. An improved B-memory cell response is intended to mean an increased frequency of peripheral blood B lymphocytes capable of differentiation into antibody-secreting plasma cells upon antigen encounter as measured by stimulation of *in vitro* differentiation (see Example sections, e.g. methods of Elispot B cells memory).

In a still another embodiment, the immunogenic composition increases the primary immune response as well as the CD8 response. The administration of a single dose of the immunogenic composition for first vaccination provides better sero-protection and induces an improved CD4 T-cell, or CD8 T-cell immune response against a specific antigen compared to that obtained with the un-adjuvanted formulation. This may result in reducing the overall morbidity and mortality rate and preventing emergency admissions to hospital for pneumonia and other influenza-like illness. This method allows inducing a CD4 T cell response which is more persistent in time, e.g. still present one year after the first vaccination, compared to the response induced with the un-adjuvanted formulation.

Preferably the CD4 T-cell immune response, such as the improved CD4 T-cell immune response obtained in an unprimed subject, involves the induction of a cross-reactive CD4 T helper response. In particular, the amount of cross-reactive CD4 T cells is increased. The term "cross-reactive" CD4 response refers to CD4 T-cell targeting shared epitopes for example between influenza strains.

The dose of PD-1 antagonist enhances an immune response to an antigen in a human. In particular a suitable PD-1 antagonist amount is that which improves the immunological potential of the composition compared to the unadjuvanted composition, or compared to the composition adjuvanted with another PD-1 antagonist amount. Usually an immunogenic composition dose will range from about 0.5 ml to about 1 ml. Typical vaccine doses are 0.5 ml, 0.6 ml, 0.7 ml, 0.8 ml, 0.9 ml or 1 ml. In a preferred embodiment, a final concentration of 50 µg of PD-1 antagonist, preferably PD-L2-Ig, is contained per ml of vaccine composition, or 25 µg per 0.5 ml vaccine dose. In other preferred embodiments, final concentrations of 35.7 µg or 71.4 µg of PD-1 antagonist is contained per ml of vaccine composition. Specifically, a 0.5 ml vaccine dose volume contains 25 µg or 50 µg of PD-1 antagonist per dose. In still another embodiment, the dose is 100 µg or more. Immunogenic compositions usually contain 15 µg of antigen component as measured by single radial immunodiffusion (SRD) (J. M. Wood et al.: J. Biol. Stand. 5 (1977) 237-247; J. M. Wood et al., J. Biol. Stand. 9 (1981) 317-330).

Subjects can be revaccinated with the immunogenic compositions. Typically revaccination is made at least 6 months after the first vaccination(s), preferably 8 to 14 months after, more preferably at around 10 to 12 months after.

The immunogenic composition for revaccination (the boosting composition) may contain any type of antigen preparation, either inactivated or live attenuated. It may contain the same type of antigen preparation, for example split influenza virus or split influenza virus antigenic preparation thereof, a whole virion, a purified subunit vaccine or a virosome, as the immunogenic composition used for the first vaccination. Alternatively the boosting composition may contain another type of antigen, i.e. split influenza virus or split influenza virus antigenic preparation thereof, a whole virion, a purified subunit vaccine or a virosome, than that used for the first vaccination.

With regard to vaccines against a virus, a boosting composition, where used, is typically given at the next viral season, e.g. approximately one year after the first immunogenic composition. The boosting composition may also be given every subsequent year (third, fourth, fifth vaccination and so forth). The boosting composition may be the same as the composition used for the first vaccination.

Preferably revaccination induces any, preferably two or all, of the following: (i) an improved effector cell response against the antigenic preparation, or (ii) an improved B cell memory response or (iii) an improved humoral response, compared to the equivalent response induced after a first vaccination with the antigenic preparation without a PD-1 antagonist. Preferably the immunological responses induced after revaccination with the immunogenic antigenic preparation containing the PD-1 antagonist are higher than the corresponding response induced after the revaccination with the un-adjuvanted composition.

The immunogenic compositions can be monovalent or multivalent, i.e, bivalent, trivalent,or quadrivalent. Preferably the immunogenic composition thereof is trivalent or quadrivalent. Multivalent refers to the number of sources of antigen, typically from different species or strains. With regard to viruses, at least one strain is associated with a pandemic outbreak or has the potential to be associated with a pandemic outbreak.

### C. Targeting Antigen Presenting Cells

Another embodiment provides contacting antigen presenting cells (APCs) with one or more of the disclosed PD-1 antagonists in an amount effective to inhibit, reduce or block PD-1 signal transduction in the APCs. Blocking PD-1 signal transduction in the APCs reinvigorates the APCs enhancing clearance of intracellular pathogens, or cells infected with intracellular pathogens.

### D. Combination Therapies

The PD-1 antagonist compositions can be administered to a subject in need thereof alone or in combination with one or more additional therapeutic agents. The additional therapeutic agents are selected based on the condition, disorder or disease to be treated. For example, aPD-1 antagonist can be co-administered with one or more additional agents that function to enhance or promote an immune response.

### E. Modulating Binding Properties

Binding properties of the PD-1 antagonists are relevant to the dose and dose regime to be administered. Existing antibody PD-1 antagonists such as MDX-1106 demonstrate sustained occupancy of 60-80% of PD-1 molecules on T cells for at least 3 months following a single dose (Brahmer, et al. J. Clin. Oncology, 27:(155) 3018 (2009)). In preferred embodiments, the disclosed PD-1 antagonists have binding properties to PD-1 that demonstrate a shorter term, or lower percentage, of occupancy of PD-1 molecules on immune cells. For example, the disclosed PD-1 antagonists typically show less than 5, 10, 15, 20, 25, 30, 35, 40, 45, of 50% occupancy of PD-1 molecules on immune cells after one week, two weeks, three weeks, or even one month after administration of a single dose. In other embodiments, the disclosed PD-1 antagonists have reduced binding affinity to PD-1 relative to MDX-1106. In relation to an antibody such as MDX-1106, the PD-1-Ig fusion protein has a relatively modest affinity for its receptor, and should therefore have a relatively fast off rate.

In other embodiments, the PD-1 antagonists are administered intermittently over a period of days, weeks or months to elicit periodic enhanced immune response which are allowed to diminish prior to the next administration, which may serve to initiate an immune response, stimulate an immune response, or enhance an immune response.

### Examples

The present invention may be further understood by reference to the following non-limiting examples.

### Example 1: B7-DC binding to PD-1

PD-1 binding activity of human B7-DC-Ig was assessed by ELISA. 96-well ELISA plates were coated with 100 µL 0.75 ug/mL recombinant human PD-1/Fc (R&D Systems) diluted in BupH Carbonate/Bicarbonate pH 9.4 buffer (Pierce) for 2 hours and then blocked with BSA solution (Jackson ImmunoResearch) for 90-120 minutes. Serially diluted human B7-DC-Ig as well as human IgG1 isotype control were allowed to bind for 90 minutes. Bound B7-DC-Ig was detected using 100 uL of 0.5 ug/mL biotin conjugated anti-human B7-DC clone MIH18 (eBioscience) followed by 1:1000 diluted HRP-Streptavidin (BD Bioscience) and TMB substrate (BioFX). Absorbance at 450 nm was read using a plate reader (Molecular Devices) and data were analyzed in SoftMax using a 4-parameter logistic fit.

PD-1 binding activity of murine B7-DC-Ig was assessed by ELISA. 96-well ELISA plates were coated with 100 µL 0.75 ug/mL recombinant mouse PD-1/Fc (R&D Systems) diluted in BupH Carbonate/Bicarbonate pH 9.4 buffer (Pierce) for 2 hours and then blocked with BSA solution (Candor-Bioscience) for 90 minutes. Serially diluted murine B7-DC-Ig (wild type, as well as D111S and K113S mutants that were selected for reduced binding to PD-1) as well as murine IgG2a isotype control were allowed to bind for 90 minutes. Bound B7-DC-Ig was detected using 100 uL of 0.25 ug/mL biotin conjugated anti-mouse B7-DC clone 112 (eBioscience) followed by 1:2000 diluted HRP-Streptavidin (BD Bioscience) and TMB substrate (BioFX). Absorbance at 450 nm was read using a plate reader (Molecular Devices) and data were analyzed in SoftMax using a 4-parameter logistic fit.

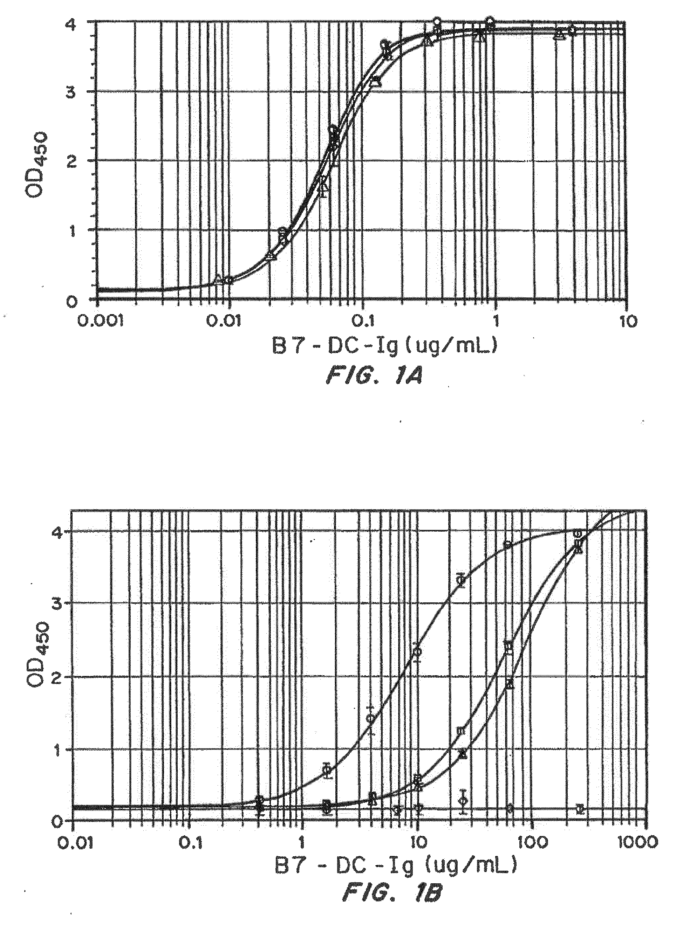

Figures 1A and 1B show line graphs of OD₄₅₀ versus amount of B7-DC-Ig (ug/ml) in a PD-1 binding ELISA. Figure 1A shows binding of four different lots of human B7-DC-Ig. Figure 1B shows binding of wild type murine B7-DC-Ig (circle), the DS mutant (B7-DC-Ig with the D111S substitution; triangle) and KS mutant (B7-DC-Ig with the K113S substitution; square), and murine IgG2a isotype control (diamond).

### Example 2: B7-DC binding to PD-1 expressing CHO cells

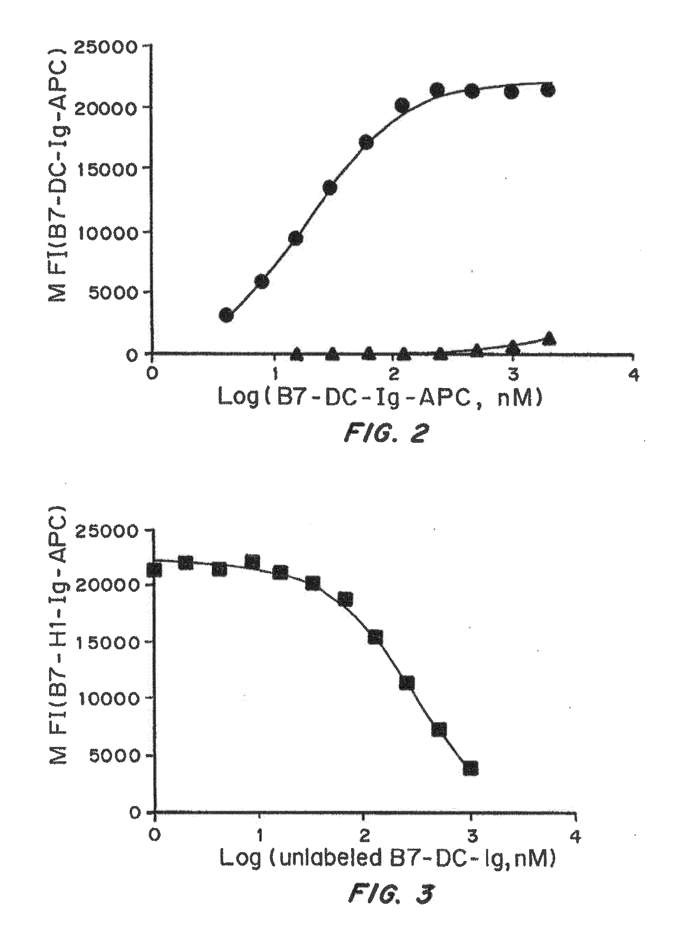

B7-DC-Ig was first conjugated with allophycocyanin (APC) and then incubated at various concentrations with a CHO cell line constitutively expressing PD-1 or parent CHO cells that do not express PD-1. Binding was analyzed by flow cytometry. Figure 2 shows the median fluorescence intensity (MFI) of B7-DC-Ig-APC (y-axis) as a function of the concentration of probe (x-axis). B7-DC-Ig-APC binds to CHO.PD-1 cells (solid circle) but not untransfected CHO cells (gray triangle).

### Example 3: B7-DC-Ig competes with B7-H1 for binding to PD-1

B7-H1-Ig was first conjugated with allophycocyanin (APC). Unlabeled B7-DC-Ig at various concentrations was first incubated with a CHO cell line constitutively expressing PD-1 before adding B7-H1-Ig-APC to the probe and cell mixture. Figure 3 shows the median fluorescence intensity (MFI) of B7-H1-Ig-APC (y-axis) as a function of the concentration of unlabeled B7-DC-Ig competitor (x-axis) added. As the concentration of unlabeled B7-DC-Ig is increased the amount of B7-H1-Ig-APC bound to CHO cells decreases, demonstrating that B7-DC-Ig competes with B7-H1 for binding to PD-1.

### Example 4: Combination of cyclophosphamide and B7-DC-Ig can

### generate tumor specific, memory cytotoxic T lymphocytes

Balb/C mice at age of 9 to 11 weeks were implanted subcutaneously with 1.0 x 105 CT26 colorectal tumor cells. On day 10 post tumor implantation, mice received 100 mg/kg of cyclophosphamide. B7-DC-Ig treatment started 1 day later, on day 11. Mice were treated with 100 ug of B7-DC-Ig, 2 doses per week, for 4 weeks and total 8 doses. 75% of the mice that received the CTX + B7-DC-Ig treatment regimen eradicated the established tumors by Day 44, whereas all mice in the control CTX alone group died as a result of tumor growth or were euthanized because tumors exceeded the sizes approved by IACUC .

Mice eradicated established CT26 colorectal tumors from the above described experiment were rechallenged with 1x105 CT26 cells on Day 44 and Day 70. No tumors grew out from the rechallenge suggesting they had developed long term anti-tumor immunity from the cyclophosphamide and B7-DC-Ig combination treatment. All mice in the vehicle control group developed tumors. This demonstrated the effectiveness of the treatment on established tumors and that the B7-DCIg combination treatment resulted in memory responses to tumor antigens.

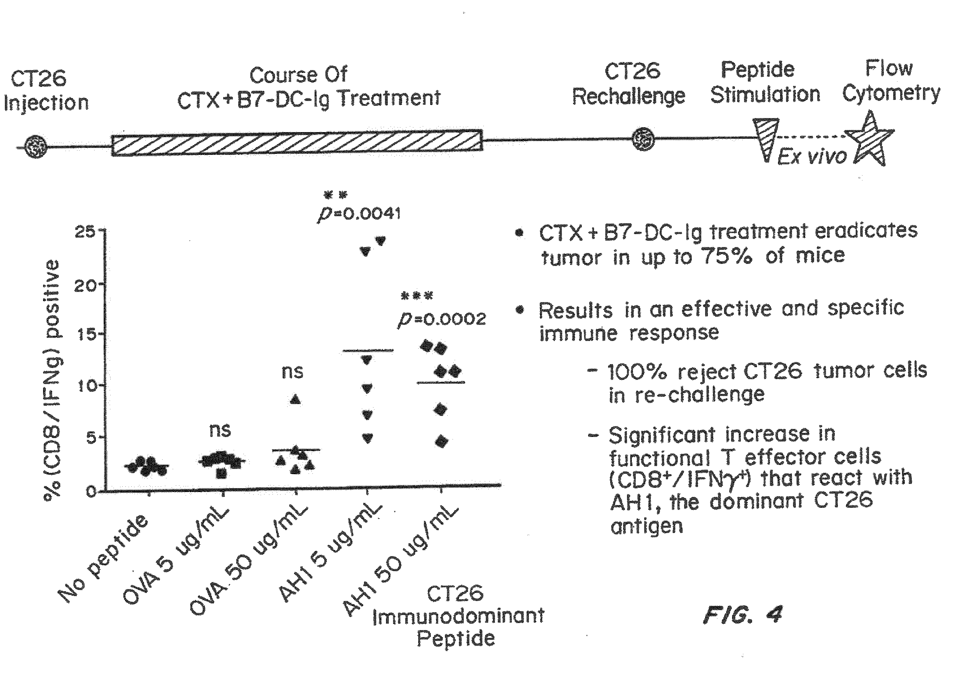

Mice eradiated established CT26 colorectal tumors from the above described experiment were rechallenged with 2.5x105 CT26 cells on Day 44. Seven days later, mouse spleens were isolated. Mouse splenocytes were pulsed with 5 or 50 ug/mL of ovalbumin (OVA) or AH1 peptides for 6 hours in the presence of a Golgi blocker (BD BioScience). Memory T effector cells were analyzed by assessing CD8+/IFNγ+ T cells. Results in Figure 4 show that there were significant amount of CT26 specific T effector cells in the CT26 tumor-eradicated mice.

### Example 5: B7-DC-Ig reduced HSV viral particle shedding and enhanced mouse survival.

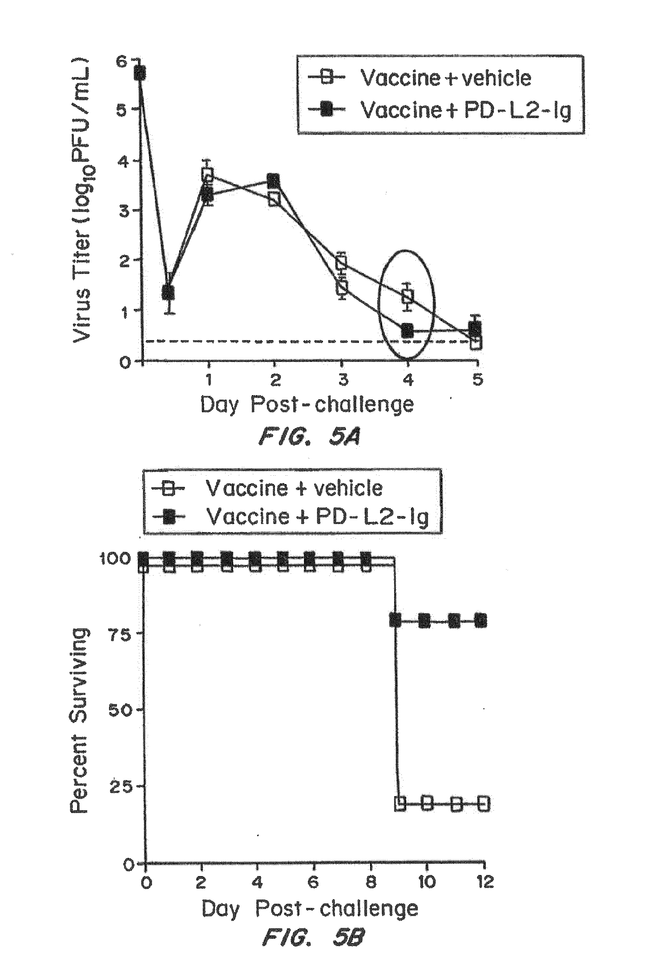

Balb/C mice at age of 8 to 10 weeks were first immunized with a live attenuated HSV-2 vaccine at a dose of 4x10⁴ PFU together with vehicle (open square) or 300 µg of B7-DC-Ig (solid square) (Figures 2A and 2B). One month later, all the mice were challenged with 5x10⁵ PFU of HSV-2 strain G-6 intravaginally. Figure 5A reveals viral particle titers of swabs of vaginal area at 9 hr, 1,2,3,4, and 5 days post virus challenge. Figure 5B shows mouse survival on day 12 post virus challenge. This demonstrates that the presence B7-DC-Ig in combination with a vaccine can reduce viral load and increase survival of animals.

The invention will now be defined by the following clauses:
1. A method of modulating an immune response comprising administering an effective amount a PD-1 antagonist to induce, augment, or enhance an immune response against a disease pathogen or infected cell, wherein the dose of the molecule, the timing of administration of the molecule and/or the affinity of the molecule allows for intermittent access of a ligand to the PD-1 receptor.
2. The method of clause 1 wherein the PD-1 antagonist inhibits or reduces binding of endogenous PD-L1 to PD-1.
3. The method of clause 1 wherein the PD-1 antagonist inhibits or reduces binding of endogenous PD-L2 to PD-1.
4. The method of clause 1 wherein the PD-1 antagonist binds to PD-1.
5. The method of clause 1 wherein the PD-1. antagonist is selected from the group consisting of PD-1, PD-L1, PD-L2, B7.1, and fragments thereof.
6. The method of clause 1 wherein the molecule binds to PD-1 or a ligand thereof for three months or less after *in vivo* administration.
7. The method of clause 1 wherein more than one PD-1 antagonist is administered.
8. The method of clause 1, wherein the infection is a chronic viral infection, a bacterial infection, a fungal infection, a mycoplasm infection, a parasitic infection, elicits disease mediated by a toxin during the acute phase of infection or where the infection is characterized by reduced T cell response.
9. The method of clause 8, wherein the viral infection is an infection with a hepatitis virus, a human immunodeficiency virus, a human T-lymphotrophic virus, a herpes virus, an Epstein-Barr virus, filovirus, a human papilloma virus, an Epstein Barr virus, an influenza virus, a respiratory synticial virus, an encephalitis virus, a dengue fever virus, and a papilloma virus.
10. The method of clsuse 4, wherein the parasitic infection is malaria or Leishmania.
11. The method of clause 8, wherein the bacterial infection is caused by a bacterium selected from the group consisting of *Mycobacterium tuberculosis, Bacillus anthracis, Staphylococcus, Listeria,* and *Clamydia trachomatis.*
12. The method of clause 1 further comprising administering a disease antigen in combination with the PD-1 antagonist to enhance an immune response against the disease.
13. The method of clause 1, wherein the PD-1 antagonist is a fusion protein of a PD-1 ligand.
14. The method of clause 13, wherein the fusion protein comprises the extracellular domain of PD-L2 or a fragment thereof capable of binding to PD-1.
15. The method of clause 14 wherein the fusion protein has an amino acid sequence according to SEQ ID NO:83.
16. The method of clause 1, further comprising administering with the PD-1 antagonist an additional active agent selected from the group consisting of immunomodulators, agents that deplete or inhibit the function of Tregs, and costimulatory molecules.
17. The method of clause 17, wherein the additional active agent is an agent that depletes or inhibits the function of CD4+CD25+ Tregs.
18. The method of clause 17, wherein the agent that depletes or inhibits the function of CD4+CD25+ Tregs is cyclophosphamide.
19. The method of clause 1 for enhancing antigen presenting cell function comprising contacting APCs with a PD-1 antagonist in an amount effective to inhibit, reduce, or block PD-1 signal transduction in the APCs or enhance clearance of diseased or infected cells.
20. A composition comprising a PD-1 antagonist in combination with one or more disease antigens.
21. A composition comprising a PD-1 antagonist in combination with a vaccine.

## Claims

1. A pharmaceutical composition comprising a fusion protein wherein the fusion protein comprises the extracellular domain of PD-L2 or a fragment thereof capable of binding to PD-1 for use in a method of modulating an immune response in a human, the method comprising administering an effective amount of the pharmaceutical composition.

2. The pharmaceutical composition for use according to claim 1, wherein the fusion protein comprises the amino acid set forth in SEQ ID NO:57 at a dose between 5 mg/kg and 20 mg/kg to said human to induce, augment, or enhance an immune response against an infection, wherein the polypeptide binds to PD-1 for three months or less after *in vivo* administration.

3. The pharmaceutical composition for use according to claim 1 or claim 2,
wherein the infection is a chronic viral infection, a bacterial infection, a fungal infection, a mycoplasm infection, a parasitic infection, elicits disease mediated by a toxin during the acute phase of infection or where the infection is **characterized by** reduced T cell response

4. The pharmaceutical composition for use according to any one of the previous claims, wherein the viral infection is an infection with a hepatitis virus, a human immunodeficiency virus, a human T-lymphotrophic virus, a herpes virus, an Epstein-Barr virus, filovirus, a human papilloma virus, an Epstein Barr virus, an influenza virus, a respiratory synticial virus, an encephalitis virus, a dengue fever virus, and a papilloma virus.

5. The pharmaceutical composition for use according to any one of the previous claims, wherein the parasitic infection is malaria or Leishmania.

6. The pharmaceutical composition for use according to any one of the previous claims, wherein the bacterial infection is caused by a bacterium selected from the group consisting of *Mycobacterium tuberculosis, Bacillus anthracis, Staphylococcus, Listeria,* and *Clamydia trachomatis.*

7. The pharmaceutical composition for use according to any one of the previous claims, wherein the use further comprises administering one or more disease antigens in combination with the fusion protein to enhance an immune response against the disease.

8. The pharmaceutical composition for use according to any one of the previous claims, wherein the use further comprises administering with the PD-1 antagonist an additional active agent selected from the group consisting of immunomodulators, agents that deplete or inhibit the function of Tregs, and costimulatory molecules.

9. The pharmaceutical composition for use according to claim 8, wherein the additional active agent is an agent that depletes or inhibits the function of CD4+CD25+ Tregs.

10. The pharmaceutical composition for use according to claim 9, wherein the agent that depletes or inhibits the function of CD4+CD25+ Tregs is cyclophosphamide.

11. The pharmaceutical composition for use according to any one of the previous claims, wherein the use further comprises administering a vaccine in combination with the fusion protein to enhance an immune response against the disease.

12. The pharmaceutical composition for use according to any one of the previous claims, wherein the fusion protein binds to PD-1 without triggering signal transduction.

13. The pharmaceutical composition for use according to any one of the previous claims, wherein the fusion protein further comprises one or more domains of an Ig heavy chain constant region.

14. The pharmaceutical composition for use according to any one of the previous claims, wherein the fusion protein further comprises an amino acid sequence corresponding to the hinge, C_{H}2 and C_{H}3 regions of a human immunoglobulin Cγ1 chain.


SEQUENCE LISTING

<110>  Amplimmune, Inc.
       Solomon, Langermann

<120>  PD-1 ANTAGONISTS AND METHODS FOR TREATING INFECTIOUS DISEASE
       CROSS-REFERENCE TO RELATED APPLICATIONS

<130>  AMP 224.NP 3

<140>  PCT/US2009/054970
<141>  2009-08-25

<150>  61/091,502
<151>  2008-08-25

<150>  61/091,694
<151>  2008-08-25

<150>  61/091,709
<151>  2008-08-25

<150>  61/091,705
<151>  2008-08-25

<150>   61/142,548
<151>  2009-01-05

<150>  61/165,652
<151>  2009-04-01

<160>  60

<170>  PatentIn version 3.3

<210>  1
<211>  247
<212>  PRT
<213>  Mus musculus

<400>  1

Met Leu Leu Leu Leu Pro Ile Leu Asn Leu Ser Leu Gln Leu His Pro
1               5                   10                  15

Val Ala Ala Leu Phe Thr Val Thr Ala Pro Lys Glu Val Tyr Thr Val
            20                  25                  30

Asp Val Gly Ser Ser Val Ser Leu Glu Cys Asp Phe Asp Arg Arg Glu
        35                  40                  45

Cys Thr Glu Leu Glu Gly Ile Arg Ala Ser Leu Gln Lys Val Glu Asn
    50                  55                  60

Asp Thr Ser Leu Gln Ser Glu Arg Ala Thr Leu Leu Glu Glu Gln Leu
65                  70                  75                  80

Pro Leu Gly Lys Ala Leu Phe His Ile Pro Ser Val Gln Val Arg Asp

                85                  90                  95

Ser Gly Gln Tyr Arg Cys Leu Val Ile Cys Gly Ala Ala Trp Asp Tyr
            100                 105                 110

Lys Tyr Leu Thr Val Lys Val Lys Ala Ser Tyr Met Arg Ile Asp Thr
        115                 120                 125

Arg Ile Leu Glu Val Pro Gly Thr Gly Glu Val Gln Leu Thr Cys Gln
    130                 135                 140

Ala Arg Gly Tyr Pro Leu Ala Glu Val Ser Trp Gln Asn Val Ser Val
145                 150                 155                 160

Pro Ala Asn Thr Ser His Ile Arg Thr Pro Glu Gly Leu Tyr Gln Val
                165                 170                 175

Thr Ser Val Leu Arg Leu Lys Pro Gln Pro Ser Arg Asn Phe Ser Cys
            180                 185                 190

Met Phe Trp Asn Ala His Met Lys Glu Leu Thr Ser Ala Ile Ile Asp
        195                 200                 205

Pro Leu Ser Arg Met Glu Pro Lys Val Pro Arg Thr Trp Pro Leu His
    210                 215                 220

Val Phe Ile Pro Ala Cys Thr Ile Ala Leu Ile Phe Leu Ala Ile Val
225                 230                 235                 240

Ile Ile Gln Arg Lys Arg Ile
                245

<210> 2
<211> 228
<212> PRT
<213> Mus musculus

<400> 2

Leu Phe Thr Val Thr Ala Pro Lys Glu Val Tyr Thr Val Asp Val Gly
1               5                   10                  15

Ser Ser Val Ser Leu Glu Cys Asp Phe Asp Arg Arg Glu Cys Thr Glu
            20                  25                  30

Leu Glu Gly Ile Arg Ala Ser Leu Gln Lys Val Glu Asn Asp Thr Ser
        35                  40                  45

Leu Gln Ser Glu Arg Ala Thr Leu Leu Glu Glu Gln Leu Pro Leu Gly

50                  55                  60

Lys Ala Leu Phe His Ile Pro Ser Val Gln Val Arg Asp Ser Gly Gln
65                  70                  75                  80

Tyr Arg Cys Leu Val Ile Cys Gly Ala Ala Trp Asp Tyr Lys Tyr Leu
                85                  90                  95

Thr Val Lys Val Lys Ala Ser Tyr Met Arg Ile Asp Thr Arg Ile Leu
            100                 105                 110

Glu Val Pro Gly Thr Gly Glu Val Gln Leu Thr Cys Gln Ala Arg Gly
        115                 120                 125

Tyr Pro Leu Ala Glu Val Ser Trp Gln Asn Val Ser Val Pro Ala Asn
    130                 135                 140

Thr Ser His Ile Arg Thr Pro Glu Gly Leu Tyr Gln Val Thr Ser Val
145                 150                 155                 160

Leu Arg Leu Lys Pro Gln Pro Ser Arg Asn Phe Ser Cys Met Phe Trp
                165                 170                 175

Asn Ala His Met Lys Glu Leu Thr Ser Ala Ile Ile Asp Pro Leu Ser
            180                 185                 190

Arg Met Glu Pro Lys Val Pro Arg Thr Trp Pro Leu His Val Phe Ile
        195                 200                 205

Pro Ala Cys Thr Ile Ala Leu Ile Phe Leu Ala Ile Val Ile Ile Gln
    210                 215                 220

Arg Lys Arg Ile
225

<210>  3
<211>  273
<212>  PRT
<213>  Homo sapiens

<400>  3

Met Ile Phe Leu Leu Leu Met Leu Ser Leu Glu Leu Gln Leu His Gln
1               5                   10                  15

Ile Ala Ala Leu Phe Thr Val Thr Val Pro Lys Glu Leu Tyr Ile Ile
            20                  25                  30

Glu His Gly Ser Asn Val Thr Leu Glu Cys Asn Phe Asp Thr Gly Ser

35                  40                  45

His Val Asn Leu Gly Ala Ile Thr Ala Ser Leu Gln Lys Val Glu Asn
    50                  55                  60

Asp Thr Ser Pro His Arg Glu Arg Ala Thr Leu Leu Glu Glu Gln Leu
65                  70                  75                  80

Pro Leu Gly Lys Ala Ser Phe His Ile Pro Gln Val Gln Val Arg Asp
                85                  90                  95

Glu Gly Gln Tyr Gln Cys Ile Ile Ile Tyr Gly Val Ala Trp Asp Tyr
            100                 105                 110

Lys Tyr Leu Thr Leu Lys Val Lys Ala Ser Tyr Arg Lys Ile Asn Thr
        115                 120                 125

His Ile Leu Lys Val Pro Glu Thr Asp Glu Val Glu Leu Thr Cys Gln
    130                 135                 140

Ala Thr Gly Tyr Pro Leu Ala Glu Val Ser Trp Pro Asn Val Ser Val
145                 150                 155                 160

Pro Ala Asn Thr Ser His Ser Arg Thr Pro Glu Gly Leu Tyr Gln Val
                165                 170                 175

Thr Ser Val Leu Arg Leu Lys Pro Pro Pro Gly Arg Asn Phe Ser Cys
            180                 185                 190

Val Phe Trp Asn Thr His Val Arg Glu Leu Thr Leu Ala Ser Ile Asp
        195                 200                 205

Leu Gln Ser Gln Met Glu Pro Arg Thr His Pro Thr Trp Leu Leu His
    210                 215                 220

Ile Phe Ile Pro Phe Cys Ile Ile Ala Phe Ile Phe Ile Ala Thr Val
225                 230                 235                 240

Ile Ala Leu Arg Lys Gln Leu Cys Gln Lys Leu Tyr Ser Ser Lys Asp
                245                 250                 255

Thr Thr Lys Arg Pro Val Thr Thr Thr Lys Arg Glu Val Asn Ser Ala
            260                 265                 270

Ile

<210>  4
<211>  254
<212>  PRT
<213>  Homo sapiens

<400>  4

Leu Phe Thr Val Thr Val Pro Lys Glu Leu Tyr Ile Ile Glu His Gly
1               5                   10                  15

Ser Asn Val Thr Leu Glu Cys Asn Phe Asp Thr Gly Ser His Val Asn
            20                  25                  30

Leu Gly Ala Ile Thr Ala Ser Leu Gln Lys Val Glu Asn Asp Thr Ser
        35                  40                  45

Pro His Arg Glu Arg Ala Thr Leu Leu Glu Glu Gln Leu Pro Leu Gly
    50                  55                  60

Lys Ala Ser Phe His Ile Pro Gln Val Gln Val Arg Asp Glu Gly Gln
65                  70                  75                  80

Tyr Gln Cys Ile Ile Ile Tyr Gly Val Ala Trp Asp Tyr Lys Tyr Leu
                85                  90                  95

Thr Leu Lys Val Lys Ala Ser Tyr Arg Lys Ile Asn Thr His Ile Leu
            100                 105                 110

Lys Val Pro Glu Thr Asp Glu Val Glu Leu Thr Cys Gln Ala Thr Gly
        115                 120                 125

Tyr Pro Leu Ala Glu Val Ser Trp Pro Asn Val Ser Val Pro Ala Asn
    130                 135                 140

Thr Ser His Ser Arg Thr Pro Glu Gly Leu Tyr Gln Val Thr Ser Val
145                 150                 155                 160

Leu Arg Leu Lys Pro Pro Pro Gly Arg Asn Phe Ser Cys Val Phe Trp
                165                 170                 175

Asn Thr His Val Arg Glu Leu Thr Leu Ala Ser Ile Asp Leu Gln Ser
            180                 185                 190

Gln Met Glu Pro Arg Thr His Pro Thr Trp Leu Leu His Ile Phe Ile
        195                 200                 205

Pro Phe Cys Ile Ile Ala Phe Ile Phe Ile Ala Thr Val Ile Ala Leu
    210                 215                 220

Arg Lys Gln Leu Cys Gln Lys Leu Tyr Ser Ser Lys Asp Thr Thr Lys
225                 230                 235                 240

Arg Pro Val Thr Thr Thr Lys Arg Glu Val Asn Ser Ala Ile
                245                 250

<210>  5
<211>  273
<212>  PRT
<213>  Cynomolgus sp

<400>  5

Met Ile Phe Leu Leu Leu Met Leu Ser Leu Glu Leu Gln Leu His Gln
1               5                   10                  15

Ile Ala Ala Leu Phe Thr Val Thr Val Pro Lys Glu Leu Tyr Ile Ile
            20                  25                  30

Glu His Gly Ser Asn Val Thr Leu Glu Cys Asn Phe Asp Thr Gly Ser
        35                  40                  45

His Val Asn Leu Gly Ala Ile Thr Ala Ser Leu Gln Lys Val Glu Asn
    50                  55                  60

Asp Thr Ser Pro His Arg Glu Arg Ala Thr Leu Leu Glu Glu Gln Leu
65                  70                  75                  80

Pro Leu Gly Lys Ala Ser Phe His Ile Pro Gln Val Gln Val Arg Asp
                85                  90                  95

Glu Gly Gln Tyr Gln Cys Ile Ile Ile Tyr Gly Val Ala Trp Asp Tyr
            100                 105                 110

Lys Tyr Leu Thr Leu Lys Val Lys Ala Ser Tyr Arg Lys Ile Asn Thr
        115                 120                 125

His Ile Leu Lys Val Pro Glu Thr Asp Glu Val Glu Leu Thr Cys Gln
    130                 135                 140

Ala Thr Gly Tyr Pro Leu Ala Glu Val Ser Trp Pro Asn Val Ser Val
145                 150                 155                 160

Pro Ala Asn Thr Ser His Ser Arg Thr Pro Glu Gly Leu Tyr Gln Val
                165                 170                 175

Thr Ser Val Leu Arg Leu Lys Pro Pro Pro Gly Arg Asn Phe Ser Cys
            180                 185                 190

Val Phe Trp Asn Thr His Val Arg Glu Leu Thr Leu Ala Ser Ile Asp
        195                 200                 205

Leu Gln Ser Gln Met Glu Pro Arg Thr His Pro Thr Trp Leu Leu His
    210                 215                 220

Ile Phe Ile Pro Ser Cys Ile Ile Ala Phe Ile Phe Ile Ala Thr Val
225                 230                 235                 240

Ile Ala Leu Arg Lys Gln Leu Cys Gln Lys Leu Tyr Ser Ser Lys Asp
                245                 250                 255

Ala Thr Lys Arg Pro Val Thr Thr Thr Lys Arg Glu Val Asn Ser Ala
            260                 265                 270

Ile

<210>  6
<211>  254
<212>  PRT
<213>  Cynomolgus sp.

<400>  6

Leu Phe Thr Val Thr Val Pro Lys Glu Leu Tyr Ile Ile Glu His Gly
1               5                   10                  15

Ser Asn Val Thr Leu Glu Cys Asn Phe Asp Thr Gly Ser His Val Asn
            20                  25                  30

Leu Gly Ala Ile Thr Ala Ser Leu Gln Lys Val Glu Asn Asp Thr Ser
        35                  40                  45

Pro His Arg Glu Arg Ala Thr Leu Leu Glu Glu Gln Leu Pro Leu Gly
    50                  55                  60

Lys Ala Ser Phe His Ile Pro Gln Val Gln Val Arg Asp Glu Gly Gln
65                  70                  75                  80

Tyr Gln Cys Ile Ile Ile Tyr Gly Val Ala Trp Asp Tyr Lys Tyr Leu
                85                  90                  95

Thr Leu Lys Val Lys Ala Ser Tyr Arg Lys Ile Asn Thr His Ile Leu
            100                 105                 110

Lys Val Pro Glu Thr Asp Glu Val Glu Leu Thr Cys Gln Ala Thr Gly
        115                 120                 125

Tyr Pro Leu Ala Glu Val Ser Trp Pro Asn Val Ser Val Pro Ala Asn
    130                 135                 140

Thr Ser His Ser Arg Thr Pro Glu Gly Leu Tyr Gln Val Thr Ser Val
145                 150                 155                 160

Leu Arg Leu Lys Pro Pro Pro Gly Arg Asn Phe Ser Cys Val Phe Trp
                165                 170                 175

Asn Thr His Val Arg Glu Leu Thr Leu Ala Ser Ile Asp Leu Gln Ser
            180                 185                 190

Gln Met Glu Pro Arg Thr His Pro Thr Trp Leu Leu His Ile Phe Ile
        195                 200                 205

Pro Ser Cys Ile Ile Ala Phe Ile Phe Ile Ala Thr Val Ile Ala Leu
    210                 215                 220

Arg Lys Gln Leu Cys Gln Lys Leu Tyr Ser Ser Lys Asp Ala Thr Lys
225                 230                 235                 240

Arg Pro Val Thr Thr Thr Lys Arg Glu Val Asn Ser Ala Ile
                245                 250

<210>  7
<211>  290
<212>  PRT
<213>  Mus musculus

<400>  7

Met Arg Ile Phe Ala Gly Ile Ile Phe Thr Ala Cys Cys His Leu Leu
1               5                   10                  15

Arg Ala Phe Thr Ile Thr Ala Pro Lys Asp Leu Tyr Val Val Glu Tyr
            20                  25                  30

Gly Ser Asn Val Thr Met Glu Cys Arg Phe Pro Val Glu Arg Glu Leu
        35                  40                  45

Asp Leu Leu Ala Leu Val Val Tyr Trp Glu Lys Glu Asp Glu Gln Val
    50                  55                  60

Ile Gln Phe Val Ala Gly Glu Glu Asp Leu Lys Pro Gln His Ser Asn
65                  70                  75                  80

Phe Arg Gly Arg Ala Ser Leu Pro Lys Asp Gln Leu Leu Lys Gly Asn
                85                  90                  95

Ala Ala Leu Gln Ile Thr Asp Val Lys Leu Gln Asp Ala Gly Val Tyr
            100                 105                 110

Cys Cys Ile Ile Ser Tyr Gly Gly Ala Asp Tyr Lys Arg Ile Thr Leu
        115                 120                 125

Lys Val Asn Ala Pro Tyr Arg Lys Ile Asn Gln Arg Ile Ser Val Asp
    130                 135                 140

Pro Ala Thr Ser Glu His Glu Leu Ile Cys Gln Ala Glu Gly Tyr Pro
145                 150                 155                 160

Glu Ala Glu Val Ile Trp Thr Asn Ser Asp His Gln Pro Val Ser Gly
                165                 170                 175

Lys Arg Ser Val Thr Thr Ser Arg Thr Glu Gly Met Leu Leu Asn Val
            180                 185                 190

Thr Ser Ser Leu Arg Val Asn Ala Thr Ala Asn Asp Val Phe Tyr Cys
        195                 200                 205

Thr Phe Trp Arg Ser Gln Pro Gly Gln Asn His Thr Ala Glu Leu Ile
    210                 215                 220

Ile Pro Glu Leu Pro Ala Thr His Pro Pro Gln Asn Arg Thr His Trp
225                 230                 235                 240

Val Leu Leu Gly Ser Ile Leu Leu Phe Leu Ile Val Val Ser Thr Val
                245                 250                 255

Leu Leu Phe Leu Arg Lys Gln Val Arg Met Leu Asp Val Glu Lys Cys
            260                 265                 270

Gly Val Glu Asp Thr Ser Ser Lys Asn Arg Asn Asp Thr Gln Phe Glu
        275                 280                 285

Glu Thr
    290

<210> 8
<211> 271
<212> PRT
<213> Mus musculus

<400> 8

Thr Ile Thr Ala Pro Lys Asp Leu Tyr Val Val Glu Tyr Gly Ser Asn
1               5                   10                  15

Val Thr Met Glu Cys Arg Phe Pro Val Glu Arg Glu Leu Asp Leu Leu
            20                  25                  30

Ala Leu Val Val Tyr Trp Glu Lys Glu Asp Glu Gln Val Ile Gln Phe
        35                  40                  45

Val Ala Gly Glu Glu Asp Leu Lys Pro Gln His Ser Asn Phe Arg Gly
    50                  55                  60

Arg Ala Ser Leu Pro Lys Asp Gln Leu Leu Lys Gly Asn Ala Ala Leu
65                  70                  75                  80

Gln Ile Thr Asp Val Lys Leu Gln Asp Ala Gly Val Tyr Cys Cys Ile
                85                  90                  95

Ile Ser Tyr Gly Gly Ala Asp Tyr Lys Arg Ile Thr Leu Lys Val Asn
            100                 105                 110

Ala Pro Tyr Arg Lys Ile Asn Gln Arg Ile Ser Val Asp Pro Ala Thr
        115                 120                 125

Ser Glu His Glu Leu Ile Cys Gln Ala Glu Gly Tyr Pro Glu Ala Glu
    130                 135                 140

Val Ile Trp Thr Asn Ser Asp His Gln Pro Val Ser Gly Lys Arg Ser
145                 150                 155                 160

Val Thr Thr Ser Arg Thr Glu Gly Met Leu Leu Asn Val Thr Ser Ser
                165                 170                 175

Leu Arg Val Asn Ala Thr Ala Asn Asp Val Phe Tyr Cys Thr Phe Trp
            180                 185                 190

Arg Ser Gln Pro Gly Gln Asn His Thr Ala Glu Leu Ile Ile Pro Glu
        195                 200                 205

Leu Pro Ala Thr His Pro Pro Gln Asn Arg Thr His Trp Val Leu Leu
    210                 215                 220

Gly Ser Ile Leu Leu Phe Leu Ile Val Val Ser Thr Val Leu Leu Phe
225                 230                 235                 240

Leu Arg Lys Gln Val Arg Met Leu Asp Val Glu Lys Cys Gly Val Glu
                245                 250                 255

Asp Thr Ser Ser Lys Asn Arg Asn Asp Thr Gln Phe Glu Glu Thr
            260                 265                 270

<210>  9
<211>  290
<212>  PRT
<213>  Homo sapiens

<400>  9

Met Arg Ile Phe Ala Val Phe Ile Phe Met Thr Tyr Trp His Leu Leu
1               5                   10                  15

Asn Ala Phe Thr Val Thr Val Pro Lys Asp Leu Tyr Val Val Glu Tyr
            20                  25                  30

Gly Ser Asn Met Thr Ile Glu Cys Lys Phe Pro Val Glu Lys Gln Leu
        35                  40                  45

Asp Leu Ala Ala Leu Ile Val Tyr Trp Glu Met Glu Asp Lys Asn Ile
    50                  55                  60

Ile Gln Phe Val His Gly Glu Glu Asp Leu Lys Val Gln His Ser Ser
65                  70                  75                  80

Tyr Arg Gln Arg Ala Arg Leu Leu Lys Asp Gln Leu Ser Leu Gly Asn
                85                  90                  95

Ala Ala Leu Gln Ile Thr Asp Val Lys Leu Gln Asp Ala Gly Val Tyr
            100                 105                 110

Arg Cys Met Ile Ser Tyr Gly Gly Ala Asp Tyr Lys Arg Ile Thr Val
        115                 120                 125

Lys Val Asn Ala Pro Tyr Asn Lys Ile Asn Gln Arg Ile Leu Val Val
    130                 135                 140

Asp Pro Val Thr Ser Glu His Glu Leu Thr Cys Gln Ala Glu Gly Tyr
145                 150                 155                 160

Pro Lys Ala Glu Val Ile Trp Thr Ser Ser Asp His Gln Val Leu Ser
                165                 170                 175

Gly Lys Thr Thr Thr Thr Asn Ser Lys Arg Glu Glu Lys Leu Phe Asn
            180                 185                 190

Val Thr Ser Thr Leu Arg Ile Asn Thr Thr Thr Asn Glu Ile Phe Tyr
        195                 200                 205

Cys Thr Phe Arg Arg Leu Asp Pro Glu Glu Asn His Thr Ala Glu Leu
    210                 215                 220

Val Ile Pro Glu Leu Pro Leu Ala His Pro Pro Asn Glu Arg Thr His
225                 230                 235                 240

Leu Val Ile Leu Gly Ala Ile Leu Leu Cys Leu Gly Val Ala Leu Thr
                245                 250                 255

Phe Ile Phe Arg Leu Arg Lys Gly Arg Met Met Asp Val Lys Lys Cys
            260                 265                 270

Gly Ile Gln Asp Thr Asn Ser Lys Lys Gln Ser Asp Thr His Leu Glu
        275                 280                 285

Glu Thr
    290

<210>  10
<211>  272
<212>  PRT
<213>  Homo sapiens

<400>  10

Phe Thr Val Thr Val Pro Lys Asp Leu Tyr Val Val Glu Tyr Gly Ser
1               5                   10                  15

Asn Met Thr Ile Glu Cys Lys Phe Pro Val Glu Lys Gln Leu Asp Leu
            20                  25                  30

Ala Ala Leu Ile Val Tyr Trp Glu Met Glu Asp Lys Asn Ile Ile Gln
        35                  40                  45

Phe Val His Gly Glu Glu Asp Leu Lys Val Gln His Ser Ser Tyr Arg
    50                  55                  60

Gln Arg Ala Arg Leu Leu Lys Asp Gln Leu Ser Leu Gly Asn Ala Ala
65                  70                  75                  80

Leu Gln Ile Thr Asp Val Lys Leu Gln Asp Ala Gly Val Tyr Arg Cys
                85                  90                  95

Met Ile Ser Tyr Gly Gly Ala Asp Tyr Lys Arg Ile Thr Val Lys Val
            100                 105                 110

Asn Ala Pro Tyr Asn Lys Ile Asn Gln Arg Ile Leu Val Val Asp Pro
        115                 120                 125

Val Thr Ser Glu His Glu Leu Thr Cys Gln Ala Glu Gly Tyr Pro Lys
    130                 135                 140

Ala Glu Val Ile Trp Thr Ser Ser Asp His Gln Val Leu Ser Gly Lys
145                 150                 155                 160

Thr Thr Thr Thr Asn Ser Lys Arg Glu Glu Lys Leu Phe Asn Val Thr
                165                 170                 175

Ser Thr Leu Arg Ile Asn Thr Thr Thr Asn Glu Ile Phe Tyr Cys Thr
            180                 185                 190

Phe Arg Arg Leu Asp Pro Glu Glu Asn His Thr Ala Glu Leu Val Ile
        195                 200                 205

Pro Glu Leu Pro Leu Ala His Pro Pro Asn Glu Arg Thr His Leu Val
    210                 215                 220

Ile Leu Gly Ala Ile Leu Leu Cys Leu Gly Val Ala Leu Thr Phe Ile
225                 230                 235                 240

Phe Arg Leu Arg Lys Gly Arg Met Met Asp Val Lys Lys Cys Gly Ile
                245                 250                 255

Gln Asp Thr Asn Ser Lys Lys Gln Ser Asp Thr His Leu Glu Glu Thr
            260                 265                 270

<210>  11
<211>  306
<212>  PRT
<213>  Mus musculus

<400>  11

Met Ala Cys Asn Cys Gln Leu Met Gln Asp Thr Pro Leu Leu Lys Phe
1               5                   10                  15

Pro Cys Pro Arg Leu Ile Leu Leu Phe Val Leu Leu Ile Arg Leu Ser
            20                  25                  30

Gln Val Ser Ser Asp Val Asp Glu Gln Leu Ser Lys Ser Val Lys Asp
        35                  40                  45

Lys Val Leu Leu Pro Cys Arg Tyr Asn Ser Pro His Glu Asp Glu Ser
    50                  55                  60

Glu Asp Arg Ile Tyr Trp Gln Lys His Asp Lys Val Val Leu Ser Val
65                  70                  75                  80

Ile Ala Gly Lys Leu Lys Val Trp Pro Glu Tyr Lys Asn Arg Thr Leu
                85                  90                  95

Tyr Asp Asn Thr Thr Tyr Ser Leu Ile Ile Leu Gly Leu Val Leu Ser
            100                 105                 110

Asp Arg Gly Thr Tyr Ser Cys Val Val Gln Lys Lys Glu Arg Gly Thr
        115                 120                 125

Tyr Glu Val Lys His Leu Ala Leu Val Lys Leu Ser Ile Lys Ala Asp
    130                 135                 140

Phe Ser Thr Pro Asn Ile Thr Glu Ser Gly Asn Pro Ser Ala Asp Thr
145                 150                 155                 160

Lys Arg Ile Thr Cys Phe Ala Ser Gly Gly Phe Pro Lys Pro Arg Phe
                165                 170                 175

Ser Trp Leu Glu Asn Gly Arg Glu Leu Pro Gly Ile Asn Thr Thr Ile
            180                 185                 190

Ser Gln Asp Pro Glu Ser Glu Leu Tyr Thr Ile Ser Ser Gln Leu Asp
        195                 200                 205

Phe Asn Thr Thr Arg Asn His Thr Ile Lys Cys Leu Ile Lys Tyr Gly
    210                 215                 220

Asp Ala His Val Ser Glu Asp Phe Thr Trp Glu Lys Pro Pro Glu Asp
225                 230                 235                 240

Pro Pro Asp Ser Lys Asn Thr Leu Val Leu Phe Gly Ala Gly Phe Gly
                245                 250                 255

Ala Val Ile Thr Val Val Val Ile Val Val Ile Ile Lys Cys Phe Cys
            260                 265                 270

Lys His Arg Ser Cys Phe Arg Arg Asn Glu Ala Ser Arg Glu Thr Asn
        275                 280                 285

Asn Ser Leu Thr Phe Gly Pro Glu Glu Ala Leu Ala Glu Gln Thr Val
    290                 295                 300

Phe Leu
305

<210>  12
<211>  269
<212>  PRT
<213>  Mus musculus

<400>  12

Val Asp Glu Gln Leu Ser Lys Ser Val Lys Asp Lys Val Leu Leu Pro
1               5                   10                  15

Cys Arg Tyr Asn Ser Pro His Glu Asp Glu Ser Glu Asp Arg Ile Tyr
            20                  25                  30

Trp Gln Lys His Asp Lys Val Val Leu Ser Val Ile Ala Gly Lys Leu
        35                  40                  45

Lys Val Trp Pro Glu Tyr Lys Asn Arg Thr Leu Tyr Asp Asn Thr Thr
    50                  55                  60

Tyr Ser Leu Ile Ile Leu Gly Leu Val Leu Ser Asp Arg Gly Thr Tyr
65                  70                  75                  80

Ser Cys Val Val Gln Lys Lys Glu Arg Gly Thr Tyr Glu Val Lys His
                85                  90                  95

Leu Ala Leu Val Lys Leu Ser Ile Lys Ala Asp Phe Ser Thr Pro Asn
            100                 105                 110

Ile Thr Glu Ser Gly Asn Pro Ser Ala Asp Thr Lys Arg Ile Thr Cys
        115                 120                 125

Phe Ala Ser Gly Gly Phe Pro Lys Pro Arg Phe Ser Trp Leu Glu Asn
    130                 135                 140

Gly Arg Glu Leu Pro Gly Ile Asn Thr Thr Ile Ser Gln Asp Pro Glu
145                 150                 155                 160

Ser Glu Leu Tyr Thr Ile Ser Ser Gln Leu Asp Phe Asn Thr Thr Arg
                165                 170                 175

Asn His Thr Ile Lys Cys Leu Ile Lys Tyr Gly Asp Ala His Val Ser
            180                 185                 190

Glu Asp Phe Thr Trp Glu Lys Pro Pro Glu Asp Pro Pro Asp Ser Lys
        195                 200                 205

Asn Thr Leu Val Leu Phe Gly Ala Gly Phe Gly Ala Val Ile Thr Val
    210                 215                 220

Val Val Ile Val Val Ile Ile Lys Cys Phe Cys Lys His Arg Ser Cys
225                 230                 235                 240

Phe Arg Arg Asn Glu Ala Ser Arg Glu Thr Asn Asn Ser Leu Thr Phe
                245                 250                 255

Gly Pro Glu Glu Ala Leu Ala Glu Gln Thr Val Phe Leu
            260                 265

<210> 13
<211> 288
<212> PRT
<213> Homo sapiens

<400> 13

Met Gly His Thr Arg Arg Gln Gly Thr Ser Pro Ser Lys Cys Pro Tyr
1               5                   10                  15

Leu Asn Phe Phe Gln Leu Leu Val Leu Ala Gly Leu Ser His Phe Cys
            20                  25                  30

Ser Gly Val Ile His Val Thr Lys Glu Val Lys Glu Val Ala Thr Leu
        35                  40                  45

Ser Cys Gly His Asn Val Ser Val Glu Glu Leu Ala Gln Thr Arg Ile
    50                  55                  60

Tyr Trp Gln Lys Glu Lys Lys Met Val Leu Thr Met Met Ser Gly Asp
65                  70                  75                  80

Met Asn Ile Trp Pro Glu Tyr Lys Asn Arg Thr Ile Phe Asp Ile Thr
                85                  90                  95

Asn Asn Leu Ser Ile Val Ile Leu Ala Leu Arg Pro Ser Asp Glu Gly
            100                 105                 110

Thr Tyr Glu Cys Val Val Leu Lys Tyr Glu Lys Asp Ala Phe Lys Arg
        115                 120                 125

Glu His Leu Ala Glu Val Thr Leu Ser Val Lys Ala Asp Phe Pro Thr
    130                 135                 140

Pro Ser Ile Ser Asp Phe Glu Ile Pro Thr Ser Asn Ile Arg Arg Ile
145                 150                 155                 160

Ile Cys Ser Thr Ser Gly Gly Phe Pro Glu Pro His Leu Ser Trp Leu
                165                 170                 175

Glu Asn Gly Glu Glu Leu Asn Ala Ile Asn Thr Thr Val Ser Gln Asp
            180                 185                 190

Pro Glu Thr Glu Leu Tyr Ala Val Ser Ser Lys Leu Asp Phe Asn Met
        195                 200                 205

Thr Thr Asn His Ser Phe Met Cys Leu Ile Lys Tyr Gly His Leu Arg
    210                 215                 220

Val Asn Gln Thr Phe Asn Trp Asn Thr Thr Lys Gln Glu His Phe Pro
225                 230                 235                 240

Asp Asn Leu Leu Pro Ser Trp Ala Ile Thr Leu Ile Ser Val Asn Gly
                245                 250                 255

Ile Phe Val Ile Cys Cys Leu Thr Tyr Cys Phe Ala Pro Arg Cys Arg
            260                 265                 270

Glu Arg Arg Arg Asn Glu Arg Leu Arg Arg Glu Ser Val Arg Pro Val
        275                 280                 285

<210> 14
<211> 254
<212> PRT
<213> Homo sapiens

<400> 14

Val Ile His Val Thr Lys Glu Val Lys Glu Val Ala Thr Leu Ser Cys
1               5                   10                  15

Gly His Asn Val Ser Val Glu Glu Leu Ala Gln Thr Arg Ile Tyr Trp
            20                  25                  30

Gln Lys Glu Lys Lys Met Val Leu Thr Met Met Ser Gly Asp Met Asn
        35                  40                  45

Ile Trp Pro Glu Tyr Lys Asn Arg Thr Ile Phe Asp Ile Thr Asn Asn
    50                  55                  60

Leu Ser Ile Val Ile Leu Ala Leu Arg Pro Ser Asp Glu Gly Thr Tyr
65                  70                  75                  80

Glu Cys Val Val Leu Lys Tyr Glu Lys Asp Ala Phe Lys Arg Glu His
                85                  90                  95

Leu Ala Glu Val Thr Leu Ser Val Lys Ala Asp Phe Pro Thr Pro Ser
            100                 105                 110

Ile Ser Asp Phe Glu Ile Pro Thr Ser Asn Ile Arg Arg Ile Ile Cys
        115                 120                 125

Ser Thr Ser Gly Gly Phe Pro Glu Pro His Leu Ser Trp Leu Glu Asn
    130                 135                 140

Gly Glu Glu Leu Asn Ala Ile Asn Thr Thr Val Ser Gln Asp Pro Glu
145                 150                 155                 160

Thr Glu Leu Tyr Ala Val Ser Ser Lys Leu Asp Phe Asn Met Thr Thr
                165                 170                 175

Asn His Ser Phe Met Cys Leu Ile Lys Tyr Gly His Leu Arg Val Asn
            180                 185                 190

Gln Thr Phe Asn Trp Asn Thr Thr Lys Gln Glu His Phe Pro Asp Asn
        195                 200                 205

Leu Leu Pro Ser Trp Ala Ile Thr Leu Ile Ser Val Asn Gly Ile Phe
    210                 215                 220

Val Ile Cys Cys Leu Thr Tyr Cys Phe Ala Pro Arg Cys Arg Glu Arg
225                 230                 235                 240

Arg Arg Asn Glu Arg Leu Arg Arg Glu Ser Val Arg Pro Val
                245                 250

<210>  15
<211>  288
<212>  PRT
<213>  Homo sapiens

<400>  15

Met Gln Ile Pro Gln Ala Pro Trp Pro Val Val Trp Ala Val Leu Gln
1               5                   10                  15

Leu Gly Trp Arg Pro Gly Trp Phe Leu Asp Ser Pro Asp Arg Pro Trp
            20                  25                  30

Asn Pro Pro Thr Phe Phe Pro Ala Leu Leu Val Val Thr Glu Gly Asp
        35                  40                  45

Asn Ala Thr Phe Thr Cys Ser Phe Ser Asn Thr Ser Glu Ser Phe Val
    50                  55                  60

Leu Asn Trp Tyr Arg Met Ser Pro Ser Asn Gln Thr Asp Lys Leu Ala
65                  70                  75                  80

Ala Phe Pro Glu Asp Arg Ser Gln Pro Gly Gln Asp Cys Arg Phe Arg
                85                  90                  95

Val Thr Gln Leu Pro Asn Gly Arg Asp Phe His Met Ser Val Val Arg
            100                 105                 110

Ala Arg Arg Asn Asp Ser Gly Thr Tyr Leu Cys Gly Ala Ile Ser Leu
        115                 120                 125

Ala Pro Lys Ala Gln Ile Lys Glu Ser Leu Arg Ala Glu Leu Arg Val
    130                 135                 140

Thr Glu Arg Arg Ala Glu Val Pro Thr Ala His Pro Ser Pro Ser Pro
145                 150                 155                 160

Arg Pro Ala Gly Gln Phe Gln Thr Leu Val Val Gly Val Val Gly Gly
                165                 170                 175

Leu Leu Gly Ser Leu Val Leu Leu Val Trp Val Leu Ala Val Ile Cys
            180                 185                 190

Ser Arg Ala Ala Arg Gly Thr Ile Gly Ala Arg Arg Thr Gly Gln Pro
        195                 200                 205

Leu Lys Glu Asp Pro Ser Ala Val Pro Val Phe Ser Val Asp Tyr Gly
    210                 215                 220

Glu Leu Asp Phe Gln Trp Arg Glu Lys Thr Pro Glu Pro Pro Val Pro
225                 230                 235                 240

Cys Val Pro Glu Gln Thr Glu Tyr Ala Thr Ile Val Phe Pro Ser Gly
                245                 250                 255

Met Gly Thr Ser Ser Pro Ala Arg Arg Gly Ser Ala Asp Gly Pro Arg
            260                 265                 270

Ser Ala Gln Pro Leu Arg Pro Glu Asp Gly His Cys Ser Trp Pro Leu
        275                 280                 285

<210> 16
<211> 288
<212> PRT
<213> Cynomolgus sp.

<400> 16

Met Gln Ile Pro Gln Ala Pro Trp Pro Val Val Trp Ala Val Leu Gln
1               5                   10                  15

Leu Gly Trp Arg Pro Gly Trp Phe Leu Glu Ser Pro Asp Arg Pro Trp
            20                  25                  30

Asn Ala Pro Thr Phe Ser Pro Ala Leu Leu Leu Val Thr Glu Gly Asp
        35                  40                  45

Asn Ala Thr Phe Thr Cys Ser Phe Ser Asn Ala Ser Glu Ser Phe Val
    50                  55                  60

Leu Asn Trp Tyr Arg Met Ser Pro Ser Asn Gln Thr Asp Lys Leu Ala
65                  70                  75                  80

Ala Phe Pro Glu Asp Arg Ser Gln Pro Gly Gln Asp Cys Arg Phe Arg
                85                  90                  95

Val Thr Arg Leu Pro Asn Gly Arg Asp Phe His Met Ser Val Val Arg
            100                 105                 110

Ala Arg Arg Asn Asp Ser Gly Thr Tyr Leu Cys Gly Ala Ile Ser Leu
        115                 120                 125

Ala Pro Lys Ala Gln Ile Lys Glu Ser Leu Arg Ala Glu Leu Arg Val
    130                 135                 140

Thr Glu Arg Arg Ala Glu Val Pro Thr Ala His Pro Ser Pro Ser Pro
145                 150                 155                 160

Arg Pro Ala Gly Gln Phe Gln Thr Leu Val Val Gly Val Val Gly Gly
                165                 170                 175

Leu Leu Gly Ser Leu Val Leu Leu Val Trp Val Leu Ala Val Ile Cys
            180                 185                 190

Ser Arg Ala Ala Arg Gly Thr Ile Gly Ala Arg Arg Thr Gly Gln Pro
        195                 200                 205

Leu Lys Glu Asp Pro Ser Ala Val Pro Val Phe Ser Val Asp Tyr Gly
    210                 215                 220

Glu Leu Asp Phe Gln Trp Arg Glu Lys Thr Pro Glu Pro Pro Val Pro
225                 230                 235                 240

Cys Val Pro Glu Gln Thr Glu Tyr Ala Thr Ile Val Phe Pro Ser Gly
                245                 250                 255

Met Gly Thr Ser Ser Pro Ala Arg Arg Gly Ser Ala Asp Gly Pro Arg
            260                 265                 270

Ser Ala Gln Pro Leu Arg Pro Glu Asp Gly His Cys Ser Trp Pro Leu
        275                 280                 285

<210>  17

<211>  663
<212>  DNA
<213>  Homo sapiens

<400>  17
atgatctttc ttctcttgat gctgtctttg gaattgcaac ttcaccaaat cgcggccctc      60

tttactgtga ccgtgccaaa agaactgtat atcattgagc acgggtccaa tgtgaccctc     120

gaatgtaact ttgacaccgg cagccacgtt aacctggggg ccatcactgc cagcttgcaa     180

aaagttgaaa acgacacttc acctcaccgg gagagggcaa ccctcttgga ggagcaactg     240

ccattgggga aggcctcctt tcatatccct caggtgcagg ttcgggatga gggacagtac     300

cagtgcatta ttatctacgg cgtggcttgg gattacaagt atctgaccct gaaggtgaaa     360

gcgtcctatc ggaaaattaa cactcacatt cttaaggtgc cagagacgga cgaggtggaa     420

ctgacatgcc aagccaccgg ctacccgttg gcagaggtca gctggcccaa cgtgagcgta     480

cctgctaaca cttctcattc taggacaccc gagggcctct accaggttac atccgtgctc     540

cgcctcaaac cgcccccagg ccggaatttt agttgcgtgt tttggaatac ccacgtgcga     600

gagctgactc ttgcatctat tgatctgcag tcccagatgg agccacggac tcatccaact     660

tgg                                                                   663

<210>  18
<211>  261
<212>  PRT
<213>  Homo sapiens

<400>  18

Met Ile Phe Leu Leu Leu Met Leu Ser Leu Glu Leu Gln Leu His Gln
1               5                   10                  15

Ile Ala Ala Leu Phe Thr Val Thr Val Pro Lys Glu Leu Tyr Ile Ile
            20                  25                  30

Glu His Gly Ser Asn Val Thr Leu Met Ile Phe Leu Leu Leu Met Leu
        35                  40                  45

Ser Leu Glu Leu Gln Leu His Gln Ile Ala Ala Leu Phe Thr Val Thr
    50                  55                  60

Val Pro Lys Glu Leu Tyr Ile Ile Glu His Gly Ser Asn Val Thr Leu
65                  70                  75                  80

Glu Cys Asn Phe Asp Thr Gly Ser His Val Asn Leu Gly Ala Ile Thr
                85                  90                  95

Ala Ser Leu Gln Lys Val Glu Asn Asp Thr Ser Pro His Arg Glu Arg
            100                 105                 110

Ala Thr Leu Leu Glu Glu Gln Leu Pro Leu Gly Lys Ala Ser Phe His
        115                 120                 125

Ile Pro Gln Val Gln Val Arg Asp Glu Gly Gln Tyr Gln Cys Ile Ile
    130                 135                 140

Ile Tyr Gly Val Ala Trp Asp Tyr Lys Tyr Leu Thr Leu Lys Val Lys
145                 150                 155                 160

Ala Ser Tyr Arg Lys Ile Asn Thr His Ile Leu Lys Val Pro Glu Thr
                165                 170                 175

Asp Glu Val Glu Leu Thr Cys Gln Ala Thr Gly Tyr Pro Leu Ala Glu
            180                 185                 190

Val Ser Trp Pro Asn Val Ser Val Pro Ala Asn Thr Ser His Ser Arg
        195                 200                 205

Thr Pro Glu Gly Leu Tyr Gln Val Thr Ser Val Leu Arg Leu Lys Pro
    210                 215                 220

Pro Pro Gly Arg Asn Phe Ser Cys Val Phe Trp Asn Thr His Val Arg
225                 230                 235                 240

Glu Leu Thr Leu Ala Ser Ile Asp Leu Gln Ser Gln Met Glu Pro Arg
                245                 250                 255

Thr His Pro Thr Trp
            260

<210>  19
<211>  202
<212>  PRT
<213>  Homo sapiens

<400>  19

Leu Phe Thr Val Thr Val Pro Lys Glu Leu Tyr Ile Ile Glu His Gly
1               5                   10                  15

Ser Asn Val Thr Leu Glu Cys Asn Phe Asp Thr Gly Ser His Val Asn
            20                  25                  30

Leu Gly Ala Ile Thr Ala Ser Leu Gln Lys Val Glu Asn Asp Thr Ser
        35                  40                  45

Pro His Arg Glu Arg Ala Thr Leu Leu Glu Glu Gln Leu Pro Leu Gly
    50                  55                  60

Lys Ala Ser Phe His Ile Pro Gln Val Gln Val Arg Asp Glu Gly Gln
65                  70                  75                  80

Tyr Gln Cys Ile Ile Ile Tyr Gly Val Ala Trp Asp Tyr Lys Tyr Leu
                85                  90                  95

Thr Leu Lys Val Lys Ala Ser Tyr Arg Lys Ile Asn Thr His Ile Leu
            100                 105                 110

Lys Val Pro Glu Thr Asp Glu Val Glu Leu Thr Cys Gln Ala Thr Gly
        115                 120                 125

Tyr Pro Leu Ala Glu Val Ser Trp Pro Asn Val Ser Val Pro Ala Asn
    130                 135                 140

Thr Ser His Ser Arg Thr Pro Glu Gly Leu Tyr Gln Val Thr Ser Val
145                 150                 155                 160

Leu Arg Leu Lys Pro Pro Pro Gly Arg Asn Phe Ser Cys Val Phe Trp
                165                 170                 175

Asn Thr His Val Arg Glu Leu Thr Leu Ala Ser Ile Asp Leu Gln Ser
            180                 185                 190

Gln Met Glu Pro Arg Thr His Pro Thr Trp
        195                 200

<210>  20
<211>  294
<212>  DNA
<213>  Homo sapiens

<400>  20
tttactgtga ccgtgccaaa agaactgtat atcattgagc acgggtccaa tgtgaccctc      60

gaatgtaact ttgacaccgg cagccacgtt aacctggggg ccatcactgc cagcttgcaa     120

aaagttgaaa acgacacttc acctcaccgg gagagggcaa ccctcttgga ggagcaactg     180

ccattgggga aggcctcctt tcatatccct caggtgcagg ttcgggatga gggacagtac     240

cagtgcatta ttatctacgg cgtggcttgg gattacaagt atctgaccct gaag           294

<210>  21
<211>  98
<212>  PRT
<213>  Homo sapiens

<400>  21

Phe Thr Val Thr Val Pro Lys Glu Leu Tyr Ile Ile Glu His Gly Ser

1               5                   10                  15

Asn Val Thr Leu Glu Cys Asn Phe Asp Thr Gly Ser His Val Asn Leu
            20                  25                  30

Gly Ala Ile Thr Ala Ser Leu Gln Lys Val Glu Asn Asp Thr Ser Pro
        35                  40                  45

His Arg Glu Arg Ala Thr Leu Leu Glu Glu Gln Leu Pro Leu Gly Lys
    50                  55                  60

Ala Ser Phe His Ile Pro Gln Val Gln Val Arg Asp Glu Gly Gln Tyr
65                  70                  75                  80

Gln Cys Ile Ile Ile Tyr Gly Val Ala Trp Asp Tyr Lys Tyr Leu Thr
                85                  90                  95

Leu Lys

<210>  22
<211>  663
<212>  DNA
<213>  Cynomolgus sp.

<400>  22
atgatcttcc tcctgctaat gttgagcctg gaattgcagc ttcaccagat agcagcttta      60

ttcacagtga cagtccctaa ggaactgtac ataatagagc atggcagcaa tgtgaccctg     120

gaatgcaact ttgacactgg aagtcatgtg aaccttggag caataacagc cagtttgcaa     180

aaggtggaaa atgatacatc cccacaccgt gaaagagcca ctttgctgga ggagcagctg     240

cccctaggga aggcctcgtt ccacatacct caagtccaag tgagggacga aggacagtac     300

caatgcataa tcatctatgg ggtcgcctgg gactacaagt acctgactct gaaagtcaaa     360

gcttcctaca ggaaaataaa cactcacatc ctaaaggttc cagaaacaga tgaggtagag     420

ctcacctgcc aggctacagg ttatcctctg gcagaagtat cctggccaaa cgtcagcgtt     480

cctgccaaca ccagccactc caggacccct gaaggcctct accaggtcac cagtgttctg     540

cgcctaaagc caccccctgg cagaaacttc agctgtgtgt tctggaatac tcacgtgagg     600

gaacttactt tggccagcat tgaccttcaa agtcagatgg aacccaggac ccatccaact     660

tgg                                                                   663

<210>  23
<211>  221
<212>  PRT
<213>  Cynomolgus sp.

<400>  23

Met Ile Phe Leu Leu Leu Met Leu Ser Leu Glu Leu Gln Leu His Gln
1               5                   10                  15

Ile Ala Ala Leu Phe Thr Val Thr Val Pro Lys Glu Leu Tyr Ile Ile
            20                  25                  30

Glu His Gly Ser Asn Val Thr Leu Glu Cys Asn Phe Asp Thr Gly Ser
        35                  40                  45

His Val Asn Leu Gly Ala Ile Thr Ala Ser Leu Gln Lys Val Glu Asn
    50                  55                  60

Asp Thr Ser Pro His Arg Glu Arg Ala Thr Leu Leu Glu Glu Gln Leu
65                  70                  75                  80

Pro Leu Gly Lys Ala Ser Phe His Ile Pro Gln Val Gln Val Arg Asp
                85                  90                  95

Glu Gly Gln Tyr Gln Cys Ile Ile Ile Tyr Gly Val Ala Trp Asp Tyr
            100                 105                 110

Lys Tyr Leu Thr Leu Lys Val Lys Ala Ser Tyr Arg Lys Ile Asn Thr
        115                 120                 125

His Ile Leu Lys Val Pro Glu Thr Asp Glu Val Glu Leu Thr Cys Gln
    130                 135                 140

Ala Thr Gly Tyr Pro Leu Ala Glu Val Ser Trp Pro Asn Val Ser Val
145                 150                 155                 160

Pro Ala Asn Thr Ser His Ser Arg Thr Pro Glu Gly Leu Tyr Gln Val
                165                 170                 175

Thr Ser Val Leu Arg Leu Lys Pro Pro Pro Gly Arg Asn Phe Ser Cys
            180                 185                 190

Val Phe Trp Asn Thr His Val Arg Glu Leu Thr Leu Ala Ser Ile Asp
        195                 200                 205

Leu Gln Ser Gln Met Glu Pro Arg Thr His Pro Thr Trp
    210                 215                 220

<210>  24
<211>  202
<212>  PRT
<213>  Cynomolgus sp.

<400>  24

Leu Phe Thr Val Thr Val Pro Lys Glu Leu Tyr Ile Ile Glu His Gly
1               5                   10                  15

Ser Asn Val Thr Leu Glu Cys Asn Phe Asp Thr Gly Ser His Val Asn
            20                  25                  30

Leu Gly Ala Ile Thr Ala Ser Leu Gln Lys Val Glu Asn Asp Thr Ser
        35                  40                  45

Pro His Arg Glu Arg Ala Thr Leu Leu Glu Glu Gln Leu Pro Leu Gly
    50                  55                  60

Lys Ala Ser Phe His Ile Pro Gln Val Gln Val Arg Asp Glu Gly Gln
65                  70                  75                  80

Tyr Gln Cys Ile Ile Ile Tyr Gly Val Ala Trp Asp Tyr Lys Tyr Leu
                85                  90                  95

Thr Leu Lys Val Lys Ala Ser Tyr Arg Lys Ile Asn Thr His Ile Leu
            100                 105                 110

Lys Val Pro Glu Thr Asp Glu Val Glu Leu Thr Cys Gln Ala Thr Gly
        115                 120                 125

Tyr Pro Leu Ala Glu Val Ser Trp Pro Asn Val Ser Val Pro Ala Asn
    130                 135                 140

Thr Ser His Ser Arg Thr Pro Glu Gly Leu Tyr Gln Val Thr Ser Val
145                 150                 155                 160

Leu Arg Leu Lys Pro Pro Pro Gly Arg Asn Phe Ser Cys Val Phe Trp
                165                 170                 175

Asn Thr His Val Arg Glu Leu Thr Leu Ala Ser Ile Asp Leu Gln Ser
            180                 185                 190

Gln Met Glu Pro Arg Thr His Pro Thr Trp
        195                 200

<210>  25
<211>  294
<212>  PRT
<213>  Cynomolgus sp.

<400>  25

Thr Thr Cys Ala Cys Ala Gly Thr Gly Ala Cys Ala Gly Thr Cys Cys
1               5                   10                  15

Cys Thr Ala Ala Gly Gly Ala Ala Cys Thr Gly Thr Ala Cys Ala Thr
            20                  25                  30

Ala Ala Thr Ala Gly Ala Gly Cys Ala Thr Gly Gly Cys Ala Gly Cys
        35                  40                  45

Ala Ala Thr Gly Thr Gly Ala Cys Cys Cys Thr Gly Gly Ala Ala Thr
    50                  55                  60

Gly Cys Ala Ala Cys Thr Thr Thr Gly Ala Cys Ala Cys Thr Gly Gly
65                  70                  75                  80

Ala Ala Gly Thr Cys Ala Thr Gly Thr Gly Ala Ala Cys Cys Thr Thr
                85                  90                  95

Gly Gly Ala Gly Cys Ala Ala Thr Ala Ala Cys Ala Gly Cys Cys Ala
            100                 105                 110

Gly Thr Thr Thr Gly Cys Ala Ala Ala Ala Gly Gly Thr Gly Gly Ala
        115                 120                 125

Ala Ala Ala Thr Gly Ala Thr Ala Cys Ala Thr Cys Cys Cys Cys Ala
    130                 135                 140

Cys Ala Cys Cys Gly Thr Gly Ala Ala Ala Gly Ala Gly Cys Cys Ala
145                 150                 155                 160

Cys Thr Thr Thr Gly Cys Thr Gly Gly Ala Gly Gly Ala Gly Cys Ala
                165                 170                 175

Gly Cys Thr Gly Cys Cys Cys Cys Thr Ala Gly Gly Gly Ala Ala Gly
            180                 185                 190

Gly Cys Cys Thr Cys Gly Thr Thr Cys Cys Ala Cys Ala Thr Ala Cys
        195                 200                 205

Cys Thr Cys Ala Ala Gly Thr Cys Cys Ala Ala Gly Thr Gly Ala Gly
    210                 215                 220

Gly Gly Ala Cys Gly Ala Ala Gly Gly Ala Cys Ala Gly Thr Ala Cys
225                 230                 235                 240

Cys Ala Ala Thr Gly Cys Ala Thr Ala Ala Thr Cys Ala Thr Cys Thr
                245                 250                 255

Ala Thr Gly Gly Gly Gly Thr Cys Gly Cys Cys Thr Gly Gly Gly Ala

260                 265                 270

Cys Thr Ala Cys Ala Ala Gly Thr Ala Cys Cys Thr Gly Ala Cys Thr
        275                 280                 285

Cys Thr Gly Ala Ala Ala
    290

<210>  26
<211>  98
<212>  PRT
<213>  Cynomolgus sp.

<400>  26

Phe Thr Val Thr Val Pro Lys Glu Leu Tyr Ile Ile Glu His Gly Ser
1               5                   10                  15

Asn Val Thr Leu Glu Cys Asn Phe Asp Thr Gly Ser His Val Asn Leu
            20                  25                  30

Gly Ala Ile Thr Ala Ser Leu Gln Lys Val Glu Asn Asp Thr Ser Pro
        35                  40                  45

His Arg Glu Arg Ala Thr Leu Leu Glu Glu Gln Leu Pro Leu Gly Lys
    50                  55                  60

Ala Ser Phe His Ile Pro Gln Val Gln Val Arg Asp Glu Gly Gln Tyr
65                  70                  75                  80

Gln Cys Ile Ile Ile Tyr Gly Val Ala Trp Asp Tyr Lys Tyr Leu Thr
                85                  90                  95

Leu Lys

<210>  27
<211>  663
<212>  DNA
<213>  Mus musculus

<400>  27
atgctgctcc tgctgccgat actgaacctg agcttacaac ttcatcctgt agcagcttta      60

ttcaccgtga cagcccctaa agaagtgtac accgtagacg tcggcagcag tgtgagcctg     120

gagtgcgatt ttgaccgcag agaatgcact gaactggaag ggataagagc cagtttgcag     180

aaggtagaaa atgatacgtc tctgcaaagt gaaagagcca ccctgctgga ggagcagctg     240

cccctgggaa aggctttgtt ccacatccct agtgtccaag tgagagattc cgggcagtac     300

cgttgcctgg tcatctgcgg ggccgcctgg gactacaagt acctgacggt gaaagtcaaa     360

gcttcttaca tgaggataga cactaggatc ctggaggttc caggtacagg ggaggtgcag 420
cttacctgcc aggctagagg ttatccccta gcagaagtgt cctggcaaaa tgtcagtgtt 480
cctgccaaca ccagccacat caggaccccc gaaggcctct accaggtcac cagtgttctg 540
cgcctcaagc ctcagcctag cagaaacttc agctgcatgt tctggaatgc tcacatgaag 600
gagctgactt cagccatcat tgaccctctg agtcggatgg aacccaaagt ccccagaacg 660
tgg 663

<210> 28
<211> 221
<212> PRT
<213> Mus musculus

<400> 28

Met Leu Leu Leu Leu Pro Ile Leu Asn Leu Ser Leu Gln Leu His Pro
1               5                   10                  15

Val Ala Ala Leu Phe Thr Val Thr Ala Pro Lys Glu Val Tyr Thr Val
            20                  25                  30

Asp Val Gly Ser Ser Val Ser Leu Glu Cys Asp Phe Asp Arg Arg Glu
        35                  40                  45

Cys Thr Glu Leu Glu Gly Ile Arg Ala Ser Leu Gln Lys Val Glu Asn
    50                  55                  60

Asp Thr Ser Leu Gln Ser Glu Arg Ala Thr Leu Leu Glu Glu Gln Leu
65                  70                  75                  80

Pro Leu Gly Lys Ala Leu Phe His Ile Pro Ser Val Gln Val Arg Asp
                85                  90                  95

Ser Gly Gln Tyr Arg Cys Leu Val Ile Cys Gly Ala Ala Trp Asp Tyr
            100                 105                 110

Lys Tyr Leu Thr Val Lys Val Lys Ala Ser Tyr Met Arg Ile Asp Thr
        115                 120                 125

Arg Ile Leu Glu Val Pro Gly Thr Gly Glu Val Gln Leu Thr Cys Gln
    130                 135                 140

Ala Arg Gly Tyr Pro Leu Ala Glu Val Ser Trp Gln Asn Val Ser Val
145                 150                 155                 160

Pro Ala Asn Thr Ser His Ile Arg Thr Pro Glu Gly Leu Tyr Gln Val
                165                 170                 175

Thr Ser Val Leu Arg Leu Lys Pro Gln Pro Ser Arg Asn Phe Ser Cys
            180                 185                 190

Met Phe Trp Asn Ala His Met Lys Glu Leu Thr Ser Ala Ile Ile Asp
        195                 200                 205

Pro Leu Ser Arg Met Glu Pro Lys Val Pro Arg Thr Trp
    210                 215                 220

<210>  29
<211>  202
<212>  PRT
<213>  Mus musculus

<400>  29

Leu Phe Thr Val Thr Ala Pro Lys Glu Val Tyr Thr Val Asp Val Gly
1               5                   10                  15

Ser Ser Val Ser Leu Glu Cys Asp Phe Asp Arg Arg Glu Cys Thr Glu
            20                  25                  30

Leu Glu Gly Ile Arg Ala Ser Leu Gln Lys Val Glu Asn Asp Thr Ser
        35                  40                  45

Leu Gln Ser Glu Arg Ala Thr Leu Leu Glu Glu Gln Leu Pro Leu Gly
    50                  55                  60

Lys Ala Leu Phe His Ile Pro Ser Val Gln Val Arg Asp Ser Gly Gln
65                  70                  75                  80

Tyr Arg Cys Leu Val Ile Cys Gly Ala Ala Trp Asp Tyr Lys Tyr Leu
                85                  90                  95

Thr Val Lys Val Lys Ala Ser Tyr Met Arg Ile Asp Thr Arg Ile Leu
            100                 105                 110

Glu Val Pro Gly Thr Gly Glu Val Gln Leu Thr Cys Gln Ala Arg Gly
        115                 120                 125

Tyr Pro Leu Ala Glu Val Ser Trp Gln Asn Val Ser Val Pro Ala Asn
    130                 135                 140

Thr Ser His Ile Arg Thr Pro Glu Gly Leu Tyr Gln Val Thr Ser Val
145                 150                 155                 160

Leu Arg Leu Lys Pro Gln Pro Ser Arg Asn Phe Ser Cys Met Phe Trp
                165                 170                 175

Asn Ala His Met Lys Glu Leu Thr Ser Ala Ile Ile Asp Pro Leu Ser
            180                 185                 190

Arg Met Glu Pro Lys Val Pro Arg Thr Trp
        195                 200

<210> 30
<211> 294
<212> DNA
<213> Mus musculus

<400> 30
ttcaccgtga cagcccctaa agaagtgtac accgtagacg tcggcagcag tgtgagcctg 60

gagtgcgatt ttgaccgcag agaatgcact gaactggaag ggataagagc cagtttgcag 120

aaggtagaaa atgatacgtc tctgcaaagt gaaagagcca ccctgctgga ggagcagctg 180

cccctgggaa aggctttgtt ccacatccct agtgtccaag tgagagattc cgggcagtac 240

cgttgcctgg tcatctgcgg ggccgcctgg gactacaagt acctgacggt gaaa 294

<210> 31
<211> 98
<212> PRT
<213> Mus musculus

<400> 31

Phe Thr Val Thr Ala Pro Lys Glu Val Tyr Thr Val Asp Val Gly Ser
1               5                   10                  15

Ser Val Ser Leu Glu Cys Asp Phe Asp Arg Arg Glu Cys Thr Glu Leu
            20                  25                  30

Glu Gly Ile Arg Ala Ser Leu Gln Lys Val Glu Asn Asp Thr Ser Leu
        35                  40                  45

Gln Ser Glu Arg Ala Thr Leu Leu Glu Glu Gln Leu Pro Leu Gly Lys
    50                  55                  60

Ala Leu Phe His Ile Pro Ser Val Gln Val Arg Asp Ser Gly Gln Tyr
65                  70                  75                  80

Arg Cys Leu Val Ile Cys Gly Ala Ala Trp Asp Tyr Lys Tyr Leu Thr
                85                  90                  95

Val Lys

<210> 32

<211>  220
<212>  PRT
<213>  Unknown

<220>
<223>  Extracellular domain of PD-L1

<400>  32

Phe Thr Val Thr Val Pro Lys Asp Leu Tyr Val Val Glu Tyr Gly Ser
1               5                   10                  15

Asn Met Thr Ile Glu Cys Lys Phe Pro Val Glu Lys Gln Leu Asp Leu
            20                  25                  30

Ala Ala Leu Ile Val Tyr Trp Glu Met Glu Asp Lys Asn Ile Ile Gln
        35                  40                  45

Phe Val His Gly Glu Glu Asp Leu Lys Val Gln His Ser Ser Tyr Arg
    50                  55                  60

Gln Arg Ala Arg Leu Leu Lys Asp Gln Leu Ser Leu Gly Asn Ala Ala
65                  70                  75                  80

Leu Gln Ile Thr Asp Val Lys Leu Gln Asp Ala Gly Val Tyr Arg Cys
                85                  90                  95

Met Ile Ser Tyr Gly Gly Ala Asp Tyr Lys Arg Ile Thr Val Lys Val
            100                 105                 110

Asn Ala Pro Tyr Asn Lys Ile Asn Gln Arg Ile Leu Val Val Asp Pro
        115                 120                 125

Val Thr Ser Glu His Glu Leu Thr Cys Gln Ala Glu Gly Tyr Pro Lys
    130                 135                 140

Ala Glu Val Ile Trp Thr Ser Ser Asp His Gln Val Leu Ser Gly Lys
145                 150                 155                 160

Thr Thr Thr Thr Asn Ser Lys Arg Glu Glu Lys Leu Phe Asn Val Thr
                165                 170                 175

Ser Thr Leu Arg Ile Asn Thr Thr Thr Asn Glu Ile Phe Tyr Cys Thr
            180                 185                 190

Phe Arg Arg Leu Asp Pro Glu Glu Asn His Thr Ala Glu Leu Val Ile
        195                 200                 205

Pro Glu Leu Pro Leu Ala His Pro Pro Asn Glu Arg
    210                 215                 220

<210> 33
<211> 239
<212> PRT
<213> Mus musculus

<400> 33

Phe Thr Ile Thr Ala Pro Lys Asp Leu Tyr Val Val Glu Tyr Gly Ser
1               5                   10                  15

Asn Val Thr Met Glu Cys Arg Phe Pro Val Glu Arg Glu Leu Asp Leu
            20                  25                  30

Leu Ala Leu Val Val Tyr Trp Glu Lys Glu Asp Glu Gln Val Ile Gln
        35                  40                  45

Phe Val Ala Gly Glu Glu Asp Leu Lys Pro Gln His Ser Asn Phe Arg
    50                  55                  60

Gly Arg Ala Ser Leu Pro Lys Asp Gln Leu Leu Lys Gly Asn Ala Ala
65                  70                  75                  80

Leu Gln Ile Thr Asp Val Lys Leu Gln Asp Ala Gly Val Tyr Cys Cys
                85                  90                  95

Ile Ile Ser Tyr Gly Gly Ala Asp Tyr Lys Arg Ile Thr Leu Lys Val
            100                 105                 110

Asn Ala Pro Tyr Arg Lys Ile Asn Gln Arg Ile Ser Val Asp Pro Ala
        115                 120                 125

Thr Ser Glu His Glu Leu Ile Cys Gln Ala Glu Gly Tyr Pro Glu Ala
    130                 135                 140

Glu Val Ile Trp Thr Asn Ser Asp His Gln Pro Val Ser Gly Lys Arg
145                 150                 155                 160

Ser Val Thr Thr Ser Arg Thr Glu Gly Met Leu Leu Asn Val Thr Ser
                165                 170                 175

Ser Leu Arg Val Asn Ala Thr Ala Asn Asp Val Phe Tyr Cys Thr Phe
            180                 185                 190

Trp Arg Ser Gln Pro Gly Gln Asn His Thr Ala Glu Leu Ile Ile Pro
        195                 200                 205

Glu Leu Pro Ala Thr His Pro Pro Gln Asn Arg Thr His Trp Val Leu
    210                 215                 220

Leu Gly Ser Ile Leu Leu Phe Leu Ile Val Val Ser Thr Val Leu
225                 230                 235

<210>  34
<211>  738
<212>  DNA
<213>  Mus muculus

<400>  34
atggcttgca attgtcagtt gatgcaggat acaccactcc tcaagtttcc atgtccaagg      60

ctcattcttc tctttgtgct gctgattcgt ctttcacaag tgtcttcaga tgttgatgaa     120

caactgtcca agtcagtgaa agataaggta ttgctgcctt gccgttacaa ctctcctcat     180

gaagatgagt ctgaagaccg aatctactgg caaaaacatg acaaagtggt gctgtctgtc     240

attgctggga aactaaaagt gtggcccgag tataagaacc ggactttata tgacaacact     300

acctactctc ttatcatcct gggcctggtc ctttcagacc ggggcacata cagctgtgtc     360

gttcaaaaga aggaaagagg aacgtatgaa gttaaacact tggctttagt aaagttgtcc     420

atcaaagctg acttctctac ccccaacata actgagtctg gaaacccatc tgcagacact     480

aaaaggatta cctgctttgc ttccgggggt ttcccaaagc ctcgcttctc ttggttggaa     540

aatggaagag aattacctgg catcaatacg acaatttccc aggatcctga atctgaattg     600

tacaccatta gtagccaact agatttcaat acgactcgca accacaccat taagtgtctc     660

attaaatatg gagatgctca cgtgtcagag gacttcacct gggaaaaacc cccagaagac     720

cctcctgata gcaagaac                                                   738

<210>  35
<211>  246
<212>  PRT
<213>  Mus musculus

<400>  35

Met Ala Cys Asn Cys Gln Leu Met Gln Asp Thr Pro Leu Leu Lys Phe
1               5                   10                  15

Pro Cys Pro Arg Leu Ile Leu Leu Phe Val Leu Leu Ile Arg Leu Ser
            20                  25                  30

Gln Val Ser Ser Asp Val Asp Glu Gln Leu Ser Lys Ser Val Lys Asp
        35                  40                  45

Lys Val Leu Leu Pro Cys Arg Tyr Asn Ser Pro His Glu Asp Glu Ser
    50                  55                  60

Glu Asp Arg Ile Tyr Trp Gln Lys His Asp Lys Val Val Leu Ser Val

65                  70                  75                  80

Ile Ala Gly Lys Leu Lys Val Trp Pro Glu Tyr Lys Asn Arg Thr Leu
                85                  90                  95

Tyr Asp Asn Thr Thr Tyr Ser Leu Ile Ile Leu Gly Leu Val Leu Ser
            100                 105                 110

Asp Arg Gly Thr Tyr Ser Cys Val Val Gln Lys Lys Glu Arg Gly Thr
        115                 120                 125

Tyr Glu Val Lys His Leu Ala Leu Val Lys Leu Ser Ile Lys Ala Asp
    130                 135                 140

Phe Ser Thr Pro Asn Ile Thr Glu Ser Gly Asn Pro Ser Ala Asp Thr
145                 150                 155                 160

Lys Arg Ile Thr Cys Phe Ala Ser Gly Gly Phe Pro Lys Pro Arg Phe
                165                 170                 175

Ser Trp Leu Glu Asn Gly Arg Glu Leu Pro Gly Ile Asn Thr Thr Ile
            180                 185                 190

Ser Gln Asp Pro Glu Ser Glu Leu Tyr Thr Ile Ser Ser Gln Leu Asp
        195                 200                 205

Phe Asn Thr Thr Arg Asn His Thr Ile Lys Cys Leu Ile Lys Tyr Gly
    210                 215                 220

Asp Ala His Val Ser Glu Asp Phe Thr Trp Glu Lys Pro Pro Glu Asp
225                 230                 235                 240

Pro Pro Asp Ser Lys Asn
                245

<210>  36
<211>  209
<212>  PRT
<213>  Mus musculus

<400>  36

Val Asp Glu Gln Leu Ser Lys Ser Val Lys Asp Lys Val Leu Leu Pro
1               5                   10                  15

Cys Arg Tyr Asn Ser Pro His Glu Asp Glu Ser Glu Asp Arg Ile Tyr
            20                  25                  30

Trp Gln Lys His Asp Lys Val Val Leu Ser Val Ile Ala Gly Lys Leu

35                  40                  45

Lys Val Trp Pro Glu Tyr Lys Asn Arg Thr Leu Tyr Asp Asn Thr Thr
    50                  55                  60

Tyr Ser Leu Ile Ile Leu Gly Leu Val Leu Ser Asp Arg Gly Thr Tyr
65                  70                  75                  80

Ser Cys Val Val Gln Lys Lys Glu Arg Gly Thr Tyr Glu Val Lys His
                85                  90                  95

Leu Ala Leu Val Lys Leu Ser Ile Lys Ala Asp Phe Ser Thr Pro Asn
            100                 105                 110

Ile Thr Glu Ser Gly Asn Pro Ser Ala Asp Thr Lys Arg Ile Thr Cys
        115                 120                 125

Phe Ala Ser Gly Gly Phe Pro Lys Pro Arg Phe Ser Trp Leu Glu Asn
    130                 135                 140

Gly Arg Glu Leu Pro Gly Ile Asn Thr Thr Ile Ser Gln Asp Pro Glu
145                 150                 155                 160

Ser Glu Leu Tyr Thr Ile Ser Ser Gln Leu Asp Phe Asn Thr Thr Arg
                165                 170                 175

Asn His Thr Ile Lys Cys Leu Ile Lys Tyr Gly Asp Ala His Val Ser
            180                 185                 190

Glu Asp Phe Thr Trp Glu Lys Pro Pro Glu Asp Pro Pro Asp Ser Lys
        195                 200                 205

Asn

<210>  37
<211>  291
<212>  PRT
<213>  Mus musculus

<400>  37

Gly Thr Thr Gly Ala Thr Gly Ala Ala Cys Ala Ala Cys Thr Gly Thr
1               5                   10                  15

Cys Cys Ala Ala Gly Thr Cys Ala Gly Thr Gly Ala Ala Ala Gly Ala
            20                  25                  30

Thr Ala Ala Gly Gly Thr Ala Thr Thr Gly Cys Thr Gly Cys Cys Thr

        35                  40                  45

Thr Gly Cys Cys Gly Thr Thr Ala Cys Ala Ala Cys Thr Cys Thr Cys
    50                  55                  60

Cys Thr Cys Ala Thr Gly Ala Ala Gly Ala Thr Gly Ala Gly Thr Cys
65                  70                  75                  80

Thr Gly Ala Ala Gly Ala Cys Cys Gly Ala Ala Thr Cys Thr Ala Cys
                85                  90                  95

Thr Gly Gly Cys Ala Ala Ala Ala Ala Cys Ala Thr Gly Ala Cys Ala
            100                 105                 110

Ala Ala Gly Thr Gly Gly Thr Gly Cys Thr Gly Thr Cys Thr Gly Thr
        115                 120                 125

Cys Ala Thr Thr Gly Cys Thr Gly Gly Gly Ala Ala Ala Cys Thr Ala
    130                 135                 140

Ala Ala Ala Gly Thr Gly Thr Gly Gly Cys Cys Cys Gly Ala Gly Thr
145                 150                 155                 160

Ala Thr Ala Ala Gly Ala Ala Cys Cys Gly Gly Ala Cys Thr Thr Thr
                165                 170                 175

Ala Thr Ala Thr Gly Ala Cys Ala Ala Cys Ala Cys Thr Ala Cys Cys
            180                 185                 190

Thr Ala Cys Thr Cys Thr Cys Thr Thr Ala Thr Cys Ala Thr Cys Cys
        195                 200                 205

Thr Gly Gly Gly Cys Cys Thr Gly Gly Thr Cys Cys Thr Thr Thr Cys
    210                 215                 220

Ala Gly Ala Cys Cys Gly Gly Gly Gly Cys Ala Cys Ala Thr Ala Cys
225                 230                 235                 240

Ala Gly Cys Thr Gly Thr Gly Thr Cys Gly Thr Thr Cys Ala Ala Ala
                245                 250                 255

Ala Gly Ala Ala Gly Gly Ala Ala Ala Gly Ala Gly Gly Ala Ala Cys
            260                 265                 270

Gly Thr Ala Thr Gly Ala Ala Gly Thr Thr Ala Ala Ala Cys Ala Cys
        275                 280                 285

Thr Thr Gly
    290

<210>  38
<211>  97
<212>  PRT
<213>  Mus musculus

<400>  38

Val Asp Glu Gln Leu Ser Lys Ser Val Lys Asp Lys Val Leu Leu Pro
1               5                   10                  15

Cys Arg Tyr Asn Ser Pro His Glu Asp Glu Ser Glu Asp Arg Ile Tyr
            20                  25                  30

Trp Gln Lys His Asp Lys Val Val Leu Ser Val Ile Ala Gly Lys Leu
        35                  40                  45

Lys Val Trp Pro Glu Tyr Lys Asn Arg Thr Leu Tyr Asp Asn Thr Thr
    50                  55                  60

Tyr Ser Leu Ile Ile Leu Gly Leu Val Leu Ser Asp Arg Gly Thr Tyr
65                  70                  75                  80

Ser Cys Val Val Gln Lys Lys Glu Arg Gly Thr Tyr Glu Val Lys His
                85                  90                  95

Leu

<210>  39
<211>  732
<212>  DNA
<213>  Homo sapiens

<400>  39
atgggccaca cacggaggca gggaacatca ccatccaagt gtccatacct caatttcttt      60
cagctcttgg tgctggctgg tctttctcac ttctgttcag gtgttatcca cgtgaccaag     120
gaagtgaaag aagtggcaac gctgtcctgt ggtcacaatg tttctgttga agagctggca     180
caaactcgca tctactggca aaaggagaag aaaatggtgc tgactatgat gtctggggac     240
atgaatatat ggcccgagta caagaaccgg accatctttg atatcactaa taacctctcc     300
attgtgatcc tggctctgcg cccatctgac gagggcacat acgagtgtgt tgttctgaag     360
tatgaaaaag acgctttcaa gcgggaacac ctggctgaag tgacgttatc agtcaaagct     420
gacttcccta cacctagtat atctgacttt gaaattccaa cttctaatat tagaaggata     480
atttgctcaa cctctggagg ttttccagag cctcacctct cctggttgga aaatggagaa     540

gaattaaatg ccatcaacac aacagtttcc caagatcctg aaactgagct ctatgctgtt 600

agcagcaaac tggatttcaa tatgacaacc aaccacagct tcatgtgtct catcaagtat 660

ggacatttaa gagtgaatca gaccttcaac tggaatacaa ccaagcaaga gcattttcct 720

gataacctgc tc 732

<210> 40
<211> 283
<212> PRT
<213> Homo sapiens

<400> 40

Met Ile Phe Leu Leu Leu Met Leu Ser Leu Glu Leu Gln Leu His Gln
1               5                   10                  15

Ile Ala Ala Leu Phe Thr Val Thr Val Pro Lys Glu Leu Tyr Ile Ile
            20                  25                  30

Glu His Gly Ser Asn Val Thr Leu Met Gly His Thr Arg Arg Gln Gly
        35                  40                  45

Thr Ser Pro Ser Lys Cys Pro Tyr Leu Asn Phe Phe Gln Leu Leu Val
    50                  55                  60

Leu Ala Gly Leu Ser His Phe Cys Ser Gly Val Ile His Val Thr Lys
65                  70                  75                  80

Glu Val Lys Glu Val Ala Thr Leu Ser Cys Gly His Asn Val Ser Val
                85                  90                  95

Glu Glu Leu Ala Gln Thr Arg Ile Tyr Trp Gln Lys Glu Lys Lys Met
            100                 105                 110

Val Leu Thr Met Met Ser Gly Asp Met Asn Ile Trp Pro Glu Tyr Lys
        115                 120                 125

Asn Arg Thr Ile Phe Asp Ile Thr Asn Asn Leu Ser Ile Val Ile Leu
    130                 135                 140

Ala Leu Arg Pro Ser Asp Glu Gly Thr Tyr Glu Cys Val Val Leu Lys
145                 150                 155                 160

Tyr Glu Lys Asp Ala Phe Lys Arg Glu His Leu Ala Glu Val Thr Leu
                165                 170                 175

Ser Val Lys Ala Asp Phe Pro Thr Pro Ser Ile Ser Asp Phe Glu Ile
            180                 185                 190

Pro Thr Ser Asn Ile Arg Arg Ile Ile Cys Ser Thr Ser Gly Gly Phe
        195                 200                 205

Pro Glu Pro His Leu Ser Trp Leu Glu Asn Gly Glu Glu Leu Asn Ala
    210                 215                 220

Ile Asn Thr Thr Val Ser Gln Asp Pro Glu Thr Glu Leu Tyr Ala Val
225                 230                 235                 240

Ser Ser Lys Leu Asp Phe Asn Met Thr Thr Asn His Ser Phe Met Cys
                245                 250                 255

Leu Ile Lys Tyr Gly His Leu Arg Val Asn Gln Thr Phe Asn Trp Asn
            260                 265                 270

Thr Thr Lys Gln Glu His Phe Pro Asp Asn Leu
        275                 280

<210> 41
<211> 209
<212> PRT
<213> Homo sapiens

<400> 41

Val Ile His Val Thr Lys Glu Val Lys Glu Val Ala Thr Leu Ser Cys
1               5                   10                  15

Gly His Asn Val Ser Val Glu Glu Leu Ala Gln Thr Arg Ile Tyr Trp
            20                  25                  30

Gln Lys Glu Lys Lys Met Val Leu Thr Met Met Ser Gly Asp Met Asn
        35                  40                  45

Ile Trp Pro Glu Tyr Lys Asn Arg Thr Ile Phe Asp Ile Thr Asn Asn
    50                  55                  60

Leu Ser Ile Val Ile Leu Ala Leu Arg Pro Ser Asp Glu Gly Thr Tyr
65                  70                  75                  80

Glu Cys Val Val Leu Lys Tyr Glu Lys Asp Ala Phe Lys Arg Glu His
                85                  90                  95

Leu Ala Glu Val Thr Leu Ser Val Lys Ala Asp Phe Pro Thr Pro Ser
            100                 105                 110

Ile Ser Asp Phe Glu Ile Pro Thr Ser Asn Ile Arg Arg Ile Ile Cys
        115                 120                 125

Ser Thr Ser Gly Gly Phe Pro Glu Pro His Leu Ser Trp Leu Glu Asn
    130                 135                 140

Gly Glu Glu Leu Asn Ala Ile Asn Thr Thr Val Ser Gln Asp Pro Glu
145                 150                 155                 160

Thr Glu Leu Tyr Ala Val Ser Ser Lys Leu Asp Phe Asn Met Thr Thr
                165                 170                 175

Asn His Ser Phe Met Cys Leu Ile Lys Tyr Gly His Leu Arg Val Asn
            180                 185                 190

Gln Thr Phe Asn Trp Asn Thr Thr Lys Gln Glu His Phe Pro Asp Asn
        195                 200                 205

Leu

<210>  42
<211>  303
<212>  DNA
<213>  Homo sapiens

<400>  42
gttatccacg tgaccaagga agtgaaagaa gtggcaacgc tgtcctgtgg tcacaatgtt      60

tctgttgaag agctggcaca aactcgcatc tactggcaaa aggagaagaa aatggtgctg     120

actatgatgt ctggggacat gaatatatgg cccgagtaca agaaccggac catctttgat     180

atcactaata acctctccat tgtgatcctg gctctgcgcc catctgacga gggcacatac     240

gagtgtgttg ttctgaagta tgaaaaagac gctttcaagc gggaacacct ggctgaagtg     300

acg                                                                   303

<210>  43
<211>  101
<212>  PRT
<213>  Homo sapiens

<400>  43

Val Ile His Val Thr Lys Glu Val Lys Glu Val Ala Thr Leu Ser Cys
1               5                   10                  15

Gly His Asn Val Ser Val Glu Glu Leu Ala Gln Thr Arg Ile Tyr Trp
            20                  25                  30

Gln Lys Glu Lys Lys Met Val Leu Thr Met Met Ser Gly Asp Met Asn
        35                  40                  45

Ile Trp Pro Glu Tyr Lys Asn Arg Thr Ile Phe Asp Ile Thr Asn Asn
    50                  55                  60

Leu Ser Ile Val Ile Leu Ala Leu Arg Pro Ser Asp Glu Gly Thr Tyr
65                  70                  75                  80

Glu Cys Val Val Leu Lys Tyr Glu Lys Asp Ala Phe Lys Arg Glu His
                85                  90                  95

Leu Ala Glu Val Thr
            100

<210>  44
<211>  696
<212>  DNA
<213>  Homo sapiens

<400>  44
gagcctaagt catgtgacaa gacccatacg tgcccaccct gtcccgctcc agaactgctg      60

gggggaccta gcgttttctt gttcccccca aagcccaagg acaccctcat gatctcacgg     120

actcccgaag taacatgcgt agtagtcgac gtgagccacg aggatcctga agtgaagttt     180

aattggtacg tggacggagt cgaggtgcat aatgccaaaa ctaaacctcg ggaggagcag     240

tataacagta cctaccgcgt ggtatccgtc ttgacagtgc tccaccagga ctggctgaat     300

ggtaaggagt ataaatgcaa ggtcagcaac aaagctcttc ccgccccaat tgaaaagact     360

atcagcaagg ccaagggaca accccgcgag ccccaggttt acacccttcc accttcacga     420

gacgagctga ccaagaacca ggtgtctctg acttgtctgg tcaaaggttt ctatccttcc     480

gacatcgcag tggagtggga gtcaaacggg cagcctgaga ataactacaa gaccacaccc     540

ccagtgcttg atagcgatgg gagctttttc ctctacagta agctgactgt ggacaaatcc     600

cgctggcagc agggaaacgt tttctcttgt agcgtcatgc atgaggccct ccacaaccat     660

tatactcaga aaagcctgag tctgagtccc ggcaaa                               696

<210>  45
<211>  231
<212>  PRT
<213>  Homo sapiens

<400>  45

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val

35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Thr Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Asn His Tyr Thr Gln Lys Ser
    210                 215                 220

Leu Ser Leu Ser Pro Gly Lys
225                 230

<210>  46
<211>  699
<212>  DNA
<213>  Mus musculus

<400>  46
gagccaagag gtcctacgat caagccctgc ccgccttgta aatgcccagc tccaaatttg      60

ctgggtggac cgtcagtctt tatcttcccg ccaaagataa aggacgtctt gatgattagt     120

ctgagcccca tcgtgacatg cgttgtggtg gatgtttcag aggatgaccc cgacgtgcaa     180

atcagttggt tcgttaacaa cgtggaggtg cataccgctc aaacccagac ccacagagag 240

gattataaca gcaccctgcg ggtagtgtcc gccctgccga tccagcatca ggattggatg 300

agcgggaaag agttcaagtg taaggtaaac aacaaagatc tgccagcgcc gattgaacga 360

accattagca agccgaaagg gagcgtgcgc gcacctcagg tttacgtcct tcctccacca 420

gaagaggaga tgacgaaaaa gcaggtgacc ctgacatgca tggtaactga ctttatgcca 480

gaagatattt acgtggaatg gactaataac ggaaagacag agctcaatta caagaacact 540

gagcctgttc tggattctga tggcagctac tttatgtact ccaaattgag ggtcgagaag 600

aagaattggg tcgagagaaa cagttatagt tgctcagtgg tgcatgaggg cctccataat 660

catcacacca caaagtcctt cagccgaacg cccgggaaa 699

<210> 47
<211> 233
<212> PRT
<213> Mus musculus

<400> 47

Glu Pro Arg Gly Pro Thr Ile Lys Pro Cys Pro Pro Cys Lys Cys Pro
1               5                   10                  15

Ala Pro Asn Leu Leu Gly Gly Pro Ser Val Phe Ile Phe Pro Pro Lys
            20                  25                  30

Ile Lys Asp Val Leu Met Ile Ser Leu Ser Pro Ile Val Thr Cys Val
        35                  40                  45

Val Val Asp Val Ser Glu Asp Asp Pro Asp Val Gln Ile Ser Trp Phe
    50                  55                  60

Val Asn Asn Val Glu Val His Thr Ala Gln Thr Gln Thr His Arg Glu
65                  70                  75                  80

Asp Tyr Asn Ser Thr Leu Arg Val Val Ser Ala Leu Pro Ile Gln His
                85                  90                  95

Gln Asp Trp Met Ser Gly Lys Glu Phe Lys Cys Lys Val Asn Asn Lys
            100                 105                 110

Asp Leu Pro Ala Pro Ile Glu Arg Thr Ile Ser Lys Pro Lys Gly Ser
        115                 120                 125

Val Arg Ala Pro Gln Val Tyr Val Leu Pro Pro Pro Glu Glu Glu Met
    130                 135                 140

Thr Lys Lys Gln Val Thr Leu Thr Cys Met Val Thr Asp Phe Met Pro

145                 150                 155                 160

Glu Asp Ile Tyr Val Glu Trp Thr Asn Asn Gly Lys Thr Glu Leu Asn
                165                 170                 175

Tyr Lys Asn Thr Glu Pro Val Leu Asp Ser Asp Gly Ser Tyr Phe Met
            180                 185                 190

Tyr Ser Lys Leu Arg Val Glu Lys Lys Asn Trp Val Glu Arg Asn Ser
        195                 200                 205

Tyr Ser Cys Ser Val Val His Glu Gly Leu His Asn His His Thr Thr
    210                 215                 220

Lys Ser Phe Ser Arg Thr Pro Gly Lys
225                 230

<210>  48
<211>  4
<212>  PRT
<213>  Artificial sequence

<220>
<223>  Synthetic flexible peptide

<400>  48

Gly Ser Gly Ser
1

<210>  49
<211>  4
<212>  PRT
<213>  Artificial Sequence

<220>
<223>  Synthetic flexible peptide

<400>  49

Gly Gly Gly Ser
1

<210>  50
<211>  15
<212>  PRT
<213>  Artificial sequence

<220>
<223>  Synthetic flexible peptide

<400>  50

Gly Gly Gly Gly Ser Gly Gly Gly Gly Ser Gly Gly Gly Gly Ser
1               5                   10                  15

<210> 51
<211> 20
<212> PRT
<213> Artificial Sequence

<220>
<223> Synthetic flexible peptide

<400> 51

Gly Gly Gly Gly Ser Gly Gly Gly Gly Ser Gly Gly Gly Gly Ser Gly
1               5                   10                  15

Gly Gly Gly Ser
            20

<210> 52
<211> 1365
<212> DNA
<213> Artificial Sequence

<220>
<223> Synthetic flexible peptide

<400> 52
atgctgctcc tgctgccgat actgaacctg agcttacaac ttcatcctgt agcagcttta 60
ttcaccgtga cagcccctaa agaagtgtac accgtagacg tcggcagcag tgtgagcctg 120
gagtgcgatt ttgaccgcag agaatgcact gaactggaag ggataagagc cagtttgcag 180
aaggtagaaa atgatacgtc tctgcaaagt gaaagagcca ccctgctgga ggagcagctg 240
cccctgggaa aggctttgtt ccacatccct agtgtccaag tgagagattc cgggcagtac 300
cgttgcctgg tcatctgcgg ggccgcctgg gactacaagt acctgacggt gaaagtcaaa 360
gcttcttaca tgaggataga cactaggatc ctggaggttc caggtacagg ggaggtgcag 420
cttacctgcc aggctagagg ttatccccta gcagaagtgt cctggcaaaa tgtcagtgtt 480
cctgccaaca ccagccacat caggaccccc gaaggcctct accaggtcac cagtgttctg 540
cgcctcaagc ctcagcctag cagaaacttc agctgcatgt tctggaatgc tcacatgaag 600
gagctgactt cagccatcat tgaccctctg agtcggatgg aacccaaagt ccccagaacg 660
tgggagccaa gaggtcctac gatcaagccc tgcccgcctt gtaaatgccc agctccaaat 720
ttgctgggtg gaccgtcagt ctttatcttc ccgccaaaga taaaggacgt cttgatgatt 780
agtctgagcc ccatcgtgac atgcgttgtg gtggatgttt cagaggatga ccccgacgtg 840
caaatcagtt ggttcgttaa caacgtggag gtgcataccg ctcaaaccca gacccacaga 900
gaggattata acagcaccct gcgggtagtg tccgccctgc cgatccagca tcaggattgg 960
atgagcggga aagagttcaa gtgtaaggta aacaacaaag atctgccagc gccgattgaa 1020

cgaaccatta gcaagccgaa agggagcgtg cgcgcacctc aggtttacgt ccttcctcca 1080

ccagaagagg agatgacgaa aaagcaggtg accctgacat gcatggtaac tgactttatg 1140

ccagaagata tttacgtgga atggactaat aacggaaaga cagagctcaa ttacaagaac 1200

actgagcctg ttctggattc tgatggcagc tactttatgt actccaaatt gagggtcgag 1260

aagaagaatt gggtcgagag aaacagttat agttgctcag tggtgcatga gggcctccat 1320

aatcatcaca ccacaaagtc cttcagccga acgcccggga aatga 1365

<210> 53
<211> 454
<212> PRT
<213> Mus musculus

<400> 53

Met Leu Leu Leu Leu Pro Ile Leu Asn Leu Ser Leu Gln Leu His Pro
1               5                   10                  15

Val Ala Ala Leu Phe Thr Val Thr Ala Pro Lys Glu Val Tyr Thr Val
            20                  25                  30

Asp Val Gly Ser Ser Val Ser Leu Glu Cys Asp Phe Asp Arg Arg Glu
        35                  40                  45

Cys Thr Glu Leu Glu Gly Ile Arg Ala Ser Leu Gln Lys Val Glu Asn
    50                  55                  60

Asp Thr Ser Leu Gln Ser Glu Arg Ala Thr Leu Leu Glu Glu Gln Leu
65                  70                  75                  80

Pro Leu Gly Lys Ala Leu Phe His Ile Pro Ser Val Gln Val Arg Asp
                85                  90                  95

Ser Gly Gln Tyr Arg Cys Leu Val Ile Cys Gly Ala Ala Trp Asp Tyr
            100                 105                 110

Lys Tyr Leu Thr Val Lys Val Lys Ala Ser Tyr Met Arg Ile Asp Thr
        115                 120                 125

Arg Ile Leu Glu Val Pro Gly Thr Gly Glu Val Gln Leu Thr Cys Gln
    130                 135                 140

Ala Arg Gly Tyr Pro Leu Ala Glu Val Ser Trp Gln Asn Val Ser Val
145                 150                 155                 160

Pro Ala Asn Thr Ser His Ile Arg Thr Pro Glu Gly Leu Tyr Gln Val
                165                 170                 175

Thr Ser Val Leu Arg Leu Lys Pro Gln Pro Ser Arg Asn Phe Ser Cys
            180                 185                 190

Met Phe Trp Asn Ala His Met Lys Glu Leu Thr Ser Ala Ile Ile Asp
        195                 200                 205

Pro Leu Ser Arg Met Glu Pro Lys Val Pro Arg Thr Trp Glu Pro Arg
    210                 215                 220

Gly Pro Thr Ile Lys Pro Cys Pro Pro Cys Lys Cys Pro Ala Pro Asn
225                 230                 235                 240

Leu Leu Gly Gly Pro Ser Val Phe Ile Phe Pro Pro Lys Ile Lys Asp
                245                 250                 255

Val Leu Met Ile Ser Leu Ser Pro Ile Val Thr Cys Val Val Val Asp
            260                 265                 270

Val Ser Glu Asp Asp Pro Asp Val Gln Ile Ser Trp Phe Val Asn Asn
        275                 280                 285

Val Glu Val His Thr Ala Gln Thr Gln Thr His Arg Glu Asp Tyr Asn
    290                 295                 300

Ser Thr Leu Arg Val Val Ser Ala Leu Pro Ile Gln His Gln Asp Trp
305                 310                 315                 320

Met Ser Gly Lys Glu Phe Lys Cys Lys Val Asn Asn Lys Asp Leu Pro
                325                 330                 335

Ala Pro Ile Glu Arg Thr Ile Ser Lys Pro Lys Gly Ser Val Arg Ala
            340                 345                 350

Pro Gln Val Tyr Val Leu Pro Pro Pro Glu Glu Glu Met Thr Lys Lys
        355                 360                 365

Gln Val Thr Leu Thr Cys Met Val Thr Asp Phe Met Pro Glu Asp Ile
    370                 375                 380

Tyr Val Glu Trp Thr Asn Asn Gly Lys Thr Glu Leu Asn Tyr Lys Asn
385                 390                 395                 400

Thr Glu Pro Val Leu Asp Ser Asp Gly Ser Tyr Phe Met Tyr Ser Lys
                405                 410                 415

Leu Arg Val Glu Lys Lys Asn Trp Val Glu Arg Asn Ser Tyr Ser Cys
            420                 425                 430

Ser Val Val His Glu Gly Leu His Asn His His Thr Thr Lys Ser Phe
        435                 440                 445

Ser Arg Thr Pro Gly Lys
    450

<210>  54
<211>  435
<212>  PRT
<213>  Mus musculus

<400>  54

Leu Phe Thr Val Thr Ala Pro Lys Glu Val Tyr Thr Val Asp Val Gly
1               5                   10                  15

Ser Ser Val Ser Leu Glu Cys Asp Phe Asp Arg Arg Glu Cys Thr Glu
            20                  25                  30

Leu Glu Gly Ile Arg Ala Ser Leu Gln Lys Val Glu Asn Asp Thr Ser
        35                  40                  45

Leu Gln Ser Glu Arg Ala Thr Leu Leu Glu Glu Gln Leu Pro Leu Gly
    50                  55                  60

Lys Ala Leu Phe His Ile Pro Ser Val Gln Val Arg Asp Ser Gly Gln
65                  70                  75                  80

Tyr Arg Cys Leu Val Ile Cys Gly Ala Ala Trp Asp Tyr Lys Tyr Leu
                85                  90                  95

Thr Val Lys Val Lys Ala Ser Tyr Met Arg Ile Asp Thr Arg Ile Leu
            100                 105                 110

Glu Val Pro Gly Thr Gly Glu Val Gln Leu Thr Cys Gln Ala Arg Gly
        115                 120                 125

Tyr Pro Leu Ala Glu Val Ser Trp Gln Asn Val Ser Val Pro Ala Asn
    130                 135                 140

Thr Ser His Ile Arg Thr Pro Glu Gly Leu Tyr Gln Val Thr Ser Val
145                 150                 155                 160

Leu Arg Leu Lys Pro Gln Pro Ser Arg Asn Phe Ser Cys Met Phe Trp
                165                 170                 175

Asn Ala His Met Lys Glu Leu Thr Ser Ala Ile Ile Asp Pro Leu Ser
            180                 185                 190

Arg Met Glu Pro Lys Val Pro Arg Thr Trp Glu Pro Arg Gly Pro Thr
        195                 200                 205

Ile Lys Pro Cys Pro Pro Cys Lys Cys Pro Ala Pro Asn Leu Leu Gly
    210                 215                 220

Gly Pro Ser Val Phe Ile Phe Pro Pro Lys Ile Lys Asp Val Leu Met
225                 230                 235                 240

Ile Ser Leu Ser Pro Ile Val Thr Cys Val Val Val Asp Val Ser Glu
                245                 250                 255

Asp Asp Pro Asp Val Gln Ile Ser Trp Phe Val Asn Asn Val Glu Val
            260                 265                 270

His Thr Ala Gln Thr Gln Thr His Arg Glu Asp Tyr Asn Ser Thr Leu
        275                 280                 285

Arg Val Val Ser Ala Leu Pro Ile Gln His Gln Asp Trp Met Ser Gly
    290                 295                 300

Lys Glu Phe Lys Cys Lys Val Asn Asn Lys Asp Leu Pro Ala Pro Ile
305                 310                 315                 320

Glu Arg Thr Ile Ser Lys Pro Lys Gly Ser Val Arg Ala Pro Gln Val
                325                 330                 335

Tyr Val Leu Pro Pro Pro Glu Glu Glu Met Thr Lys Lys Gln Val Thr
            340                 345                 350

Leu Thr Cys Met Val Thr Asp Phe Met Pro Glu Asp Ile Tyr Val Glu
        355                 360                 365

Trp Thr Asn Asn Gly Lys Thr Glu Leu Asn Tyr Lys Asn Thr Glu Pro
    370                 375                 380

Val Leu Asp Ser Asp Gly Ser Tyr Phe Met Tyr Ser Lys Leu Arg Val
385                 390                 395                 400

Glu Lys Lys Asn Trp Val Glu Arg Asn Ser Tyr Ser Cys Ser Val Val
                405                 410                 415

His Glu Gly Leu His Asn His His Thr Thr Lys Ser Phe Ser Arg Thr
            420                 425                 430

Pro Gly Lys

        435

<210> 55
<211> 1362
<212> DNA
<213> Homo sapiens

<400> 55
atgatctttc ttctcttgat gctgtctttg gaattgcaac ttcaccaaat cgcggccctc 60

tttactgtga ccgtgccaaa agaactgtat atcattgagc acgggtccaa tgtgaccctc 120

gaatgtaact ttgacaccgg cagccacgtt aacctggggg ccatcactgc cagcttgcaa 180

aaagttgaaa acgacacttc acctcaccgg gagagggcaa ccctcttgga ggagcaactg 240

ccattgggga aggcctcctt tcatatccct caggtgcagg ttcgggatga gggacagtac 300

cagtgcatta ttatctacgg cgtggcttgg gattacaagt atctgaccct gaaggtgaaa 360

gcgtcctatc ggaaaattaa cactcacatt cttaaggtgc cagagacgga cgaggtggaa 420

ctgacatgcc aagccaccgg ctacccgttg gcagaggtca gctggcccaa cgtgagcgta 480

cctgctaaca cttctcattc taggacaccc gagggcctct accaggttac atccgtgctc 540

cgcctcaaac cgcccccagg ccggaatttt agttgcgtgt tttggaatac ccacgtgcga 600

gagctgactc ttgcatctat tgatctgcag tcccagatgg agccacggac tcatccaact 660

tgggaaccta aatcttgcga taaaactcat acctgtcccc cttgcccagc ccccgagctt 720

ctgggaggtc ccagtgtgtt tctgtttccc ccaaaaccta aggacacact tatgatatcc 780

cgaacgccgg aagtgacatg cgtggttgtg gacgtctcac acgaagaccc ggaggtgaaa 840

ttcaactggt acgttgacgg agttgaggtt cataacgcta agaccaagcc cagagaggag 900

caatacaatt ccacctatcg agtggttagt gtactgaccg ttttgcacca agactggctg 960

aatggaaaag aatacaagtg caaagtatca aacaaggctt tgcctgcacc catcgagaag 1020

acaatttcta aagccaaagg gcagcccagg gaaccgcagg tgtacacact cccaccatcc 1080

cgcgacgagc tgacaaagaa tcaagtatcc ctgacctgcc tggtgaaagg cttttaccca 1140

tctgacattg ccgtggaatg ggaatcaaat ggacaacctg agaacaacta caaaaccact 1200

ccacctgtgc ttgacagcga cgggtccttt ttcctgtaca gtaagctcac tgtcgataag 1260

tctcgctggc agcagggcaa cgtcttttca tgtagtgtga tgcacgaagc tctgcacaac 1320

cattacaccc agaagtctct gtcactgagc ccaggtaaat ga 1362

<210> 56
<211> 453
<212> PRT
<213> Homo sapiens

<400> 56

Met Ile Phe Leu Leu Leu Met Leu Ser Leu Glu Leu Gln Leu His Gln
1               5                   10                  15

Ile Ala Ala Leu Phe Thr Val Thr Val Pro Lys Glu Leu Tyr Ile Ile
            20                  25                  30

Glu His Gly Ser Asn Val Thr Leu Glu Cys Asn Phe Asp Thr Gly Ser
        35                  40                  45

His Val Asn Leu Gly Ala Ile Thr Ala Ser Leu Gln Lys Val Glu Asn
    50                  55                  60

Asp Thr Ser Pro His Arg Glu Arg Ala Thr Leu Leu Glu Glu Gln Leu
65                  70                  75                  80

Pro Leu Gly Lys Ala Ser Phe His Ile Pro Gln Val Gln Val Arg Asp
                85                  90                  95

Glu Gly Gln Tyr Gln Cys Ile Ile Ile Tyr Gly Val Ala Trp Asp Tyr
            100                 105                 110

Lys Tyr Leu Thr Leu Lys Val Lys Ala Ser Tyr Arg Lys Ile Asn Thr
        115                 120                 125

His Ile Leu Lys Val Pro Glu Thr Asp Glu Val Glu Leu Thr Cys Gln
    130                 135                 140

Ala Thr Gly Tyr Pro Leu Ala Glu Val Ser Trp Pro Asn Val Ser Val
145                 150                 155                 160

Pro Ala Asn Thr Ser His Ser Arg Thr Pro Glu Gly Leu Tyr Gln Val
                165                 170                 175

Thr Ser Val Leu Arg Leu Lys Pro Pro Pro Gly Arg Asn Phe Ser Cys
            180                 185                 190

Val Phe Trp Asn Thr His Val Arg Glu Leu Thr Leu Ala Ser Ile Asp
        195                 200                 205

Leu Gln Ser Gln Met Glu Pro Arg Thr His Pro Thr Trp Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210>  57
<211>  434
<212>  PRT
<213>  Homo sapiens

<400>  57

Leu Phe Thr Val Thr Val Pro Lys Glu Leu Tyr Ile Ile Glu His Gly
1               5                   10                  15

Ser Asn Val Thr Leu Glu Cys Asn Phe Asp Thr Gly Ser His Val Asn
            20                  25                  30

Leu Gly Ala Ile Thr Ala Ser Leu Gln Lys Val Glu Asn Asp Thr Ser
        35                  40                  45

Pro His Arg Glu Arg Ala Thr Leu Leu Glu Glu Gln Leu Pro Leu Gly
    50                  55                  60

Lys Ala Ser Phe His Ile Pro Gln Val Gln Val Arg Asp Glu Gly Gln
65                  70                  75                  80

Tyr Gln Cys Ile Ile Ile Tyr Gly Val Ala Trp Asp Tyr Lys Tyr Leu
                85                  90                  95

Thr Leu Lys Val Lys Ala Ser Tyr Arg Lys Ile Asn Thr His Ile Leu
            100                 105                 110

Lys Val Pro Glu Thr Asp Glu Val Glu Leu Thr Cys Gln Ala Thr Gly
        115                 120                 125

Tyr Pro Leu Ala Glu Val Ser Trp Pro Asn Val Ser Val Pro Ala Asn
    130                 135                 140

Thr Ser His Ser Arg Thr Pro Glu Gly Leu Tyr Gln Val Thr Ser Val
145                 150                 155                 160

Leu Arg Leu Lys Pro Pro Pro Gly Arg Asn Phe Ser Cys Val Phe Trp
                165                 170                 175

Asn Thr His Val Arg Glu Leu Thr Leu Ala Ser Ile Asp Leu Gln Ser
            180                 185                 190

Gln Met Glu Pro Arg Thr His Pro Thr Trp Glu Pro Lys Ser Cys Asp
        195                 200                 205

Lys Thr His Thr Cys Pro Pro Cys Pro Ala Pro Glu Leu Leu Gly Gly
    210                 215                 220

Pro Ser Val Phe Leu Phe Pro Pro Lys Pro Lys Asp Thr Leu Met Ile
225                 230                 235                 240

Ser Arg Thr Pro Glu Val Thr Cys Val Val Val Asp Val Ser His Glu
                245                 250                 255

Asp Pro Glu Val Lys Phe Asn Trp Tyr Val Asp Gly Val Glu Val His
            260                 265                 270

Asn Ala Lys Thr Lys Pro Arg Glu Glu Gln Tyr Asn Ser Thr Tyr Arg
        275                 280                 285

Val Val Ser Val Leu Thr Val Leu His Gln Asp Trp Leu Asn Gly Lys
    290                 295                 300

Glu Tyr Lys Cys Lys Val Ser Asn Lys Ala Leu Pro Ala Pro Ile Glu
305                 310                 315                 320

Lys Thr Ile Ser Lys Ala Lys Gly Gln Pro Arg Glu Pro Gln Val Tyr
                325                 330                 335

Thr Leu Pro Pro Ser Arg Asp Glu Leu Thr Lys Asn Gln Val Ser Leu
            340                 345                 350

Thr Cys Leu Val Lys Gly Phe Tyr Pro Ser Asp Ile Ala Val Glu Trp
        355                 360                 365

Glu Ser Asn Gly Gln Pro Glu Asn Asn Tyr Lys Thr Thr Pro Pro Val
    370                 375                 380

Leu Asp Ser Asp Gly Ser Phe Phe Leu Tyr Ser Lys Leu Thr Val Asp
385                 390                 395                 400

Lys Ser Arg Trp Gln Gln Gly Asn Val Phe Ser Cys Ser Val Met His
                405                 410                 415

Glu Ala Leu His Asn His Tyr Thr Gln Lys Ser Leu Ser Leu Ser Pro
            420                 425                 430

Gly Lys

<210>  58
<211>  453
<212>  PRT
<213>  Cynomolgus sp.

<400>  58

Met Ile Phe Leu Leu Leu Met Leu Ser Leu Glu Leu Gln Leu His Gln
1               5                   10                  15

Ile Ala Ala Leu Phe Thr Val Thr Val Pro Lys Glu Leu Tyr Ile Ile
            20                  25                  30

Glu His Gly Ser Asn Val Thr Leu Glu Cys Asn Phe Asp Thr Gly Ser
        35                  40                  45

His Val Asn Leu Gly Ala Ile Thr Ala Ser Leu Gln Lys Val Glu Asn
    50                  55                  60

Asp Thr Ser Pro His Arg Glu Arg Ala Thr Leu Leu Glu Glu Gln Leu
65                  70                  75                  80

Pro Leu Gly Lys Ala Ser Phe His Ile Pro Gln Val Gln Val Arg Asp
                85                  90                  95

Glu Gly Gln Tyr Gln Cys Ile Ile Ile Tyr Gly Val Ala Trp Asp Tyr
            100                 105                 110

Lys Tyr Leu Thr Leu Lys Val Lys Ala Ser Tyr Arg Lys Ile Asn Thr
        115                 120                 125

His Ile Leu Lys Val Pro Glu Thr Asp Glu Val Glu Leu Thr Cys Gln
    130                 135                 140

Ala Thr Gly Tyr Pro Leu Ala Glu Val Ser Trp Pro Asn Val Ser Val
145                 150                 155                 160

Pro Ala Asn Thr Ser His Ser Arg Thr Pro Glu Gly Leu Tyr Gln Val
                165                 170                 175

Thr Ser Val Leu Arg Leu Lys Pro Pro Pro Gly Arg Asn Phe Ser Cys
            180                 185                 190

Val Phe Trp Asn Thr His Val Arg Glu Leu Thr Leu Ala Ser Ile Asp
        195                 200                 205

Leu Gln Ser Gln Met Glu Pro Arg Thr His Pro Thr Trp Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser

290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210>  59
<211>  434
<212>  PRT
<213>  Cynomolgus sp.

<400>  59

Leu Phe Thr Val Thr Val Pro Lys Glu Leu Tyr Ile Ile Glu His Gly
1               5                   10                  15

Ser Asn Val Thr Leu Glu Cys Asn Phe Asp Thr Gly Ser His Val Asn
            20                  25                  30

Leu Gly Ala Ile Thr Ala Ser Leu Gln Lys Val Glu Asn Asp Thr Ser
        35                  40                  45

Pro His Arg Glu Arg Ala Thr Leu Leu Glu Glu Gln Leu Pro Leu Gly

50                  55                  60

Lys Ala Ser Phe His Ile Pro Gln Val Gln Val Arg Asp Glu Gly Gln
65                  70                  75                  80

Tyr Gln Cys Ile Ile Ile Tyr Gly Val Ala Trp Asp Tyr Lys Tyr Leu
                85                  90                  95

Thr Leu Lys Val Lys Ala Ser Tyr Arg Lys Ile Asn Thr His Ile Leu
            100                 105                 110

Lys Val Pro Glu Thr Asp Glu Val Glu Leu Thr Cys Gln Ala Thr Gly
        115                 120                 125

Tyr Pro Leu Ala Glu Val Ser Trp Pro Asn Val Ser Val Pro Ala Asn
    130                 135                 140

Thr Ser His Ser Arg Thr Pro Glu Gly Leu Tyr Gln Val Thr Ser Val
145                 150                 155                 160

Leu Arg Leu Lys Pro Pro Pro Gly Arg Asn Phe Ser Cys Val Phe Trp
                165                 170                 175

Asn Thr His Val Arg Glu Leu Thr Leu Ala Ser Ile Asp Leu Gln Ser
            180                 185                 190

Gln Met Glu Pro Arg Thr His Pro Thr Trp Glu Pro Lys Ser Cys Asp
        195                 200                 205

Lys Thr His Thr Cys Pro Pro Cys Pro Ala Pro Glu Leu Leu Gly Gly
    210                 215                 220

Pro Ser Val Phe Leu Phe Pro Pro Lys Pro Lys Asp Thr Leu Met Ile
225                 230                 235                 240

Ser Arg Thr Pro Glu Val Thr Cys Val Val Val Asp Val Ser His Glu
                245                 250                 255

Asp Pro Glu Val Lys Phe Asn Trp Tyr Val Asp Gly Val Glu Val His
            260                 265                 270

Asn Ala Lys Thr Lys Pro Arg Glu Glu Gln Tyr Asn Ser Thr Tyr Arg
        275                 280                 285

Val Val Ser Val Leu Thr Val Leu His Gln Asp Trp Leu Asn Gly Lys
    290                 295                 300

Glu Tyr Lys Cys Lys Val Ser Asn Lys Ala Leu Pro Ala Pro Ile Glu
305                 310                 315                 320

Lys Thr Ile Ser Lys Ala Lys Gly Gln Pro Arg Glu Pro Gln Val Tyr
                325                 330                 335

Thr Leu Pro Pro Ser Arg Asp Glu Leu Thr Lys Asn Gln Val Ser Leu
            340                 345                 350

Thr Cys Leu Val Lys Gly Phe Tyr Pro Ser Asp Ile Ala Val Glu Trp
        355                 360                 365

Glu Ser Asn Gly Gln Pro Glu Asn Asn Tyr Lys Thr Thr Pro Pro Val
    370                 375                 380

Leu Asp Ser Asp Gly Ser Phe Phe Leu Tyr Ser Lys Leu Thr Val Asp
385                 390                 395                 400

Lys Ser Arg Trp Gln Gln Gly Asn Val Phe Ser Cys Ser Val Met His
                405                 410                 415

Glu Ala Leu His Asn His Tyr Thr Gln Lys Ser Leu Ser Leu Ser Pro
            420                 425                 430

Gly Lys

<210>  60
<211>  5
<212>  PRT
<213>  Artificial sequence

<220>
<223>  Sub fragement of amino acids within SEQ ID NO: 3 and 23

<400>  60

Trp Asp Tyr Lys Tyr
1               5